# EUROPEAN PATENT APPLICATION

(11) **EP 4 092 137 A1**
(43) Date of publication of application: **23.11.2022**
(21) Application number: 21382469.1
(22) Date of filing: 21.05.2021
(51) Int. Cl.: C12Q 1/6876, C12Q 1/6883

(54) **METHOD FOR MONITORING OR PREDICTING WHETHER A PATIENT SUFFERING FROM OBESITY IS RESPONDING OR WILL RESPOND TO A VERY-LOW-CALORIE KETOGENIC DIET (VLCKD)**

(71) Applicant: Fundación Instituto de Investigación Sanitaria De Santiago de Compostela (FIDIS), Santiago de Compostela (ES); Servizo Galego de Saude, 15703 Santiago de Compostela (ES); Consorcio Centro de Investigación Biomédica en Red, M.P. (CIBER), 28029 Madrid (ES); Universidade de Santiago de Compostela, 15782 Santiago de Compostela (ES); Universidad De Valladolid, 47002 Valladolid (ES); Pronokal Health Group S.L., 08009 Barcelona (ES)
(72) Inventor: CRUJEIRAS MARTÍNEZ, Ana Belén, 15706 Santiago de Compostela (ES); CASANUEVA FREIJO, Felipe, 15706 Santiago de Compostela (ES); GONZÁLEZ IZQUIERDO, Andrea, 15706 Santiago de Compostela (ES); DE LUIS ROMAN, Daniel Antonio, 47005 Valladolid (ES); NÚÑEZ, Maitane, 08009 Barcelona (ES); SAJOUX, Ignacio, 08009 Barcelona (ES)
(74) Representative: Hoffmann Eitle

(57) **Abstract**

The present invention refers to an *in vitro* method for screening and/or selecting genes whose methylation status indicates whether a patient suffering from obesity is responding or will respond to a treatment with VLCKD. Moreover, the present invention refers to an *in vitro* method for monitoring or predicting whether a patient suffering from obesity is responding or will respond to VLCKD.

## Description

### FIELD OF THE INVENTION

The present invention refers to the medical field. Particularly, the present invention refers to an *in vitro* method for screening and/or selecting genes whose methylation status indicates whether a patient suffering from obesity is responding or will respond to a treatment with a very-low-calorie ketogenic diet (VLCKD). Moreover, the present invention refers to an *in vitro* method for monitoring or predicting whether a patient suffering from obesity is responding or will respond to VLCKD.

### STATE OF THE ART

Ketosis has gained interest over recent years due to its induced benefits that it imparts on several health conditions. Ketosis is associated with a delay in the onset of diseases and increased longevity. Similarly, ketosis is suggested to have an extensive range of health benefits, from increased physical endurance in athletes to delayed aging. Also, to improve conditions such as neurodegenerative disease cancer, cardiovascular disease, and obesity. Some of these studies involved high fat ketogenic diets and even though the main characteristic of ketogenic diets is the carbohydrates restriction, the specific composition in macronutrients and calories should be taken into consideration for the impact in clinical practice.

VLCKD was demonstrated to be an effective strategy in managing obesity, including weight loss and maintenance, increased preservation of muscle mass, and enhanced resting metabolic rate. Moreover, it can improve metabolic parameters in patients with obesity and type 2 diabetes. Additionally, it was demonstrated that VLCKD can reduce food craving and improve psychobiological parameters to help improve quality of life in patients with obesity. However, the molecular mechanisms underlying these benefits of ketogenic diet remain unknown.

So, there is an unmet medical need of finding a molecular method for monitoring or predicting whether a patient suffering from obesity is responding or will respond to VLCKD, particularly whether an improvement of metabolic parameters has occurred in a patient suffering from obesity after the induction of nutritional ketosis by means of a treatment with VLCKD. The present invention aims to solve this need and an evaluation regarding how VLCKD might affect the obesity methylome is herein provided. Obesity-related methylome changes have been identified in the present invention, which are mediated by the induced weight loss or ketosis by means of treatment with VLCKD. Said methylome changes are thus herein proposed as an indication of whether a patient suffering from obesity is responding or will respond to a treatment with VLCKD.

### DESCRIPTION OF THE INVENTION

### Brief description of the invention

The present invention refers to a method for monitoring or predicting whether a patient suffering from obesity is responding or will respond to VLCKD, particularly for monitoring or predicting whether an improvement of metabolic parameters has occurred in a patient suffering from obesity after the induction of nutritional ketosis by means of a treatment with VLCKD.

Particularly, twenty-one patients with obesity (n=12 women, 47.9±1.02 yr, 33.0±0.2 kg/m2) after 6 months on a VLCKD and 12 normal weight volunteers (n=6 women, 50.3±6.2 yrs, 22.7±1.5 kg/m2) were studied. Data from the Infinium MethylationEPIC BeadChip methylomes of blood leukocytes were obtained at time points of ketotic phases (basal, maximum ketosis, and out of ketosis) during VLCKD (n = 10) and at baseline in volunteers (n = 12). Results were further validated by pyrosequencing in representative cohort of patients on a VLCKD (n = 18) and correlated with gene expression.

After weight reduction by VLCKD, differences were found at 988 CpG sites (786 unique genes). The VLCKD altered methylation levels in patients with obesity had high resemblance with those from normal weight volunteers and was concomitant with a downregulation of DNA methyltransferases (DNMT)1, 3a and 3b. Most of the encoded genes were involved in metabolic processes, protein metabolism, and muscle, organ, and skeletal system development. Novel genes representing the top scoring associated events were identified, including ZNF331, FGFRL1 (VLCKD-induced weight loss) and CBFA2T3, C3orf38, JSRP1, and LRFN4 (VLCKD-induced ketosis). Interestingly, ZNF331 and FGFRL1 were validated in an independent cohort and inversely correlated with gene expression.

So, the present invention is making a clear contribution as compared to the prior art, since the molecular mechanisms underlying the potential health benefits of a ketogenic diet are still unknown. So, the present invention is showing for the first time that the methylome status indicates whether a patient suffering from obesity is responding or will respond to a treatment with a VLCKD, particularly whether an improvement of metabolic parameters has occurred in a patient suffering from obesity after the induction of nutritional ketosis by means of a treatment with VLCKD.

Consequently, the special technical feature that defines a contribution over the prior art and confers unity to the present invention is that the methylation status indicates whether a patient suffering from obesity is responding or will respond to a treatment with a VLCKD, particularly whether an improvement of metabolic parameters has occurred in a patient suffering from obesity after the induction of nutritional ketosis by means of a treatment with VLCKD.

So, the first embodiment of the present invention refers to an *in vitro* method for screening and/or selecting genes whose methylation status indicates whether a patient suffering from obesity is responding or will respond to a treatment with a VLCKD, which comprises:
a. Selecting those CpGs characterized by being differentially methylated, showing a differential β value ≥ 2% and FDR < 0.10 between patients who are in the period of maximum ketosis that occurs between days 30 and 90 of the diet and the patients who are at the end of the diet that occurs between days 120 and 180, with respect to a control value represented by the level of methylation in patients who are in baseline state that occurs at the beginning of the diet; and/or
b. Selecting those CpGs characterized by being differentially methylated, showing a differential β value ≥ 2% and FDR < 0.10 between patients who are in the period of maximum ketosis that occurs between days 30 and 90 of the diet, with respect to a control value represented by the level of methylation in patients who are in baseline state that occurs at the beginning of the diet, and discarding those CpGs that are differentially methylated between the patients who are in the period of maximum ketosis that occurs between days 30 and 90 of the diet and the patients who are at the end of the diet that occurs between days 120 and 180; and
c. Selecting those genes comprising in the promoter region at least two of the differentially methylated CpG sites which have been selected according to the steps a) and/or b).

In a preferred embodiment, the genes and CpG sites which are selected according to the steps a) and c) of claim 1 are comprised in **Table 2** or **Table S2** and their methylation status indicates whether a patient suffering from obesity is responding or will respond to VLCKD giving rise to both weight loss and nutritional ketosis induction, and wherein the genes and CpG sites which are selected according to the steps b) and c) of claim 1 are comprised in **Table 3** or **Table S3** and their methylation status indicates whether a patient suffering from obesity is responding or will respond to VLCKD due to the induction of nutritional ketosis.

In a preferred embodiment, the present invention refers to an *in vitro* method for monitoring or predicting whether a patient suffering from obesity is responding or will respond to VLCKD, which comprises determining the methylation status of at least a gene selected according to the method described above, wherein a statistically significant variation or deviation of level of methylation, as compared with a pre-established level of methylation, is an indication that the subject is responding or will respond to VLCKD.

In a preferred embodiment, the present invention refers to a method for monitoring or predicting whether a patient suffering from obesity is responding or will respond to VLCKD giving rise to both weight loss and nutritional ketosis induction, which comprises determining the methylation status of at least a gene selected from **Table 2** or **Table S2,** wherein a statistically significant variation or deviation of level of methylation, as compared with a pre-established level of methylation, is an indication that the subject is responding or will respond to a VLCKD.

In a preferred embodiment, the methylation status of the genes is determined in at least a CpG site selected from **Table 2** or **Table S2.**

In a preferred embodiment, the present invention refers to a method for monitoring or predicting whether an improvement of metabolic parameters has occurred in a patient suffering from obesity after the induction of nutritional ketosis by means of a treatment with a VLCKD, which comprises determining the methylation status of at least a gene selected from **Table 3** or **Table S3** in a biological sample obtained from the patient, wherein a statistically significant variation or deviation of level of methylation, as compared with a pre-established level of methylation, is an indication that ketosis reduction has occurred.

In a preferred embodiment, the methylation status of the genes is determined in at least a CpG site selected from **Table 3** or **Table S3.**

In a preferred embodiment, an overall hypomethylation of the genes is observed when the patient is responding or will respond to the treatment with a VLCKD.

In a preferred embodiment, the methylation status detection is conducted by means of a technique selected from the group consisting of: methylation specific PCR, bisulfite sequencing, techniques based on restriction-digestion, pyrosequencing, assay ChIP-on-chip, differential conversion, differential restriction and/or differential weight of site(s) methylated.

In a preferred embodiment, the biological sample isolated from the patient is whole blood, preferably blood leucocytes.

The second embodiment of the present invention refers to the *in vitro* use of the methylation status of at least a gene selected according to the above described method for monitoring or predicting whether a patient suffering from obesity is responding or will respond to VLCKD.

In a preferred embodiment, the present invention refers to the *in vitro* use of the methylation status of at least a gene selected from **Table 2** or **Table S2** for monitoring or predicting whether a patient suffering from obesity is responding or will respond to VLCKD giving rise to both weight loss and nutritional ketosis induction.

In a preferred embodiment, the present invention refers to the *in vitro* use of the methylation status of at least a CpG site selected from **Table 2** or **Table S2.**

In a preferred embodiment, the present invention refers to the *in vitro* use of the methylation status of at least a gene selected from **Table 3** or **Table S3** for monitoring or predicting whether an improvement of metabolic parameters has occurred in a patient suffering from obesity after the induction of nutritional ketosis by means of a treatment with a VLCKD.

In a preferred embodiment, the present invention refers to the *in vitro* use of the methylation status of at least a CpG site selected from **Table 3** or **Table S3.**

On the other hand, the present invention also refers to a method for detecting hypermethylation or hypomethylation in at least a gene selected from **Tables 2, S2, 3, S3 or S4** which comprises: a) obtaining DNA from the subject, b) detecting a hypermethylation or hypomethylation in CpG sites selected from **Tables 2, S2, 3, S3 or S4,** wherein said (b) detecting is conducted by a technique selected from the group consisting of methylation specific PCR, bisulfite sequencing, techniques based on restriction-digestion, pyrosequencing, assay ChIP-on-chip, differential conversion, differential restriction and differential weight of site(s) methylated.

The present invention also refers to a computer-implemented invention, wherein a processing unit (hardware) and a software are configured to: Receive methylation level values of any of the genes selected from **Tables 2, S2, 3, S3 or S4;** process the methylation level values received for finding substantial variations or deviations; and provide an output through a terminal regarding the variation or deviation of the methylation level, wherein the variation or deviation of the methylation level indicates whether a patient suffering from obesity is responding or will respond to a treatment with a VLCKD, particularly whether an improvement of metabolic parameters has occurred in a patient suffering from obesity after the induction of nutritional ketosis by means of a treatment with VLCKD.

The last embodiment of the present invention refers to a method for treating a patient suffering from obesity with VLCKD, wherein the method comprises a first step of predicting whether the patient will respond to VLCKD by determining the methylation status of a least a gene selected from **Table 2, S2, 3, S3 or S4,** according to the method herein described.

For the purpose of the present invention the following abbreviation list is included:
- ACACB, Acetyl-CoA Carboxylase Beta.
- AEBP1, AE binding protein 1.
- ANOVA, analysis of variance.
- BMI, body mass index.
- C3orf38, chromosome 3 open reading frame 38.
- CACNA1H, Calcium Voltage-Gated Channel Subunit Alpha1 H.
- CAMKK1, Calcium/Calmodulin Dependent Protein Kinase Kinase 1.
- CBFA2T3, CBFA2/RUNX1 Partner Transcriptional Co-Repressor 3.
- cDNA, complementary DNA.
- CENPF, Centromere Protein F.
- CHAT, choline O-acetyltransferase.
- CHUK, Component Of Inhibitor Of Nuclear Factor Kappa B Kinase Complex.
- COL1A1, collagen, type I, alpha 1.
- COL5A1, collagen type V alpha 1 chain.
- COL9A2, collagen, type IX, alpha 2.
- CV, coefficient of variation.
- DMCpGs, differentially methylated CpGs.
- DNMTs, DNA methyltransferases.
- DNA, deoxyribonucleic acid.
- EWAS, epigenome-wide association study.
- FDR, false discovery rate.
- FF, fresh-frozen.
- FGFRL1, Fibroblast growth factor receptor (FGFR)-like protein 1.
- GAPDH, Glyceraldehyde-3-Phosphate Dehydrogenase.
- GO, gene ontology.
- HRAS, HRas proto-oncogene, GTPase.
- INSR, insulin receptor.
- JSRP1, Junctional Sarcoplasmic Reticulum Protein 1.
- LAMA2, Laminin Subunit Alpha 2.
- LRFN4, Leucine Rich Repeat And Fibronectin Type III Domain Containing 4.
- MKNK2, MAPK Interacting Serine/Threonine Kinase 2.
- PRKAG2, Protein Kinase AMP-Activated Non-Catalytic Subunit Gamma 2.
- PRKCZ, Protein Kinase C Zeta.
- qRT-PCR, Real-time quantitative polymerase chain reaction.
- RELA, v-rel reticuloendotheliosis viral oncogene homolog A.
- RNA, Ribonucleic acid.
- RPTOR, Regulatory Associated Protein Of MTOR Complex 1
- SD, standard deviation.
- SEM, standard error of the mean.
- SGCB, Beta-sarcoglycan.
- SMTN, Smoothelin.
- TRAF2, TNF Receptor Associated Factor 2.
- TSC2, Tuberous Sclerosis Complex 2.
- TSS 200, transcription start sites 200
- VLCKD, very-low-calorie ketogenic diet
- WC, Waist circumference.
- ZFHX3, zinc finger Homeobox 3.
- ZNF331, zinc finger protein 331.
- β-OHB, beta-hydroxy-butyrate.
- Detailed description of the invention.

For the purpose of the present invention the following terms are defined:
- The terms "pre-established threshold level" or "control level" refer to the methylation level measured in patients suffering from obesity, before being treated with VLCKD. Typically, this level can be determined experimentally, empirically, or theoretically. This value can also be arbitrarily selected based upon the existing experimental and/or clinical conditions, as would be recognized by a person of ordinary skilled in the art.
- As used herein, the term "methylation" will be understood to mean the presence of a methyl group added by the action of a DNA methyl transferase enzyme to a cytosine base or bases in a region of nucleic acid e.g. genomic DNA.
- Accordingly, the term, "methylation status" as used herein refers to the presence or absence of methylation in a specific nucleic acid region e.g., genomic region.
- The expression "higher" or "lower" level of methylation refers to an increase or decrease in the relative amount of methylation of a nucleic acid e.g., genomic DNA, as compared with the patient used as control
- As used herein, a "CpG dinucleotide","CpG methylation site" or equivalent, shall be taken to denote a cytosine linked to a guanine by a phosphodiester bond. CpG dinucleotides are targets for methylation of the cytosine residue and may reside within coding or non-coding nucleic acids. Non-coding nucleic acids are understood in the art to include introns, 5'-untranslated regions, 3' untranslated regions, promoter regions of a genomic gene, or intergenic regions.
- The term "comprising" means including, but not limited to, whatever follows the word "comprising". Thus, use of the term "comprising" indicates that the listed elements are required or mandatory, but that other elements are optional and may or may not be present.
- The term "consisting of' means including, and limited to, whatever follows the phrase "consisting of'. Thus, the phrase "consisting of' indicates that the listed elements are required or mandatory, and that no other elements may be present.
- The "β value" refers to the methylation level of each cytosine, which is calculated as the fluorescence intensity ratio of the methylated to the unmethylated version of the probe. β values ranged between 0 (unmethylated) and 1 (completely methylated) according to the combination of the Cy3 and Cy5 fluorescence intensities [Pan Du et al., 2010. Comparison of Beta-value and M-value methods for quantifying methylation levels by microarray analysis. Comparative Study. BMC Bioinformatics. 2010 Nov 30;11:587. doi: 10.1186/1471-2105-11-587. PMID: 21118553 PMCID: PMC3012676 DOI: 10.1186/1471-2105-11-587].
- "FDR" refers to false discovery rate. It is a statistical approach used in multiple hypothesis testing to correct for multiple comparisons. It is typically used in high-throughput experiments in order to correct for random events that falsely appear significant. When testing a null hypothesis to determine whether an observed score is statistically significant, a measure of confidence, the p-value, is calculated and compared to a confidence threshold α. When k hypotheses are tested simultaneously with a confidence level α, the chances of occurrence of false positives (i.e., rejecting the null hypothesis when in fact it is true) is equal to 1 - (1 - α)k, which can lead to a high error rate in the experiment. Therefore, a multiple testing correction, such as the FDR, is needed to adjust our statistical confidence measures based on the number of tests performed.
- The expression "differentially methylated" refers to the existence of different DNA methylation status across different biological samples and regarded as possible functional regions involved in gene transcriptional regulation. The biological samples can be different cells/tissues within the same individual, the same cell/tissue at different times, cells/tissues from different individuals, even different alleles in the same cell.
- "Very-low-calorie ketogenic diet" (VLCKD) is a term which pertains to the common general knowledge, and it is characterized by low fat consumption, preferably by the consumption of less than 800Kcal/day [Marco Castellana et al., 2020. Efficacy and safety of very low calorie ketogenic diet (VLCKD) in patients with overweight and obesity: A systematic review and meta-analysis. Rev Endocr Metab Disord. 2020 Mar;21(1):5-16. doi: 10.1007/s11154-019-09514-y*].*

### Description of the figures

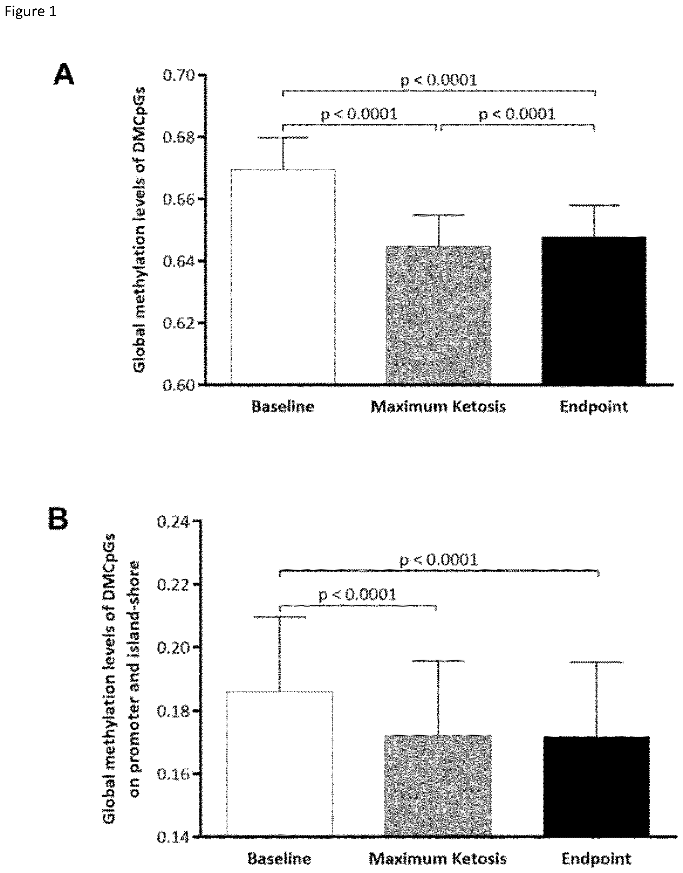

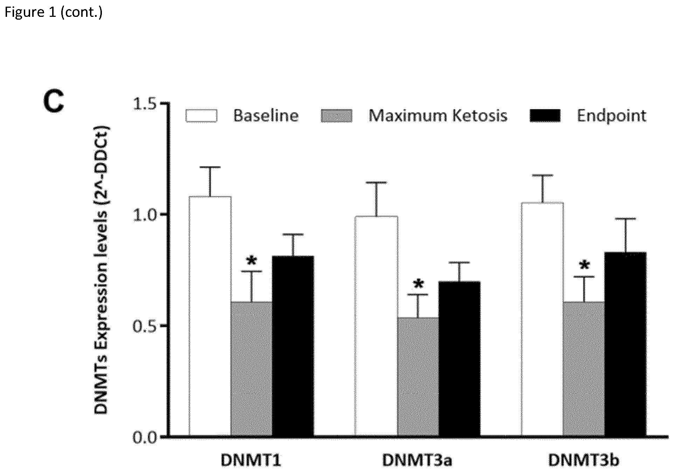

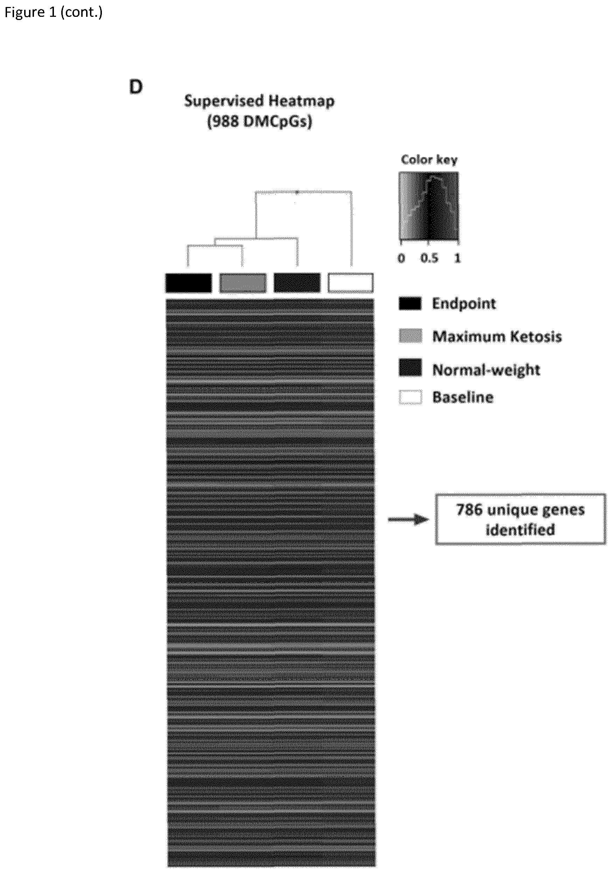

**Figure 1****. Profile of DNA methylation following the VLCKD (n = 10; Discovery cohort). (A).** Global differences in methylation levels of 988 DMCpGs identified by Infinium MethylationEPIC BeadChip analysis. **(B).** Global differences in methylation levels of DMCpGs located in the promoter and island/shore. **(C).** Expression levels of DNA methyltransferase (DNMT) 1 and the de novo methyltransferases DNMT3A and DNMT3B **(D).** Supervised clustering of the 988 CpGs that were found to be differentially methylated between patients with obesity and healthy volunteers with normal weight (n=12) groups. The identified CpG sites mapped to 786 unique genes. Differences statistically significant detected (P < 0.0001). DMCpGs, differentially methylated CpGs; VLCKD, very-low calorie ketogenic diet.

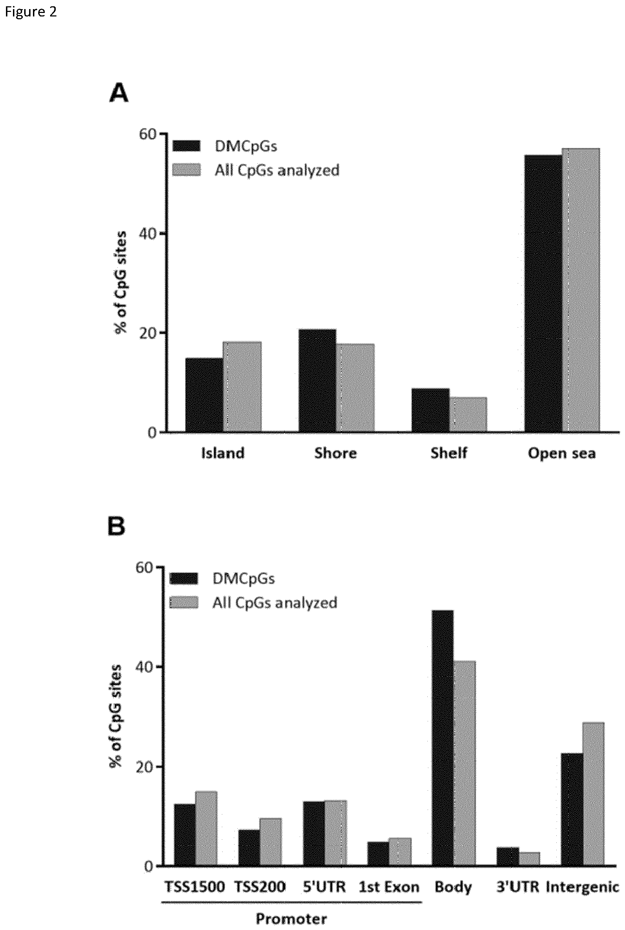

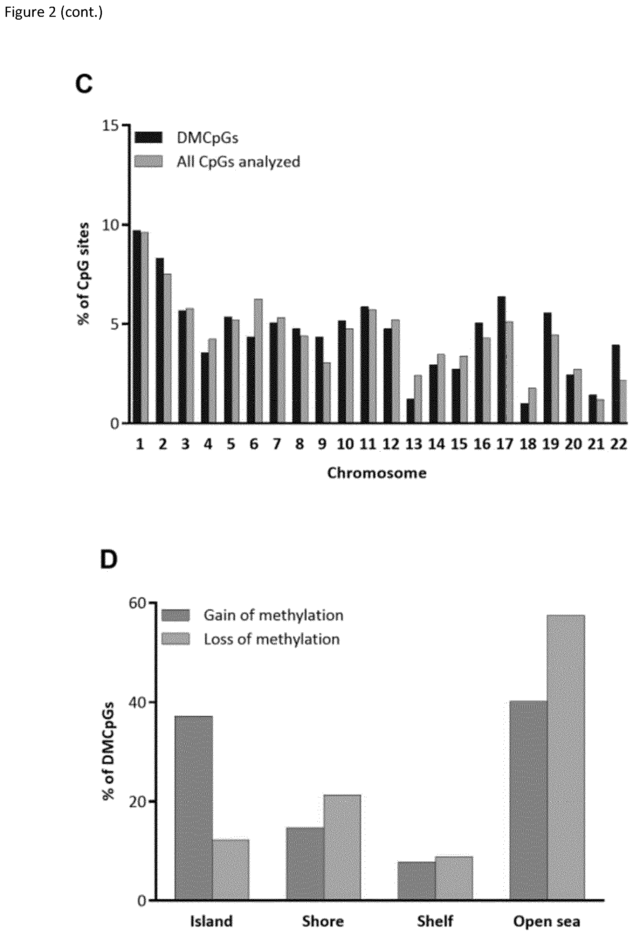

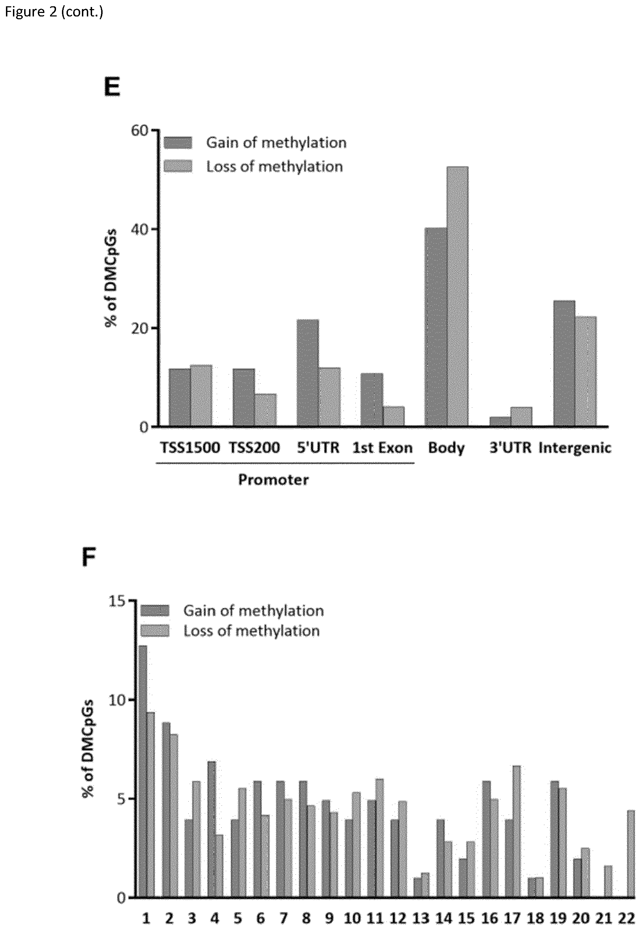

**Figure 2****. Characterization of the DMCpGs after VLCKD in the discovery cohort (n = 10). (A)** Genomic distribution of the DMCpGs and their respective locations regarding the broader CpG context, **(B)** gene region and **(C)** chromosome. **(D)** Genomic distribution of DMCpGs comparing those that exhibited an increase with those that exhibited a decrease in methylation levels following VLCKD, and their respective locations in the broader CpG context, **(E)** the gene region and (F) chromosome. DMCpGs, differentially methylated CpGs; VLCKD, very-low calorie ketogenic diet.

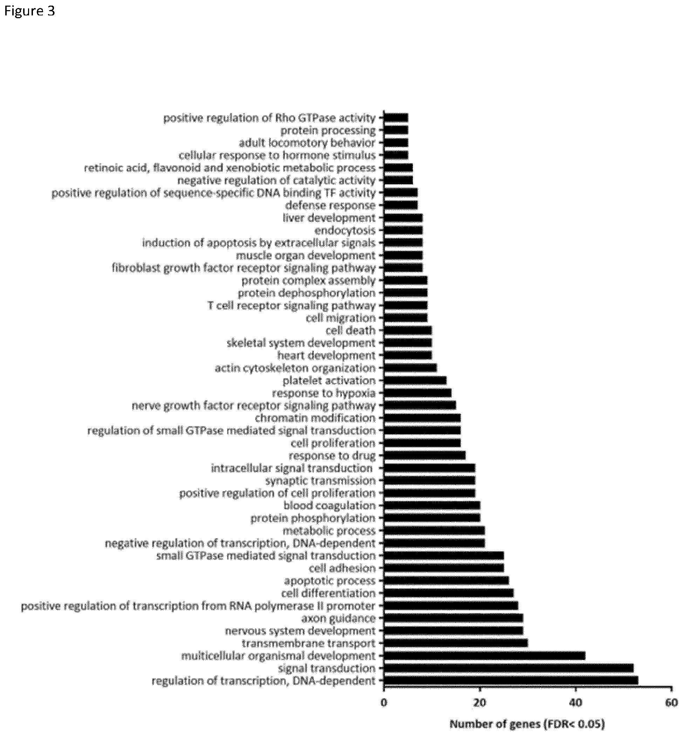

**Figure 3****. Biological implications of the DMCpGs following a VLCKD. (A)** Summary of the GO analysis of the biological process categories representing the differentially methylated genes associated with DMCpG sites. **(B)** Gene-protein interaction network-STRING analysis. Most of the genes regulated by methylation belonged to a network significantly enriched in protein interactions (p < 0.001) according to STRING analysis. DMCpGs, differentially methylated CpGs; GO, gene ontology; VLCKD, very-low calorie ketogenic diet.

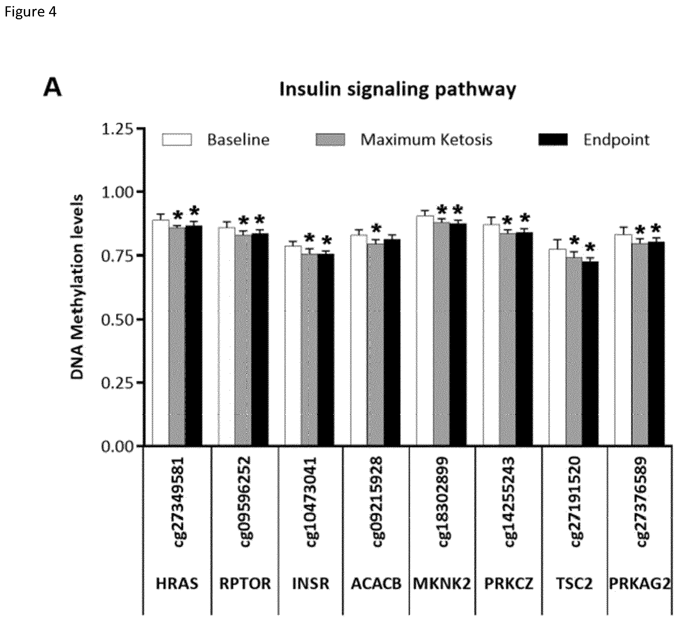

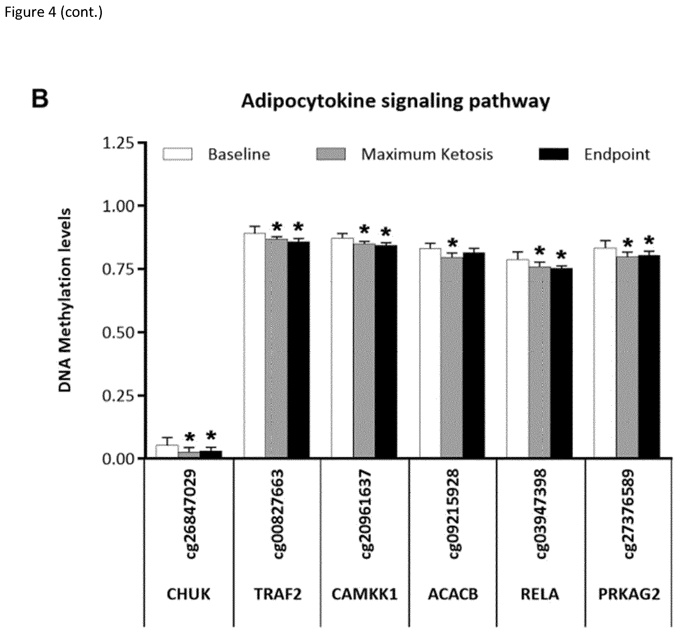

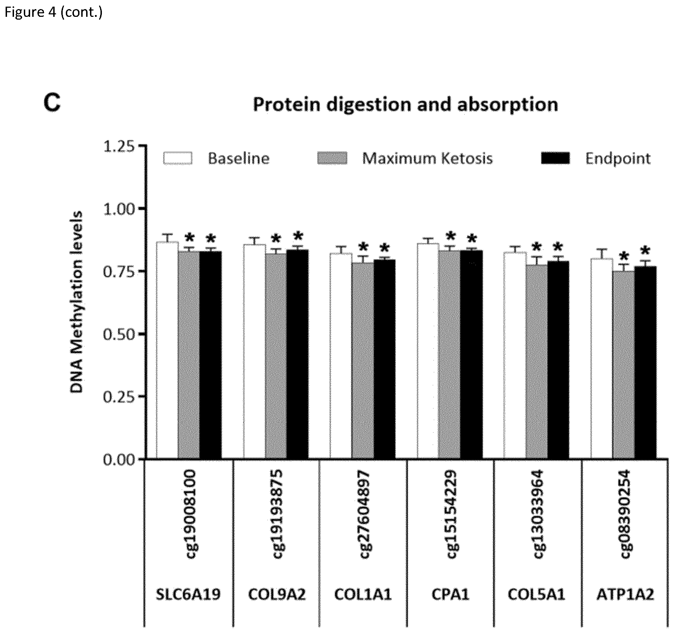

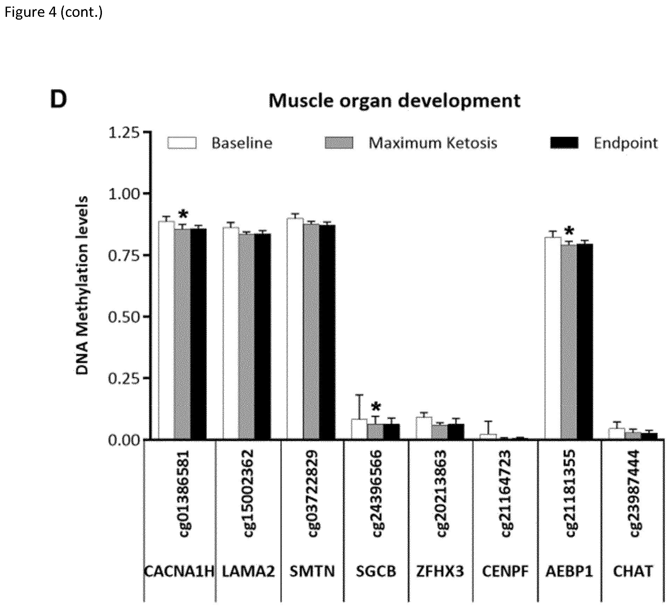

**Figure 4****. Genes with known functions that present DMCpGs following a VLCKD. (A)** Novel genes epigenetically regulated following VLCKD belonged to the insulin signaling pathway, **(B)** adipocytokine signaling pathway, **(C)** protein digestion and absorption functions, and **(D)** muscle organ development functions. DNA methylation values are expressed as b-values from the Infinium MethylationEPIC BeadChip. *Denotes differences statistically significant (P < 0.05). DMCpGs, differentially methylated CpGs; VLCKD, very-low calorie ketogenic diet.

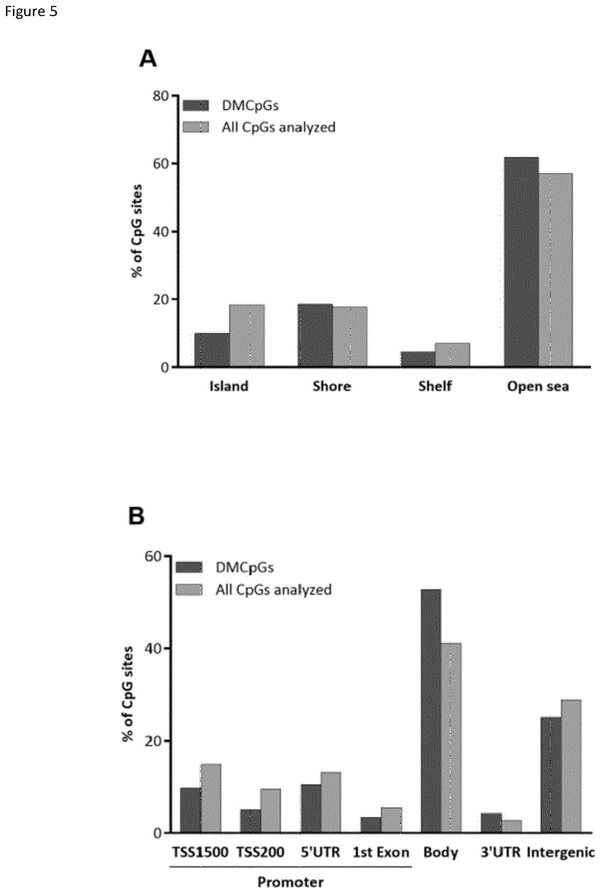

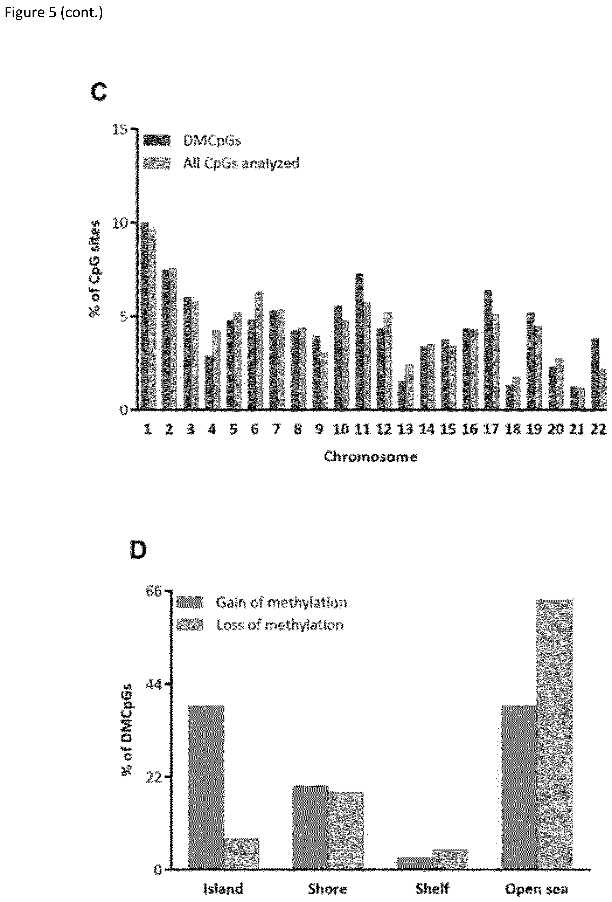

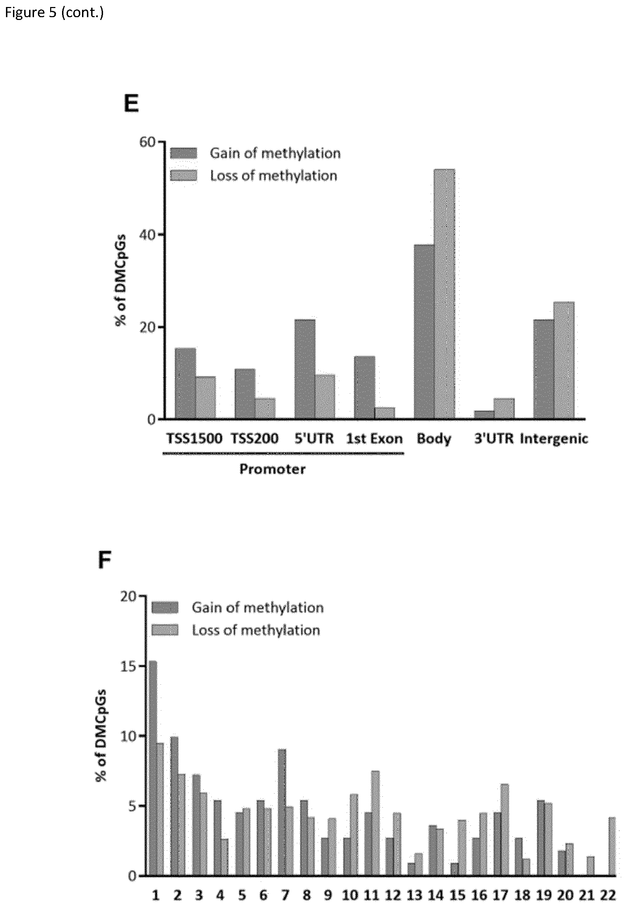

**Figure 5****. Characterization of the DMCpGs during ketosis induced by a VLCKD from the discovery cohort (n = 10).** (A) Genomic distribution of the DMCpGs and their respective locations regarding the broader CpG context, (B) gene region and (C) chromosome. (D) Genomic distribution of DMCpGs comparing those that exhibited an increase with those that exhibited a decrease in methylation levels during the VLCKD-induced ketosis and their respective locations in the broader CpG context, (E) the gene region and (F) chromosome. DMCpGs, differentially methylated CpGs; VLCKD, very-low calorie ketogenic diet.

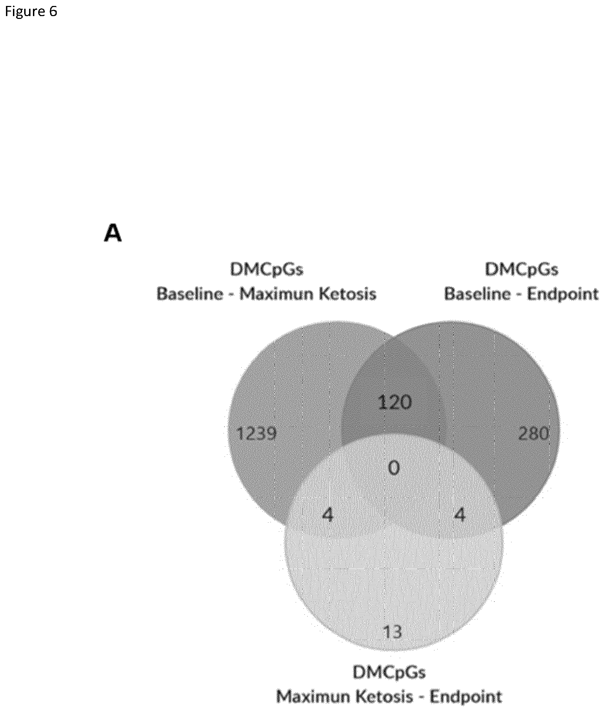

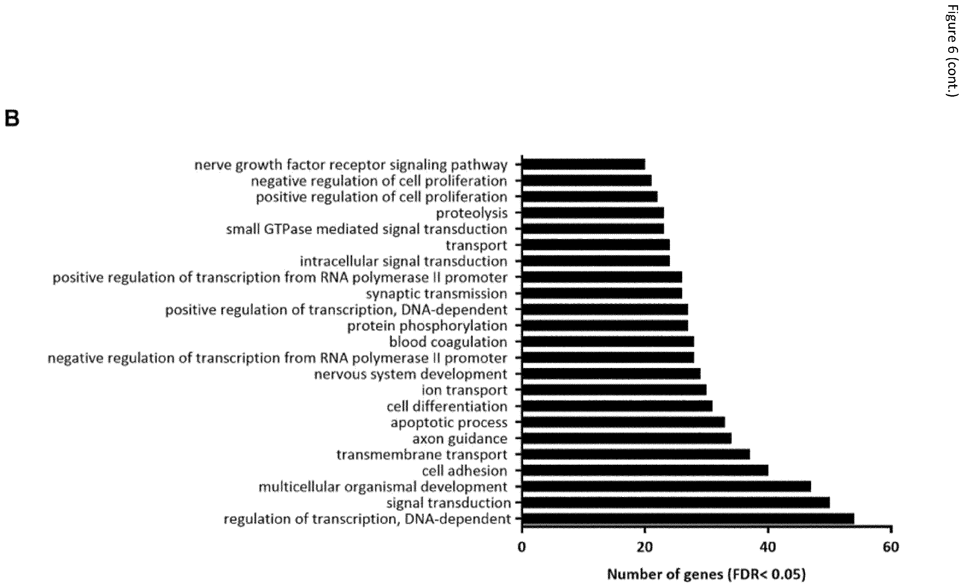

**Figure 6****. Biological implications of the DMCpGs related to VLCKD-induced ketosis. (A)** Venn diagram of the DMCpGs detected between baseline and maximum ketosis, between baseline and endpoint, and between maximum ketosis and endpoint. From this analysis, 1239 CpGs were identified as nutritional ketosis-related DMCpGs. **(B)** Summary of the GO analysis of the biological process categories representing the differentially methylated genes associated with nutritional ketosis-related DMCpG sites. DMCpGs, differentially methylated CpGs; GO, gene ontology; VLCKD, very-low calorie ketogenic diet.

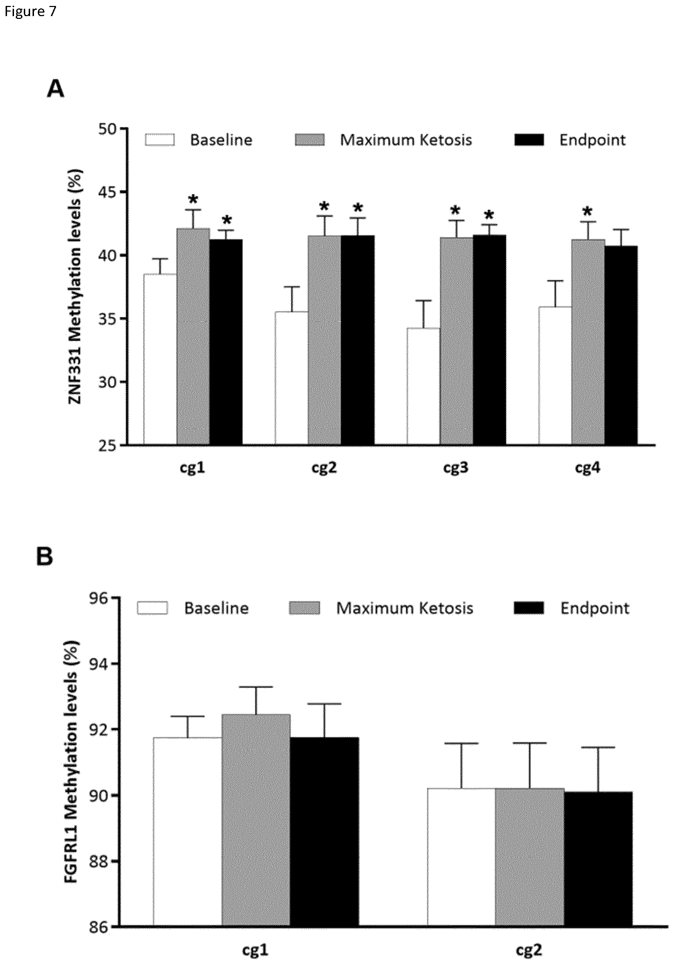

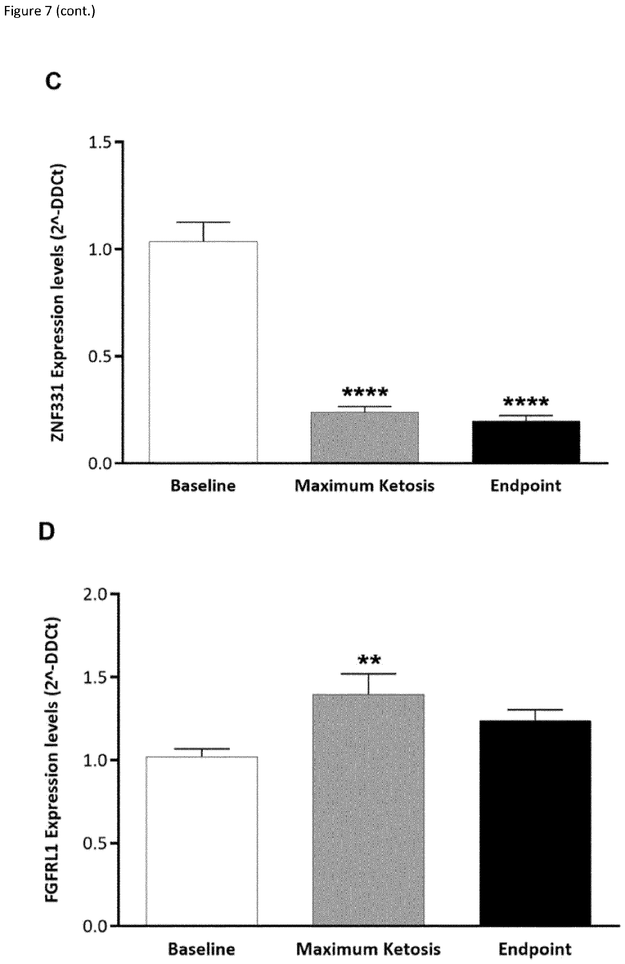

**Figure 7****. DNA methylation of ZNF331 and FGFRL1 is inversely associated with gene expression.** DNA methylation differences for ZNF331 **(A)** and FGFRL1 **(B)** after VLCKD in the validation cohort (n = 18) as measured by pyrosequencing. Differential gene expression of ZNF331 **(C)** and FGFRL1 **(D)** after VLCKD in the validation cohort (n = 18). *Denotes differences statistically significant (*P < 0.05, **P < 0.01, ***P < 0.001). VLCKD, very-low calorie ketogenic diet.

### Detailed description of the invention

### Example 1. Materials and methods

### Example 1.1 Patient cohort

The DNA and RNA for methylation and gene expression assays were isolated from blood samples of patients from a 6-month nutritional intervention study performed at the Endocrinology and Nutrition Department of the Hospital Clinico, Universitario of Valladolid; the patients were receiving treatment for obesity. In addition, samples from a group of healthy volunteers were also analyzed. The inclusion criteria were age between 18 to 65 years, body mass index (BMI) ≥ 30 kg/m², stable body weight over the previous 3 months, a desire to lose weight, and a history of failed dietary efforts. The main exclusion criteria were thyroid alteration, diabetes mellitus, obesity induced by other endocrine disorders or drugs, and participation in any active weight-loss program in the previous 3 months. In addition, patients with previous bariatric surgery, reported or suspected abuse of narcotics or alcohol, severe depression or any other psychiatric disease, severe hepatic insufficiency, any type of renal insufficiency or gout episodes, nephrolithiasis, neoplasia, previous instances of cardiovascular or cerebrovascular disease, uncontrolled hypertension, orthostatic hypotension, and hydroelectrolytic or electrocardiographic alterations were excluded. Females who were pregnant, breastfeeding, or intending to become pregnant and those with child-bearing potential who were not using adequate contraceptive methods were also excluded. Apart from obesity and metabolic syndrome, participants were generally healthy individuals. Under these criteria, 21 patients with obesity and 12 volunteers with normal weight were included in this study. The study protocol was in accordance with the Declaration of Helsinki and was approved by the Ethics Committee for Clinical Research of Hospital Clinico Universitario de Valladolid, Spain (C.I: 40/13, PNK-DHA2013-01). Participants provided written informed consent before any intervention related to the study. Participants received no monetary incentives.

### Example 1.2. Very-low-calorie ketogenic diet protocol

Patients included in this study derived from a randomized clinical trial investigating the effect of docosahexaenoic acid (DHA) supplementation in a very low-calorie ketogenic diet. The clinical trial consisted in two arms: one arm where patients follow a VLCKD and other arms where patients followed a VLCKD+DHA. Nutritional intervention was based on a commercial weight-loss program (PNK method ^{®}). Briefly, the intervention included an evaluation by the specialist physician conducting the study, an assessment by an expert dietician, and exercise recommendations. This method is based on high-biological-value protein preparations obtained from cow's milk, soy, avian eggs, green peas, and cereals. Each protein preparation contained 15g protein, 4g carbohydrates, 3g fat, and provided 90-100 kcal. The VLCKD+DHA arm was supplemented with 500mg DHA. The weight-loss program has five steps and adheres to the most recent guidelines of the EFSA (2015) on total carbohydrate intake. The first three steps consist of a VLCKD (600-800 kcal/day) that is low in carbohydrates (< 50g daily from vegetables) and lipids (only 10g of olive oil per day). The amount of high biological-value proteins ranged between 0.8 and 1.2g per kg of ideal body weight to ensure that patients were meeting their minimum bodily requirements and to prevent the loss of lean mass. In step 1, the patients ate high-biological-value protein preparations five times a day and vegetables with low glycemic indices. In step 2, one of the protein servings was substituted with a natural protein (e.g., meat or fish) either at lunch or at dinner. In step 3, a second serving of low-fat natural protein was substituted for the second serving of biological protein preparation. Throughout these ketogenic phases, supplements of vitamins and minerals, such as K, Na, Mg, Ca, and omega-3 fatty acids, were provided in accordance with international recommendations. These three steps were maintained until the patient lost the target amount of weight, ideally 80%. Because of this, the ketogenic steps varied in time depending on the individual and the weight-loss target. The total ketosis state lasted for a maximum of 60 days. In either step 4 or 5, ketosis was ended by the physician in charge of the patient based on the amount of weight lost, and the patient began a low-calorie diet (800-1500 kcal/day). At this point, the patients underwent a progressive incorporation of different food groups and participated in a program of alimentary re-education to guarantee long-term maintenance of the weight loss. The maintenance diet consisted of an eating plan balanced for carbohydrates, protein, and fat. Depending on the individual, calories consumed ranged between 1500 and 2000 kcal/day, with the goal of maintaining the weight loss and promoting a healthy lifestyle. During this study, patients followed the steps of the method until they reached the target weight, or up to a maximum of 4 months of follow-up, although patients remained under medical supervision for the following months. Patients visited the research team every 15±2 days to evaluate adherence and potential side effects. Complete anthropometry, body composition, and biochemical assessments were performed at four of the visits, which were determined according to the evolution of each patient through the steps of ketosis and weight loss: Visit 1 (Baseline), visit 2 (Maximum Ketosis), visit 3 (Reduced Ketosis) and visit 4 (Endpoint). DNA methylation and gene expression were performed at visits 1, 2, and 4.

In all visits, patients received dietary instructions, individual supportive counsel, and encouragement to exercise on a regular basis using a formal exercise program. Additionally, a program of reinforcement telephone calls was instituted, and a phone number was provided to all participants to address any concerns.

### Example 1.3. Anthropometric assessment

All anthropometric measurements were performed after an overnight fast (8 to 10 hours) under resting conditions in duplicate and performed by well-trained health workers. At each visit, patients were weighed on the same calibrated scale (Seca 200 scale, Medical Resources, EPI Inc OH, USA). BMI was calculated as body weight in kg, divided by the square of body height in meters (BMI = weight (kg)/height² (m). Waist circumference (WC) was measured using a standard flexible non-elastic metric tape placed over the midpoint between the last rib and the iliac crest, with the patient standing and exhaling.

### Example 1.4. Determining levels of ketone bodies

Ketosis was determined by measuring ketone bodies, specifically β-hydroxy-butyrate (β-OPHB), in capillary blood using a portable meter (GlucoMen LX Sensor, A. Menarini Diagnostics, Neuss, Germany; sensitivity <0.2 mmol/l). As with anthropometric assessments, all determinations of capillary ketonemia were made after an overnight fast of 8 to 10 hours. These measurements were performed daily by each patient during the entire VLCKD, and the corresponding values were reviewed using machine memory by the research team to control adherence. Additionally, β-OHB levels were determined at each visit by the physician in charge of the patient.

### Example 1.5. DNA methylation analysis

### DNA preparation and bisulphite conversion

DNA from fresh-frozen (FF) blood samples was isolated using a standard phenolchloroform/proteinase-k protocol according to the manufacturer's instructions, with slight modifications. Genomic DNA was isolated from leukocytes using the MasturPure^{™} DNA purification kit (Epicentre Biotechnologies, Madison, WI, USA). The isolated DNA was treated with RNase A for 1 h at 45 °C. All DNA samples were quantified using the fluorometric method (Quan-iT PicoGreen DsDNA Assay, Life Technologies) and were assessed for purity using a NanoDrop (Thermo Scientific) to determine 260/280 and 260/230 ratio measurements. The integrity of the FF DNA was verified by electrophoresis in 1.3% agarose gel. DNA (500 ng) was bisulfite converted using the EZ DNA methylation kit Methylation-Gold (Zymo Research, CA, USA) according to the manufacturer's instructions, which converts non-methylated cytosine into uracil.

### Infinium MethylationEPIC BeadChip

High-quality DNA samples (500 ng) obtained from blood leukocytes of patients included in the VLCKD+DHA arm of the clinical trial (discovery cohort; n = 10 patients, 3 paired samples/patient) were selected for bisulfite conversion (Zymo Research; EZ-96 DNA Methylation^{™} Kit) and hybridization to Infinium MethylationEPIC BeadChip (Illumina) following the Illumina Infinium HD methylation protocol. DNA quality checks, bisulfite modification, hybridization, data normalization, statistical filtering, and value calculations were performed as previously described. Whole-genome amplification and hybridization were then performed on a BeadChip followed by single-base extension and analysis on a HiScan SQ module (Illumina) to assess cytosine methylation states. The annotation of CG islands (CGIs) used the following categorization: 1) shore, for each of the 2-kb sequences flanking a CGI; 2) shelf, for each of the 2-kb sequences next to a shore; and 3) open sea, for DNA not included in any of the previous sequences or in CGIs. The transcription start site 200 and the transcription start site 1500 indicate regions either 200 or 1500 bp from the transcription start site, respectively.

### Pyrosequencing analysis

Pyrosequencing was used to assess selected markers in 18 patients (validation cohort: (n=7 derived from the discovery cohort and n=11 from an independent cohort of patients; 3 paired samples/patient). DNA samples analyzed in the validation cohort were derived from patients included in the two arms of the clinical trial and merged for the statistical analysis (VLCKD: n=10; VLCKD+DHA: n=8). The primer sequences used in this analysis were designed using Qiagen's PyroMark Assay Design 2.0 software to hybridize to CpG-free sites to ensure methylation-independent amplification. Genomic DNA was isolated from FF blood leukocytes using the MasturPure^{™} DNA purification kit (Epicentre Biotechnologies, Madison, WI, USA), according to the manufacturer's instructions. DNA methylation analyses were performed using bisulfite-treated DNA (Zymo Research; EZ-96 DNA Methylation^{™} Kit) followed by a highly quantitative analysis based on PCR-based pyrosequencing using the PyroMark Q24 System version 2.0.7 (Qiagen). Methylation level was expressed as the percentage of methylated cytosine over the sum of methylated and unmethylated cytosines. Non-CpG cytosine residues were used as built-in controls to verify bisulfite conversion. The values are expressed as the mean for all sites. We also included human non-methylated and methylated DNA set as controls in each run (Zymo Research). The inter-assay precision (%CV) was < 2.5%, intra-assay (%CV) was < 1.0%.

### Example 1.6. Expression assay by qRT-PCR

RNA from blood leukocytes (n = 18 patients) was extracted using Trizol (Invitrogen) according to the manufacturer's recommendations. The RNA concentrations were measured with a Nanodrop 2000 spectrophotometer (Thermo Scientific). From total extracted RNA, 2 µg were DNase treated using a DNA-free kit as a template (Ambion) to generate first-strand cDNA synthesis using the High-Capacity cDNA Reverse Transcription Kit (Applied Biosystems). Real-time quantitative polymerase chain reaction (qRT-PCR) was performed using TaqMan Universal PCR Master Mix, TaqMan Probes (Applied Biosystems), and the Step OnePlus Real-Time PCR System (Applied Biosystems). All experiments were performed in duplicate, and gene expression levels were normalized to the levels of housekeeping gene GAPDH. The fold change in gene expression was calculated using the 2^{-ΔΔCt} relative quantitation method according to the manufacturer's guidelines (Applied Biosystems), and data are reported as the geometric mean (SEM). qRT-PCR experiments were performed in compliance with the MIQE (Minimum Information for Publication of Quantitative Real-Time PCR Experiments) guidelines (http:// www.rdml.org/miqe).

### Example 1.7. Statistical analysis

The sample size of the current study was calculated to detect differences for methylation levels taking into account published values of epigenome-wide analysis in the field of obesity. The interventional differences were examined in two independent cohorts. Microarray-based DNA methylation analysis was performed in the discovery cohort (n = 10 patients; 3 paired samples/patient), and then the identified genes were validated in an independent cohort of patients (validation cohort; n = 18 patients; 3 paired samples/patient). Finally, the association between DNA methylation level of the identified CpG sites and the anthropometric or biochemical parameters was assessed in the global cohort of patients included in this study (n = 28). The methylation level of each cytosine was expressed as a β value, which was calculated as the fluorescence intensity ratio of the methylated to the unmethylated version of the probe. β values ranged between 0 (unmethylated) and 1 (completely methylated) according to the combination of the Cy3 and Cy5 fluorescence intensities. Color balance adjustment and normalization were performed to normalize the samples between the two-color channels using Genome Studio Illumina software (V2010.3). Genome Studio normalizes data using different internal controls that are present on the Infinium MethylationEPIC BeadChip. This software also normalized data depending on internal background probes. β values with detected p-values > 0.01 were considered to fall below the minimum intensity and threshold, and these CpGs were consequently removed from further analysis. Additionally, probes that contained single nucleotide polymorphisms (SNPs) at the 10 bp 3' end of the interrogating probe were filtered out. To identify consistent patterns of DMCpGs due to the nutritional intervention, a linear model was fitted using a B-spline approximation. The three linear models were fitted: Model 1 was fitted by including the three points of the nutritional intervention to evaluate the general effect of VLCKD; Model 2 including baseline and maximum ketosis to evaluate the effect of ketosis and weight loss; Model 3 including methylation levels at maximum ketosis and endpoint to evaluate the effect of only weight loss, without ketosis. P values were adjusted for multiple comparisons using the false discovery rate (FDR) procedure of Benjamini and Hochberg, and results were considered statistically significant when FDR < 0.10. Additionally, we applied a threshold for the significant sites based on the mean difference between visits with a minimum β value change of ±0.02. Euclidean cluster analysis of significant CpGs was performed using a heatmap function. The global methylation level was compared between the nutritional intervention visits by univariant ANOVA and a Bonferroni post-hoc analysis. All of the aforementioned statistical analyses were performed using R software (version 3.2.0). To estimate enrichment in biological processes, a hypergeometric test was performed using the GOstats package on the biological processes defined by gene ontology (GO). This analysis detected significant over-representation of GO terms in one set (i.e., list of identified genes) with respect to the entire genome. GO terms with an adjusted p-value < 0.05 were considered significant. With SPSS version 21.0 software (SPSS Inc., Chicago, IL) for Windows XP (Microsoft, Redmond, WA), the genomic distribution of the differentially methylated CpGs was compared with the distribution of the CpGs from all analyzed sites on the Infinium MethylationEPIC BeadChip. P values were computed using the chi-square test to determine over- or under-representation of the CpGs. The potential association between anthropometric or biochemical parameters and DNA methylation levels (β-values) was evaluated using the Spearman coefficient test. Differences in DNA methylation levels and expression of the identified genes during the time-course of the intervention and between the nutritional intervention visits were assessed by the non-parametric tests, Kruskal Wallis and Mann-Whitney U, respectively. P ≤ 0.05 was considered statistically significant.

### Example 2. Results

### Example 2.1. Patient Characteristics

Samples from a total of 21 patients who followed the nutritional intervention based on a VLCKD were compared with samples from 12 healthy volunteers with normal weight and evaluated in this study. We first evaluated the discovery cohort (n = 10 participants with obesity who followed a VLCKD+DHA (n=5 women) and n = 12 (6 women) volunteers with normal weight). An extended validation cohort composed of 11 patients (7 women) with obesity that followed a VLCKD+DHA or a VLCKD-DHA was also analyzed. Statistically significant differences were not observed between either cohort in age, gender, height, body weight, BMI, waist circumference, ketosis, or the response to weight loss treatment **(Table 1).** Differences statistically significant were only detected in body weight, BMI and waist circumference between patients with obesity and subjects with normal weight **(Table 1).** All patients lost weight after nutritional intervention (21.8±4.9%), together with reductions in BMI (21.9±5.1%) and waist circumference (19.3±4.4%).

### Example 2.2. DNA methylation changes during the global VLCKD intervention

DNA methylation profiles of blood leukocytes involving approximately 850 thousand CpGs were analyzed after VLCKD intervention. This analysis revealed statistically significant differences (cut-off point Δ ≥ 0.02; FDR ≤ 0.10) at 988 CpG sites, from a total of 739,222 valid CpGs **(Table S2).** The differentially methylated CpGs were mostly characterized as changes towards CpG hypomethylation occurring after nutritional intervention, in both total DMCpGs **(****Figure 1A****)** and in the DMCpGs located in promoters, and islands or shores **(****Figure 1B**). This result of global hypomethylation was correlated with the downregulation in the expression of DNA methyltransferase (DNMT) 1 and the *de novo* methyltransferases DNMT3A and DNMT3B **(****Figure 1C****).** The identified CpG sites mapped to 786 unique genes and we were able to separate the samples according to nutritional intervention visits using a hierarchical cluster approach **(****Figure 1D**). It should be noted that the methylation levels of the 988 CpG sites differentially methylated after nutritional intervention were altered to resemble methylation levels observed in samples from subjects with normal weight **(****Figure 1C****).** The 20 DMCpGs with the highest difference with respect to baseline among genes that are represented by more than 2 CpGs are represented in **Table 2.** Regarding the functional distribution **(****Figure 2****),** the differences in DNA methylation were mainly observed in open sea regions **(****Figure 2A****),** with 43.2% of the DMCpG sites located in promoter regions and the majority of DMCpGs in the body **(****Figure 2B****).** Moreover, the DMCpGs were mainly found in chromosomes 2, 9, 16, 17, 19, and 22 when compared with all CpGs analyzed **(****Figure 2C****).** Among the 988 DMCpGs, we found 886 (89.7%) with decreased and 102 (10.32%) with increased levels of DNA methylation after nutritional intervention. Moreover, the DMCpGs that lost methylation with respect to baseline were located mainly in the open sea **(****Figure 2D****)** and body **(****Figure 2E****).** In contrast, the DMCpGs that gained methylation after the nutritional intervention were mostly located in promoters (TSS 200 and 1^{st} exon) **(****Figure 2D****)** and in CpG islands **(****Figure 2E****).** Regarding chromosome distribution, the DMCpGs with decreased methylation levels after nutritional treatment were found on chromosomes 1, 4, 6 7, 8, 9, 14, and 16, whereas DMCpGs with increased methylation levels were mainly found in chromosomes 3, 5, 10, 11, 12, 17, 21, and 22 **(****Figure 2F****).**

### Example 2.3. Biological significance of the dietary intervention-related DMCpG sites and associated genes

Interestingly, most differentially methylated genes belonged to a network significantly enriched in protein interactions (p < 0.001) according to STRING analysis **(****Figure 3A****).** GO analysis was performed to test whether certain molecular functions or biological processes were significantly enriched within the 786 genes associated with the 988 DMCpGs discovered through VLCKD intervention **(****Figure 3B****).** Among the categories of functional processes that exhibited statistical significance (FDR < 0.05), we found processes related to regulation of transcription, signal transduction, cell differentiation, proliferation and apoptosis, metabolic processes, response to hypoxia, protein processing, muscle organ and skeletal system development, nervous system development and axon guidance **(****Figure 3B****).** Among these, we highlighted relevant pathways in the field of obesity physiopathology such as insulin signalling, protein digestion and absorption, adipocytokine signalling and muscle development **(****Figure 4A-D****).** To investigate biological relevance, the CpG sites representing promoter regions (TSS1500, TSS200, 5' UTR and 1^{st} Exon) at CpG islands/shores were selected. This selection yielded 141 CpGs representing 150 unique genes. Based on this filter we identified CpG sites that could be genetic targets whose methylation is associated with VLCKD responses. Among these, the most representative gene was *ZNF331,* which was represented by 2 CpG sites located in the promoter and island with the highest difference with respect to baseline. Furthermore, *FGFRL1* was also selected from among the DMCpG sites because of its biological relevance in metabolic pathways and obesity pathogenesis and because the methylation level of this gene in leukocytes was previously proposed as an episignature that mirrors methylation levels of dysfunctional adipose tissue in obesity.

### Example 2.4. DNA methylation changes associated with the effects of dietary-induced ketosis

An analysis comparing baseline (day 0) with maximum ketosis (day 30) yielded 1365 DMCpGs. With respect to all CpGs analyzed, these CpGs were found mainly in the open sea **(****Figure 5A****)** and body **(****Figure 5B****)** and in chromosomes 1, 10, 11, 17, 19, and 22 **(****Figure 5C****).** The DMCpGs that gained methylation were found in the island and promoter and in chromosomes 1, 2, 3, 4 and 7. The DMCpGs that lost methylation were found in the open sea and body and in chromosomes 9, 10, 11, 12, 17, 21, 22 **(****Figure 5D-F****).** With the aim of isolating the specific effects of ketosis on methylation profile, additional analysis was performed. A Venn diagram was created by including: DMCpGs Baseline - Maximum Ketosis = 1365, DMCpGs Baseline - Endpoint = 405, and DMCpGs Maximum Ketosis - Endpoint = 21. Using these conditions, we identified 280 CpGs whose methylation was affected by the induced weight loss per se (**Table S4**) and 1239 CpGs were identified as VLCKD-induced ketosis-related DMCpGs **(****Figure 6A****).** These VLCKD-induced ketosis-related DMCpGs corresponded to 966 annotated genes, and 161 of these were represented by 137 VLCKD-induced ketosis-related DMCpGs located in the promoter and island or shore (**Table S3**). Among the categories of functional processes that exhibited statistical significance (FDR < 0.05), we found processes related to regulation of transcription, signal transduction, cell adhesion, cell differentiation, proliferation and apoptosis, as well as nervous system development and axon guidance. Moreover, these ketosis-related differentially methylated genes belonged to pathways involved in focal adhesion, insulin and adipocytokine signalling pathways, MAPK and P53 signalling pathways and in cancer and type II diabetes mellitus-related pathways **(****Figure 6B****).** Overall pathways associated with obesity physiopathology. Among these, we highlighted relevant pathways in the field of obesity physiopathology such as insulin signalling, protein digestion and absorption, adipocytokine signalling and muscle development. To identify potentially novel signatures of DNA methylation associated with VLCKD-induced ketosis, those genes with more than 2 CpG sites located within CpG islands and shores and with a difference in β-values ≥ |2|% were selected. Based on these criteria, 12 genes were identified and are listed in **Table 3.** When the list was further filtered by promoter region, the genes *CBFA2T3, C3orf38, JSRP1,* and *LRFN4* showed at least one VLCKD-induced ketosis-related DMCpG located in the promoter and island/shore.

### Example 2.5. Validation of candidate genes in a representative cohort

We used pyrosequencing, a technique that is most feasible for studies of patients in hospitals, to evaluate the DNA methylation levels of *ZNF331* and *FGFRL1* in an independent cohort of samples from leukocytes (validation cohort; n = 18 patients who follow a VLCKD+DHA or VLCKD-DHA merged **Table 1**). Statistically significant differences were found in the methylation levels after nutritional intervention with respect to baseline **(****Figure 7A****).** After weight loss treatment, a statistically significant increase with respect to baseline was observed during maximal ketosis in the 4 CpGs evaluated (p = 0.049). However, no statistically significant changes were observed in *FGFRL1* methylation levels after the weight loss treatment **(****Figure 7B****).** DNA methylation has previously been associated with transcriptional repression. Accordingly, the expressions of *ZNF331* **(****Figure 7C****)** and *FGFRL1* **(****Figure 7D****)** were evaluated. A statistically significant downregulation in the expression of ZNF331was found after nutritional intervention, while the expression of *FGFRL1* was upregulated, especially at maximum ketosis compared with baseline. Even though the results for methylation level of *FGFRL1* were not validated in the independent cohort, the expression of this gene was inversely correlated with the methylation levels observed in the discovery cohort. Overall, these results suggest that epigenetic regulation of *ZNF331* and *FGFRL1* related to weight loss could have a relevant biological function. The validation part of the current study was performed by merged the two arms of the clinical trial because the initial results revealed that both arms induced similar weight loss and ketosis, the main outcome used to evaluate changes in the methylome. Indeed, a further analysis in the validation cohort keeping the two arms separated, showed that the trend in the methylation levels through the intervention was like that observed in the merged analysis for the studied genes, ZNF331 and FGFRL1. Differences through the intervention appeared to be higher in the VLCKD+DHA than in the VLCKD arm; however, a repeated measured ANOVA analysis depending on arm showed statistically significance only for the time-course of the intervention in ZNF331 methylation levels (p<0.01), without statistically significance for the interaction between time x arm, nor between the two arms (p>0.05).

### TABLES

### CHR, chromosome; FDR, false discovery rate

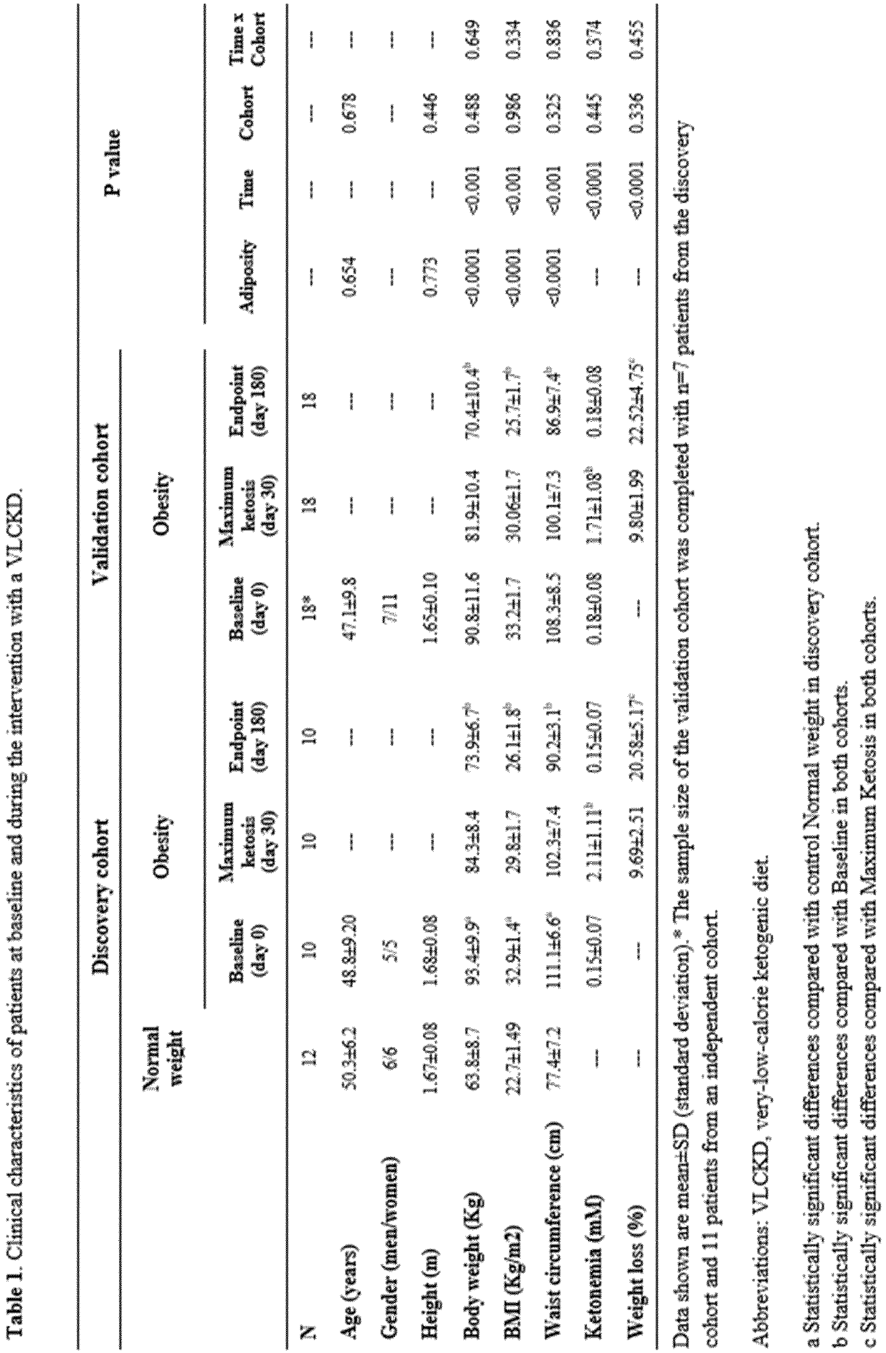

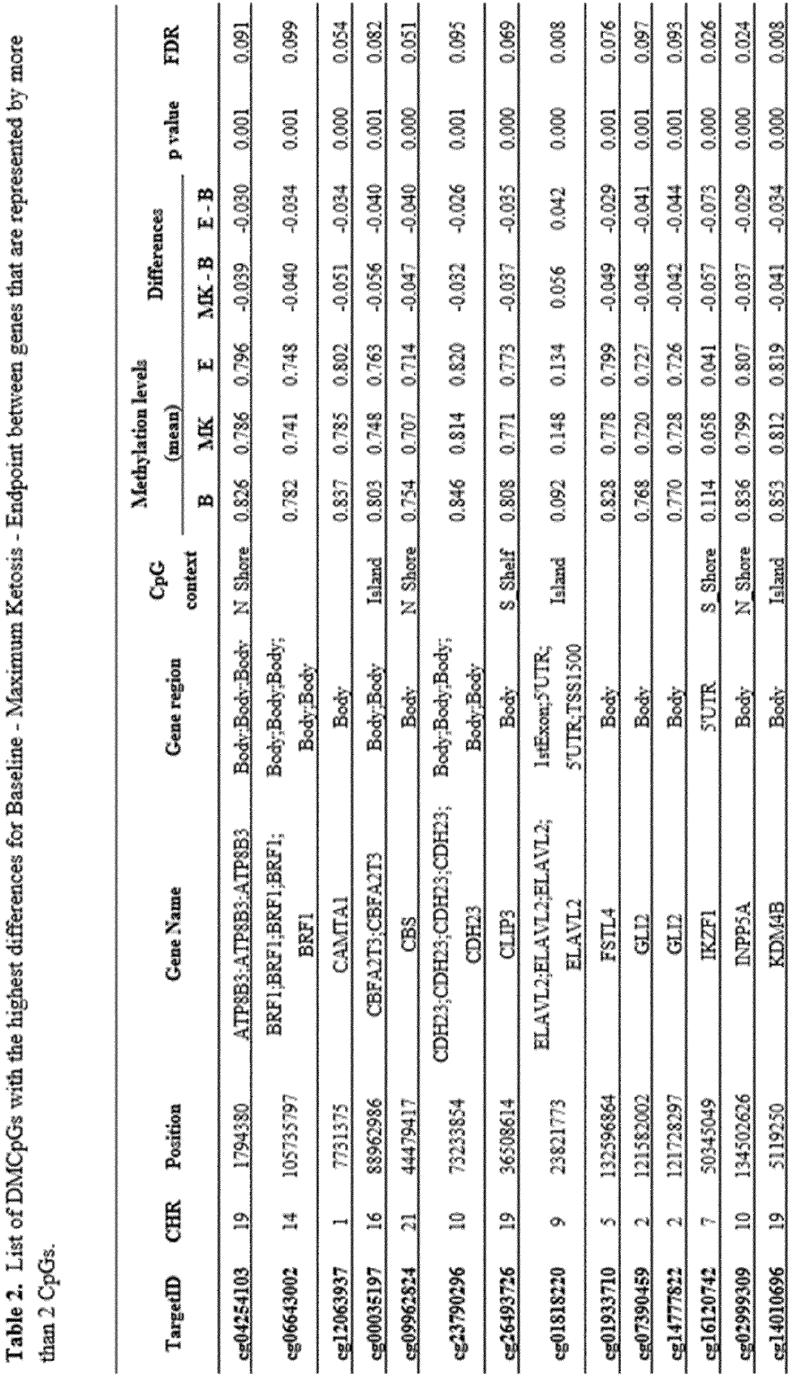

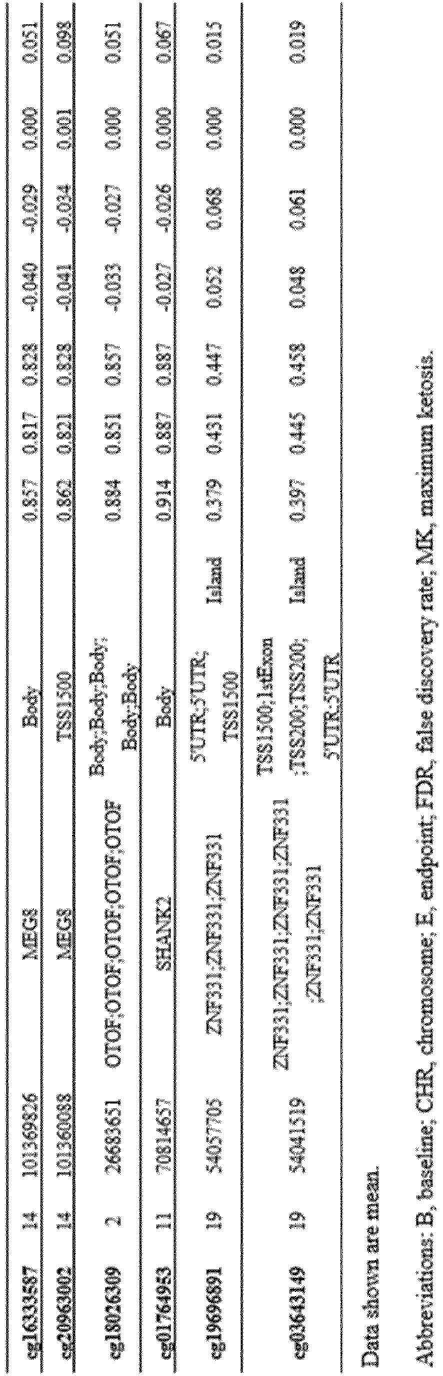

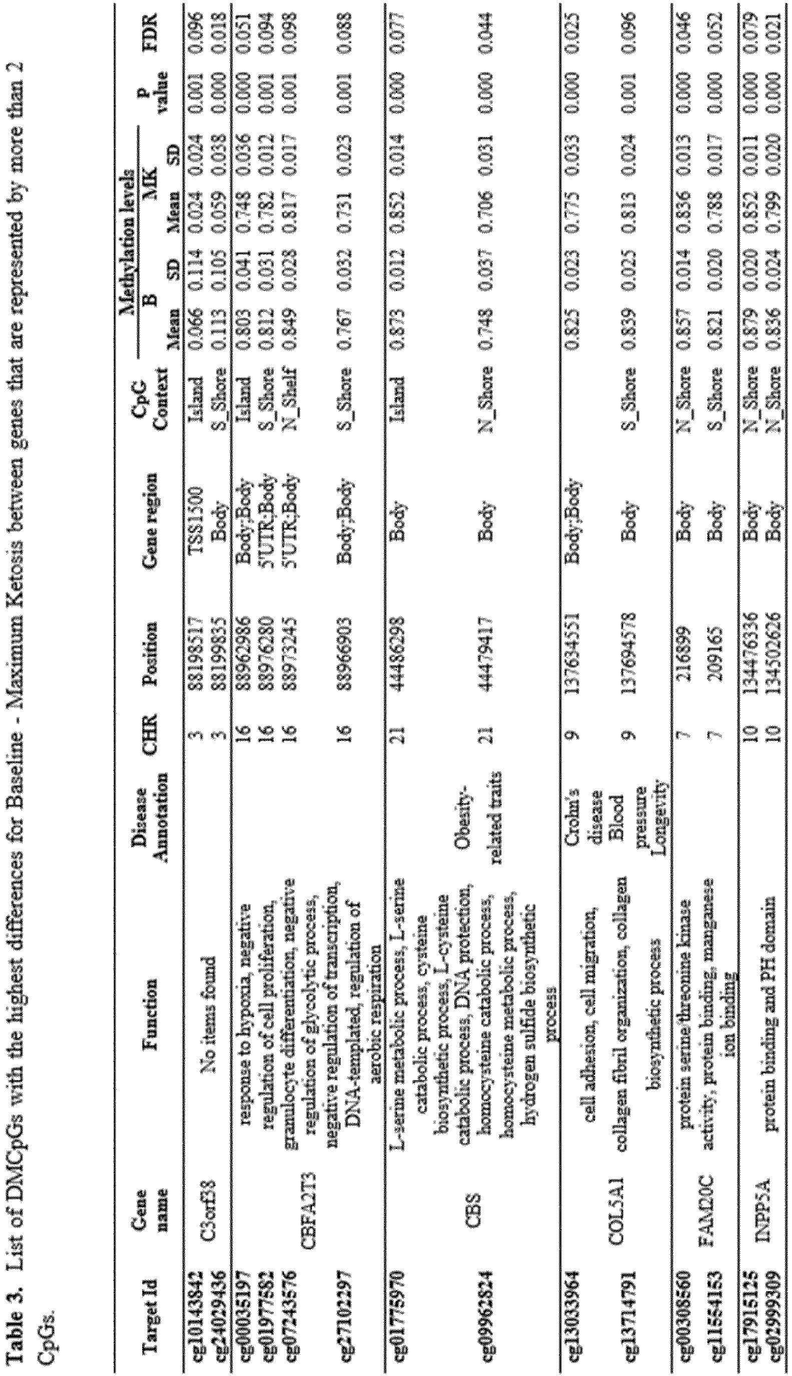

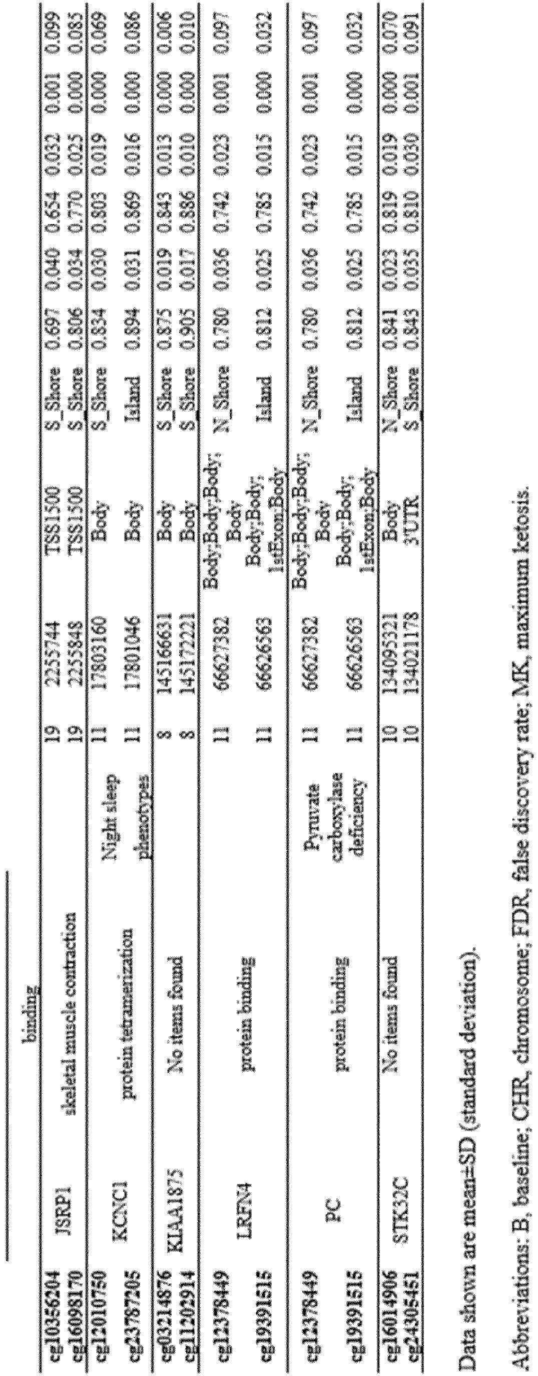

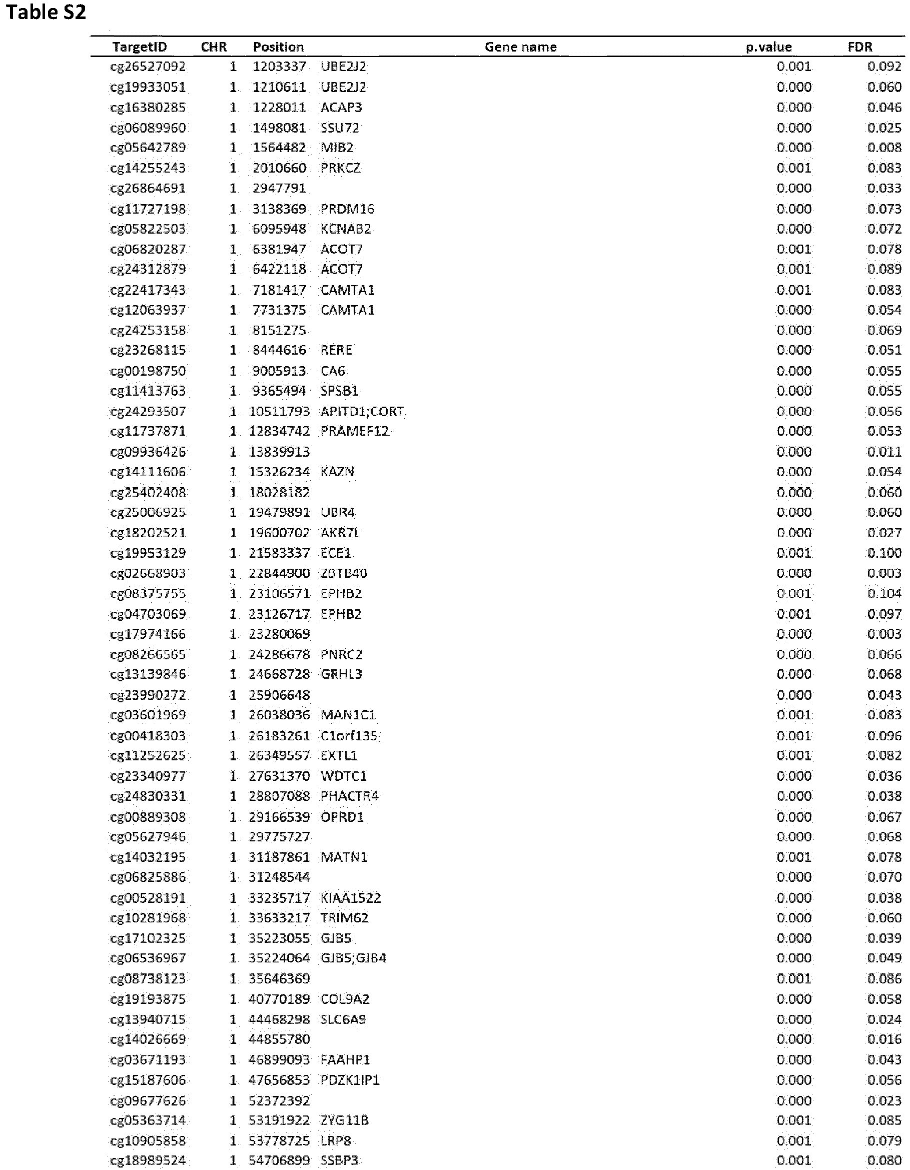

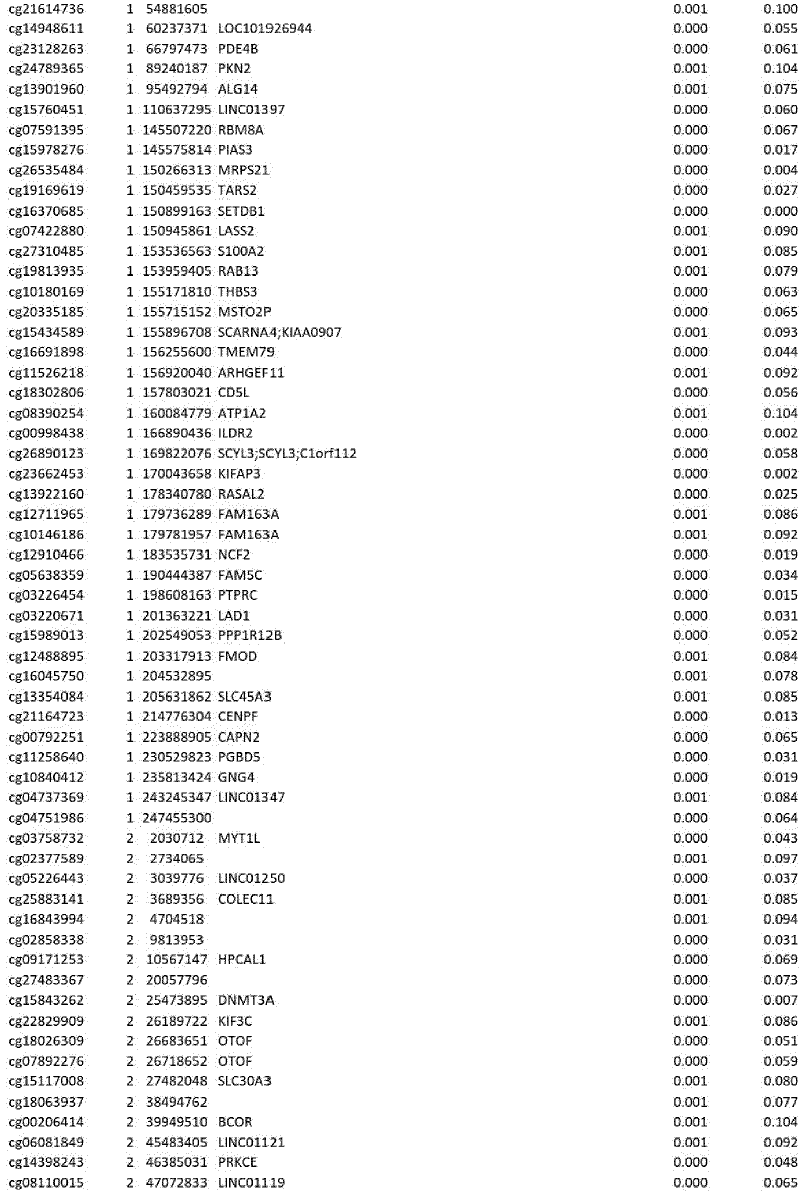

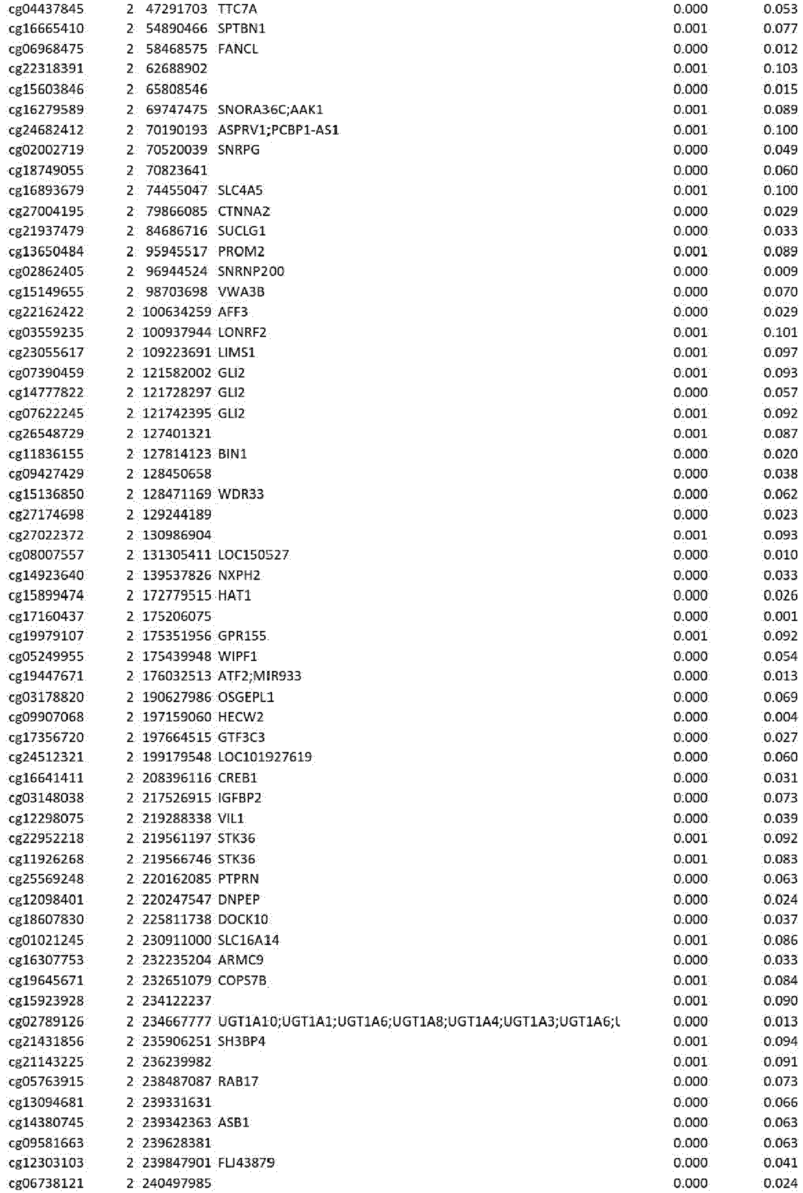

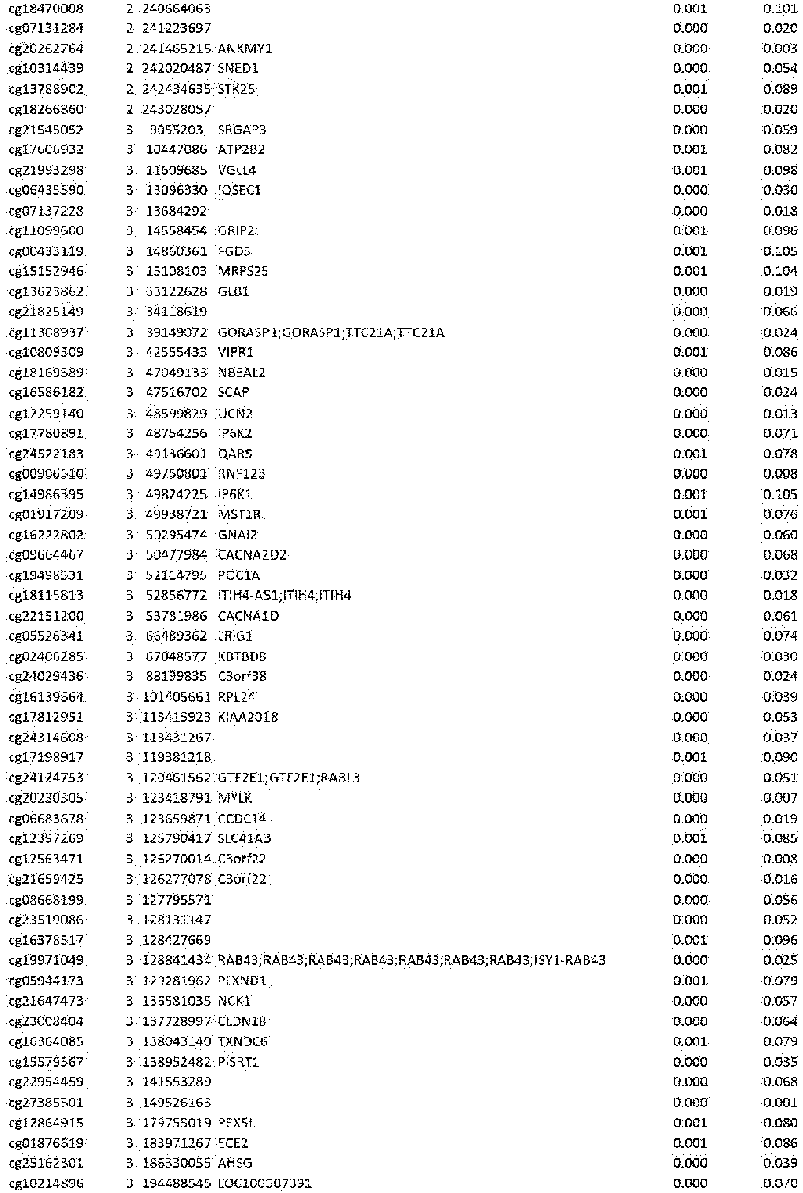

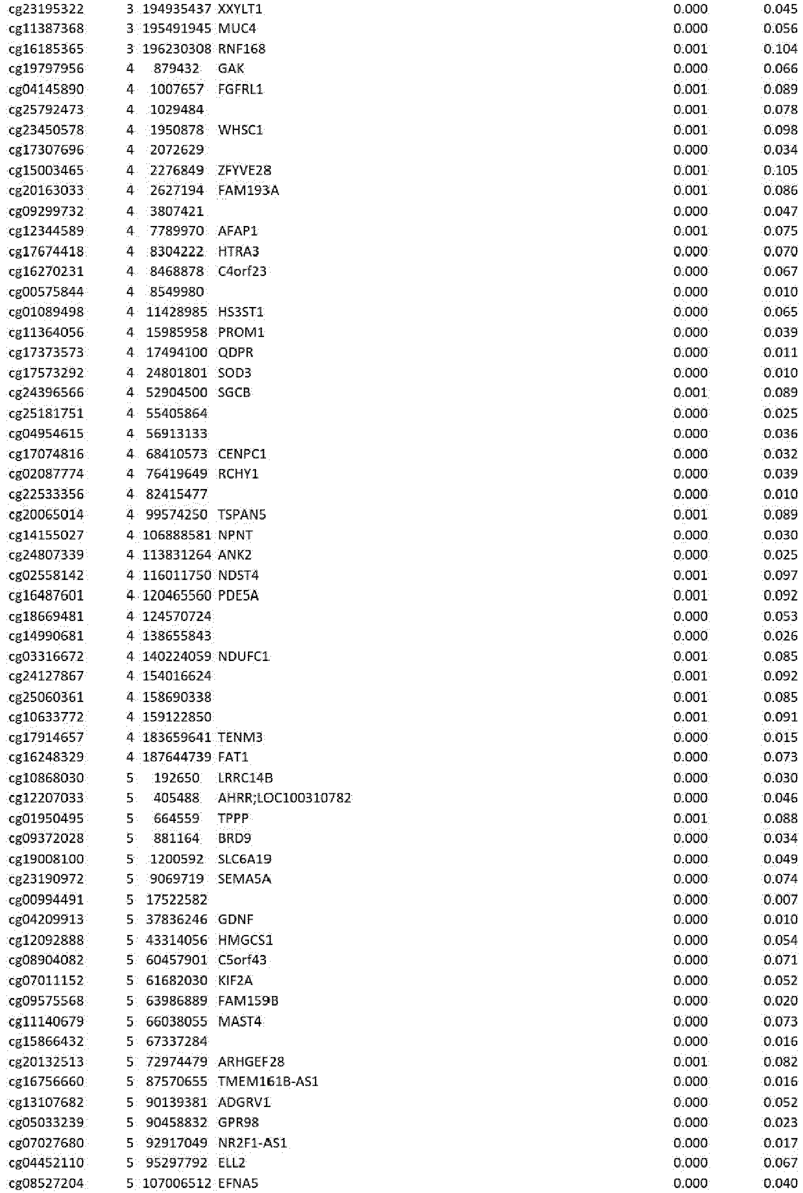

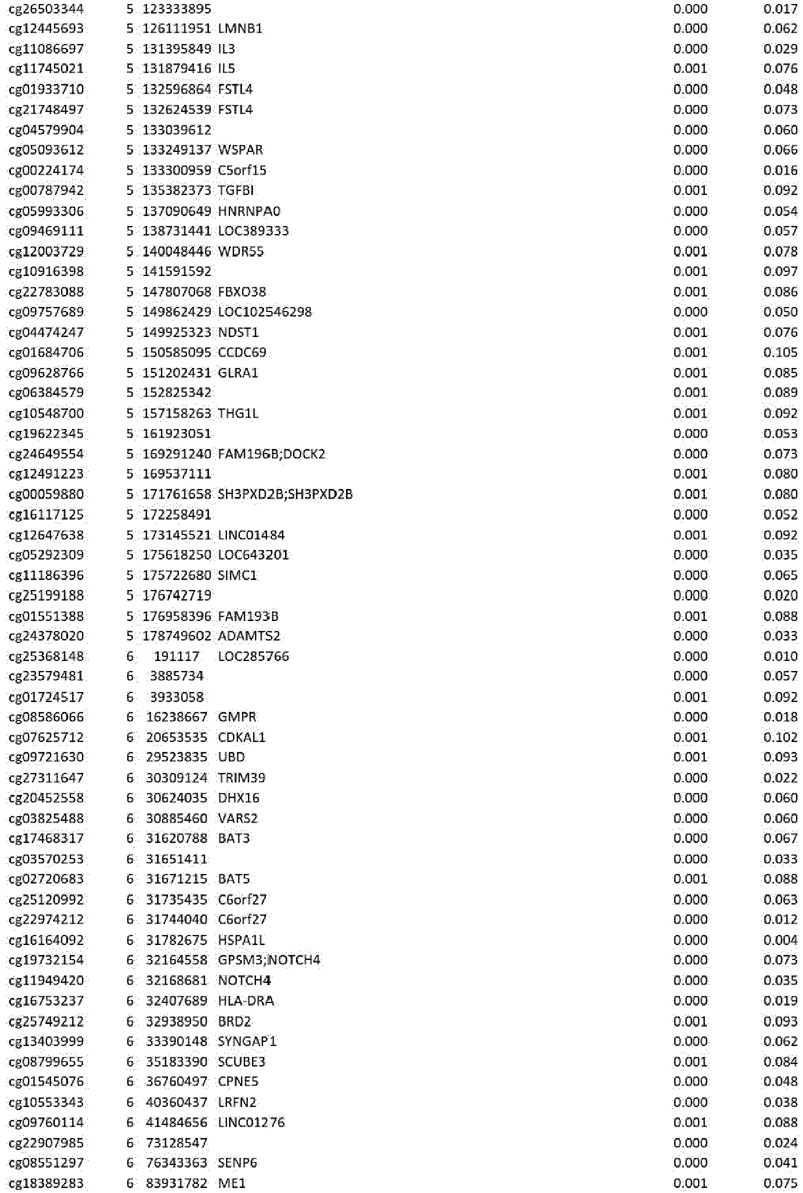

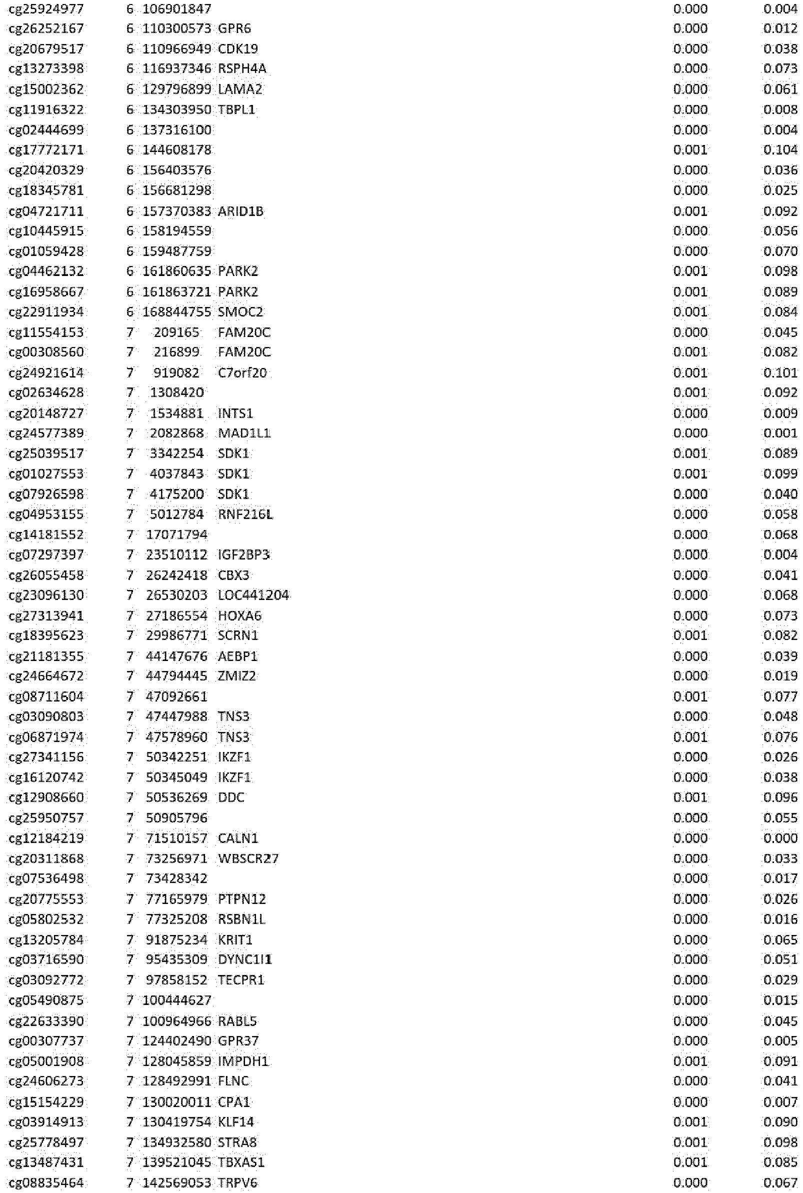

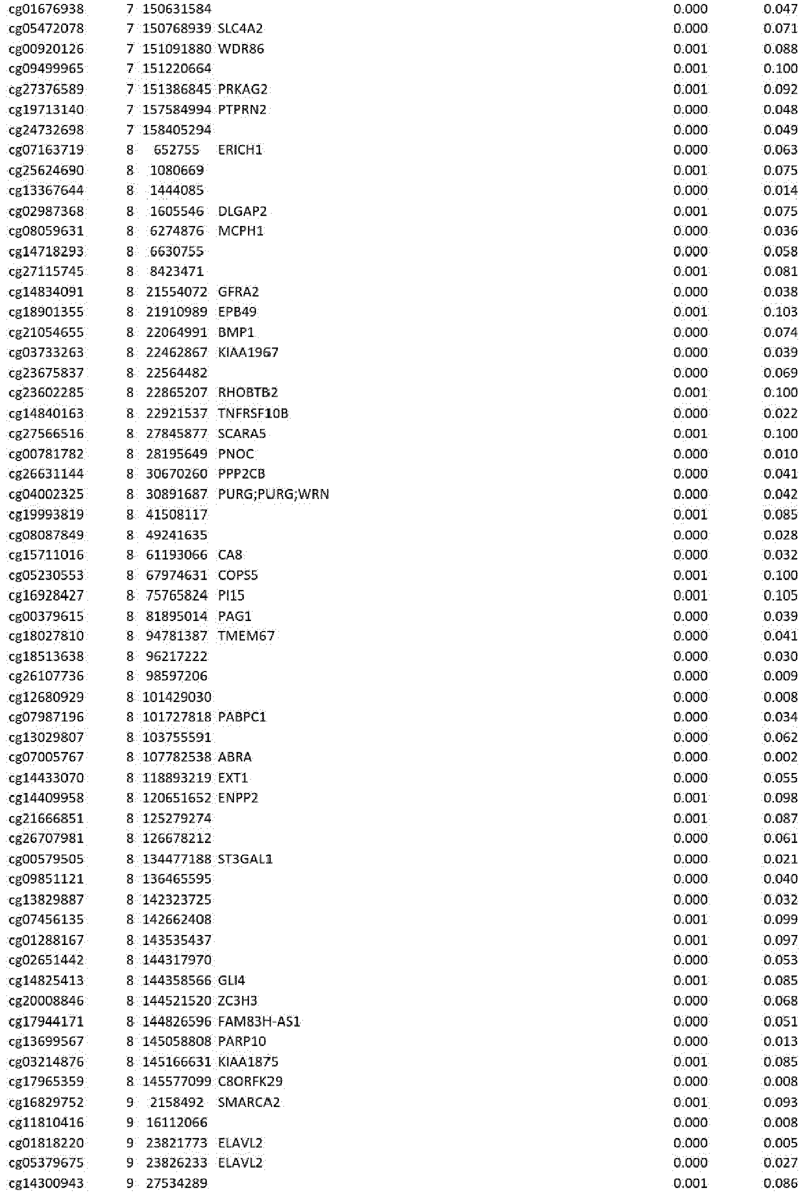

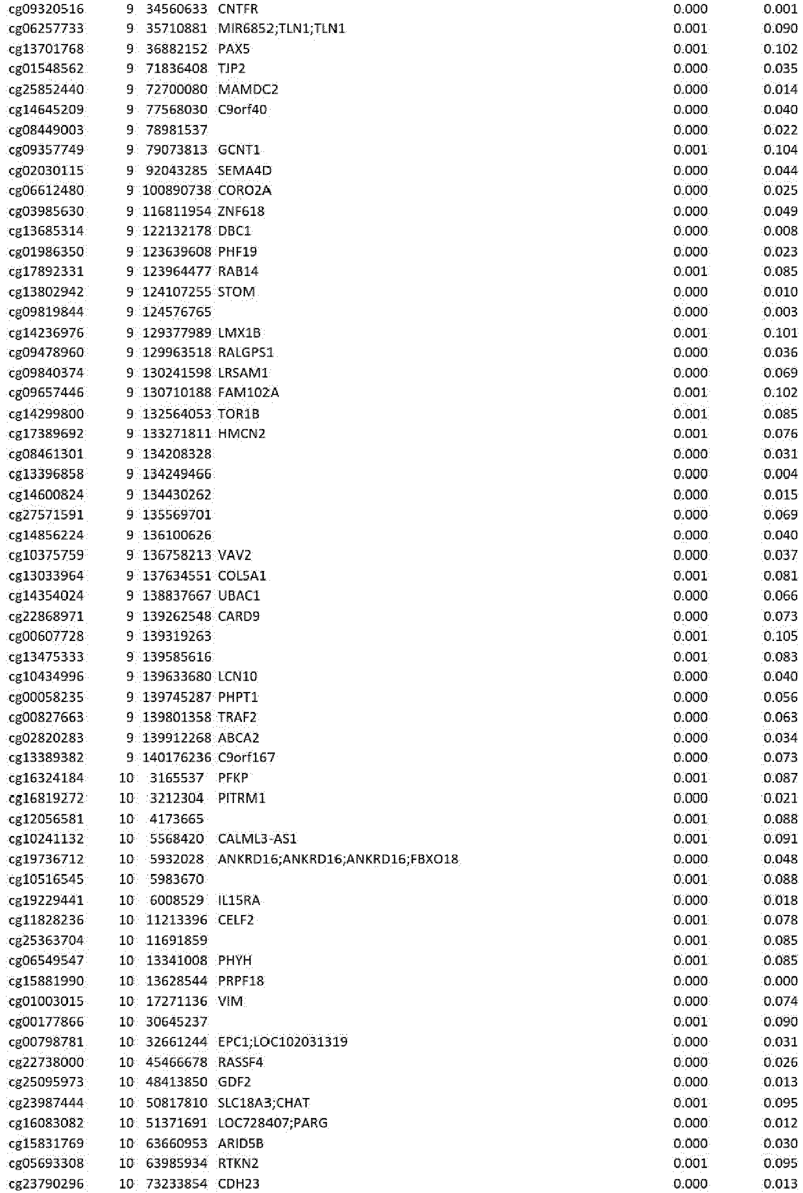

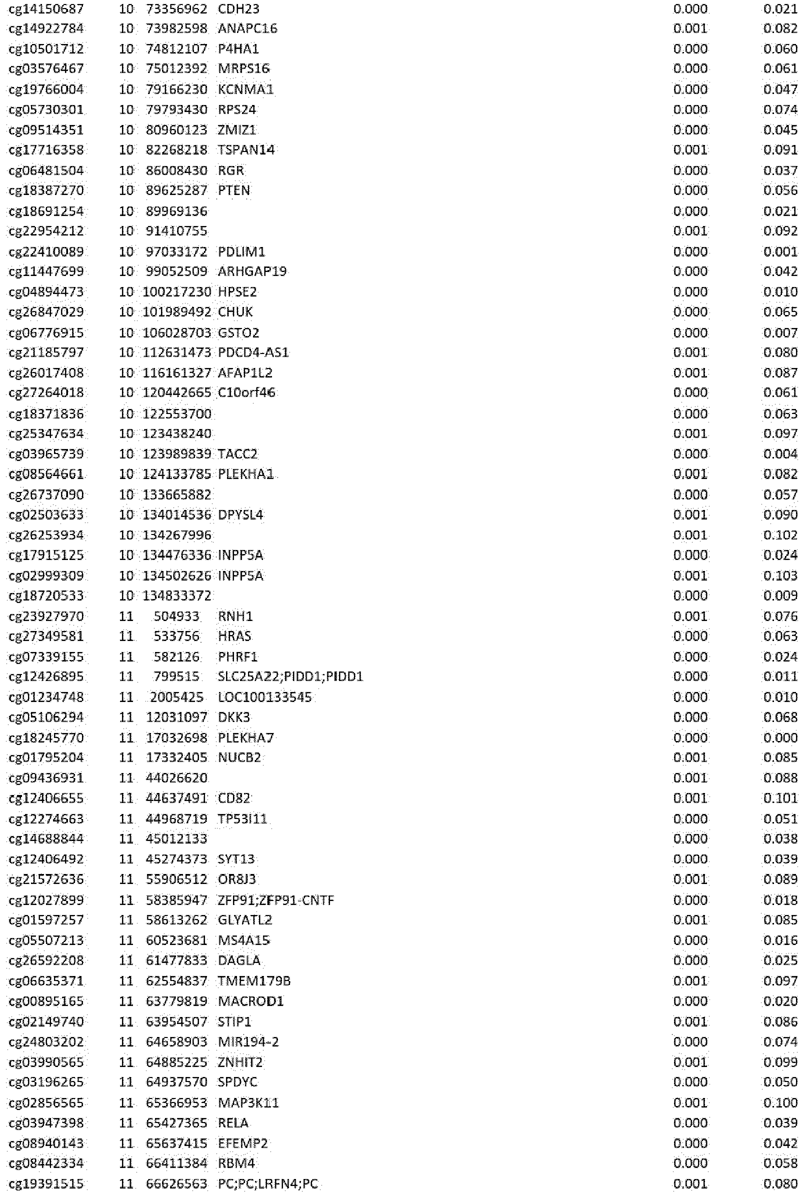

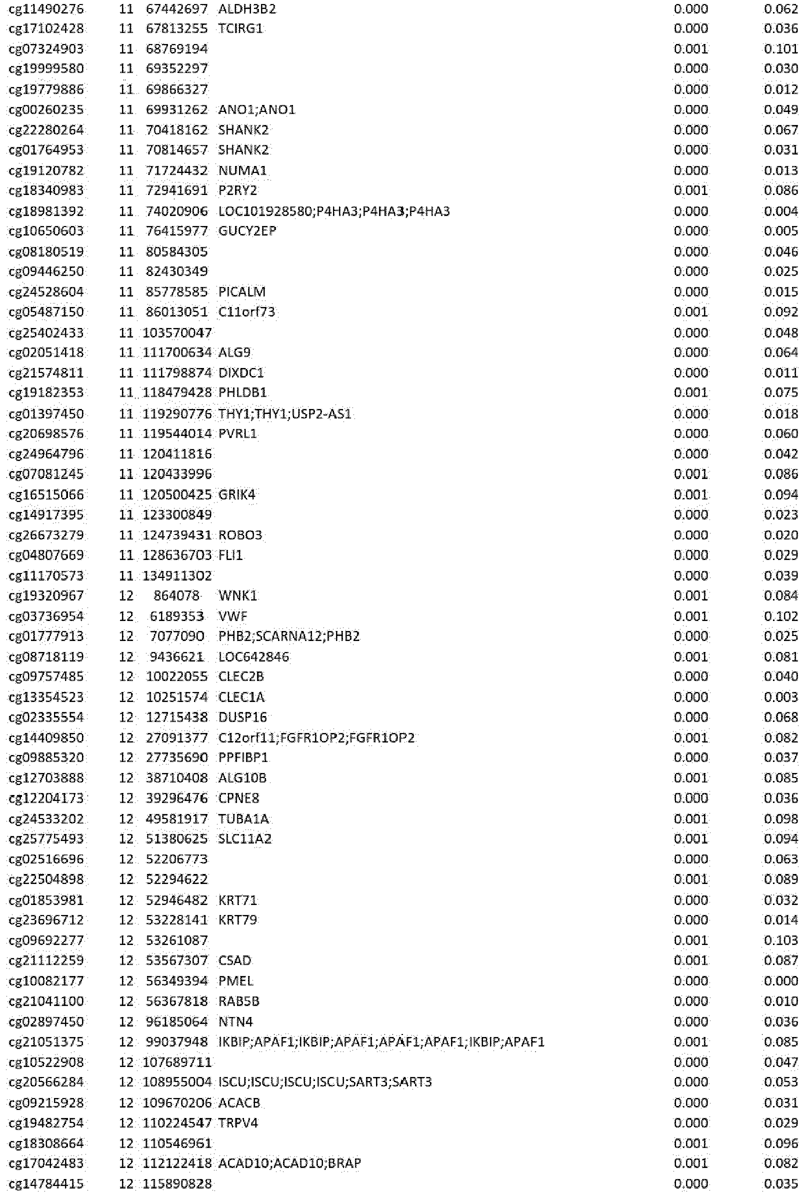

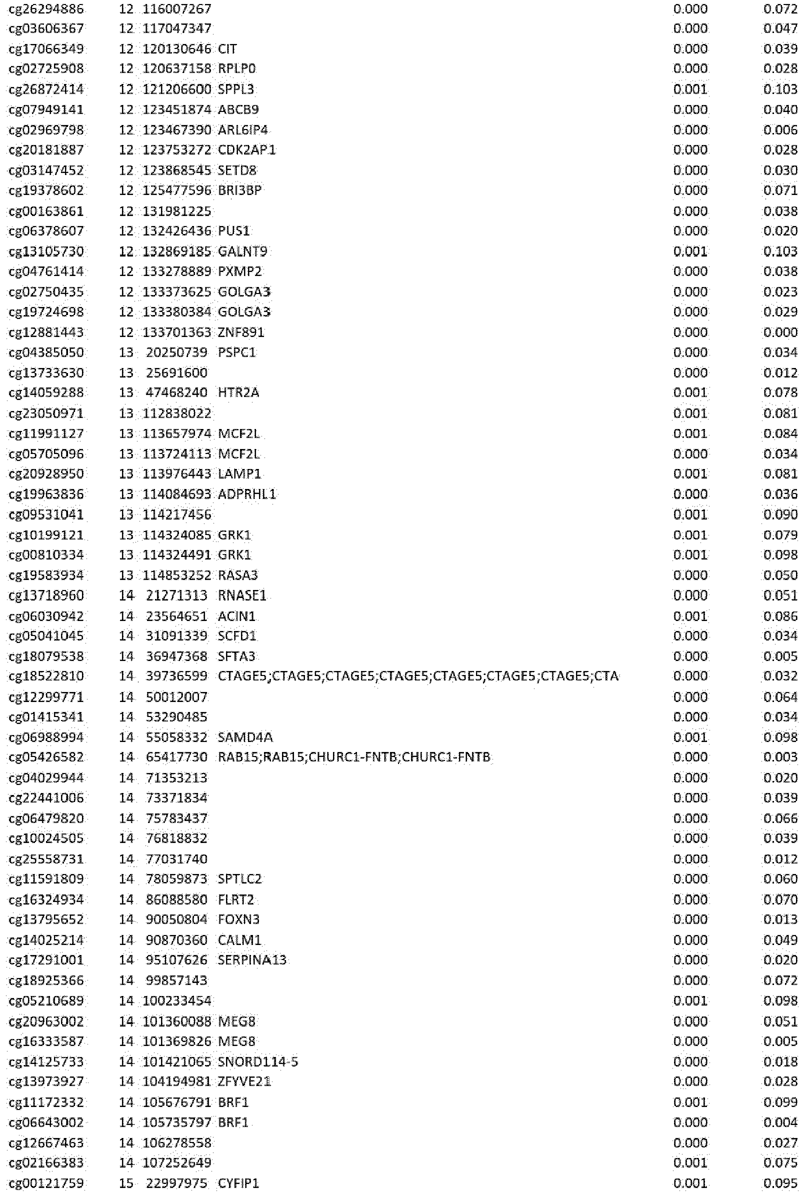

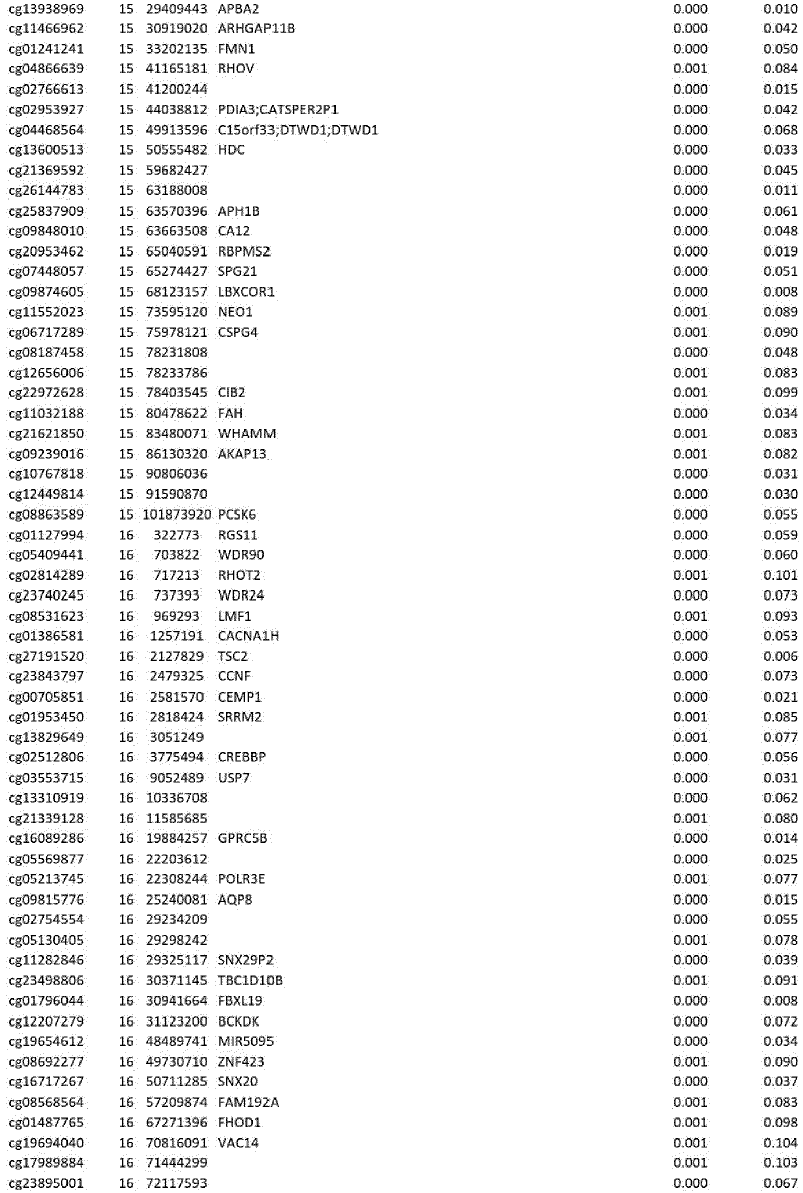

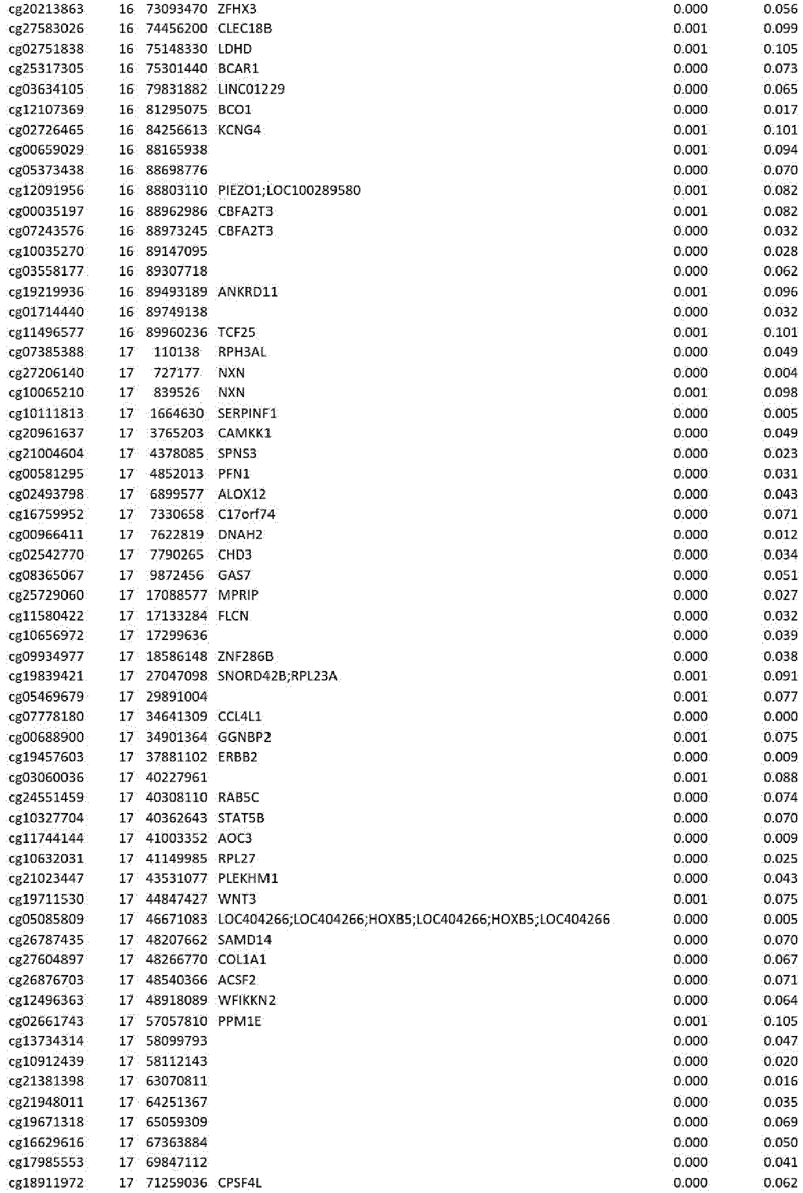

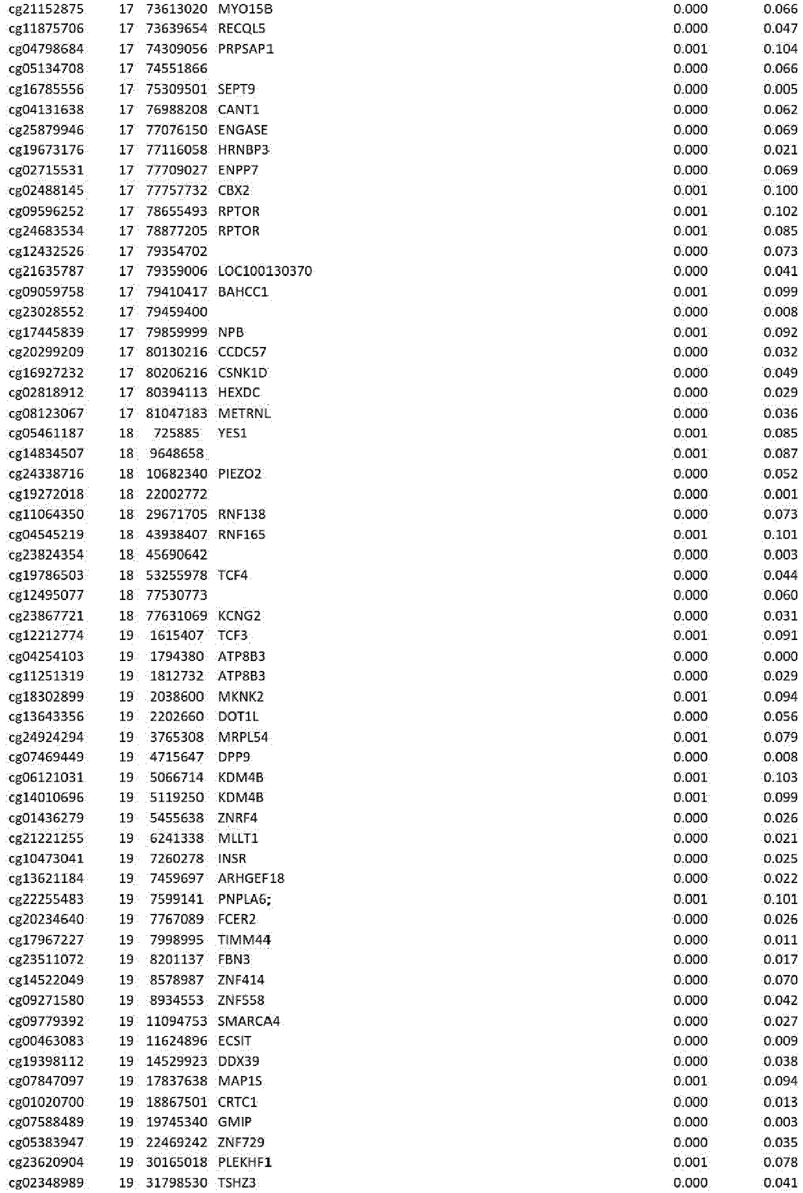

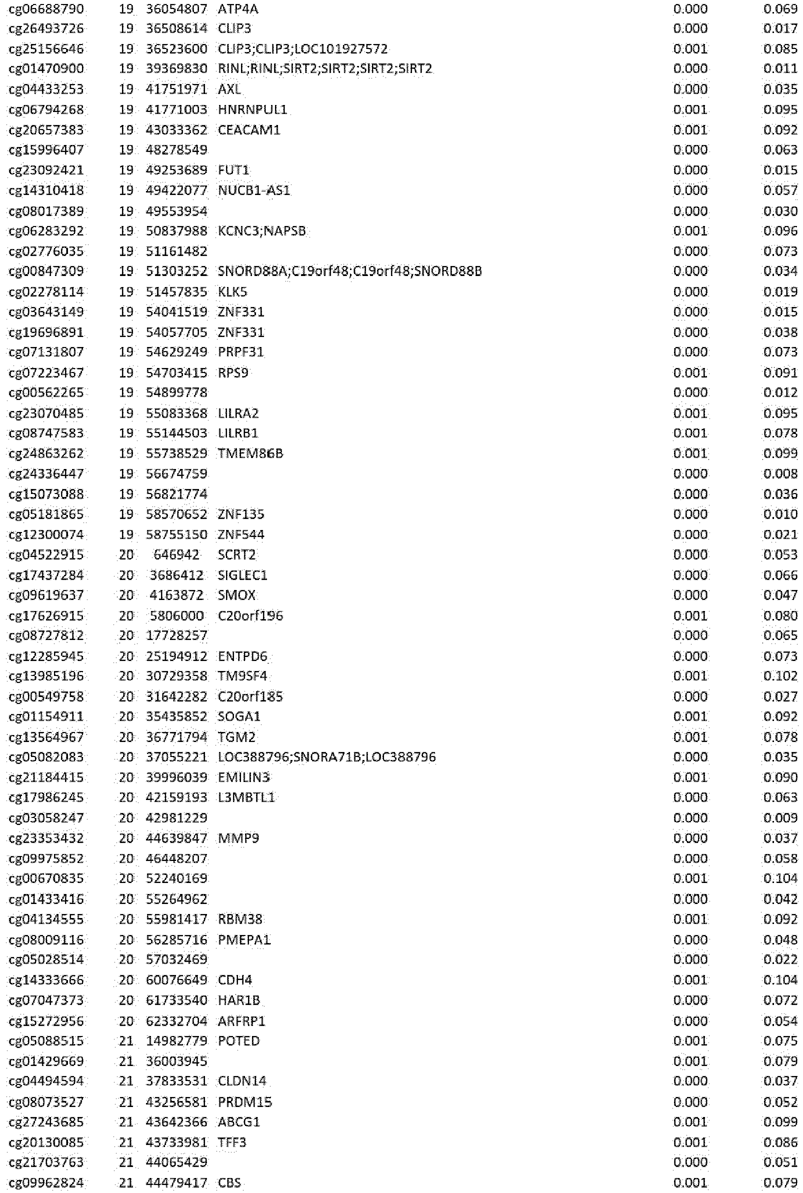

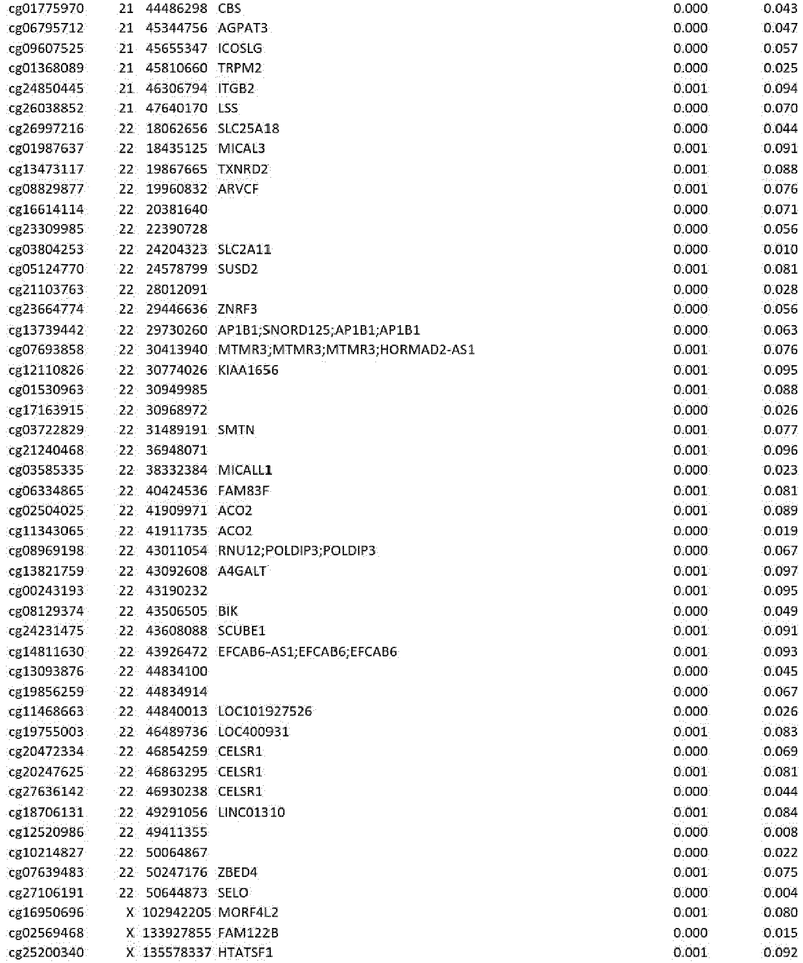

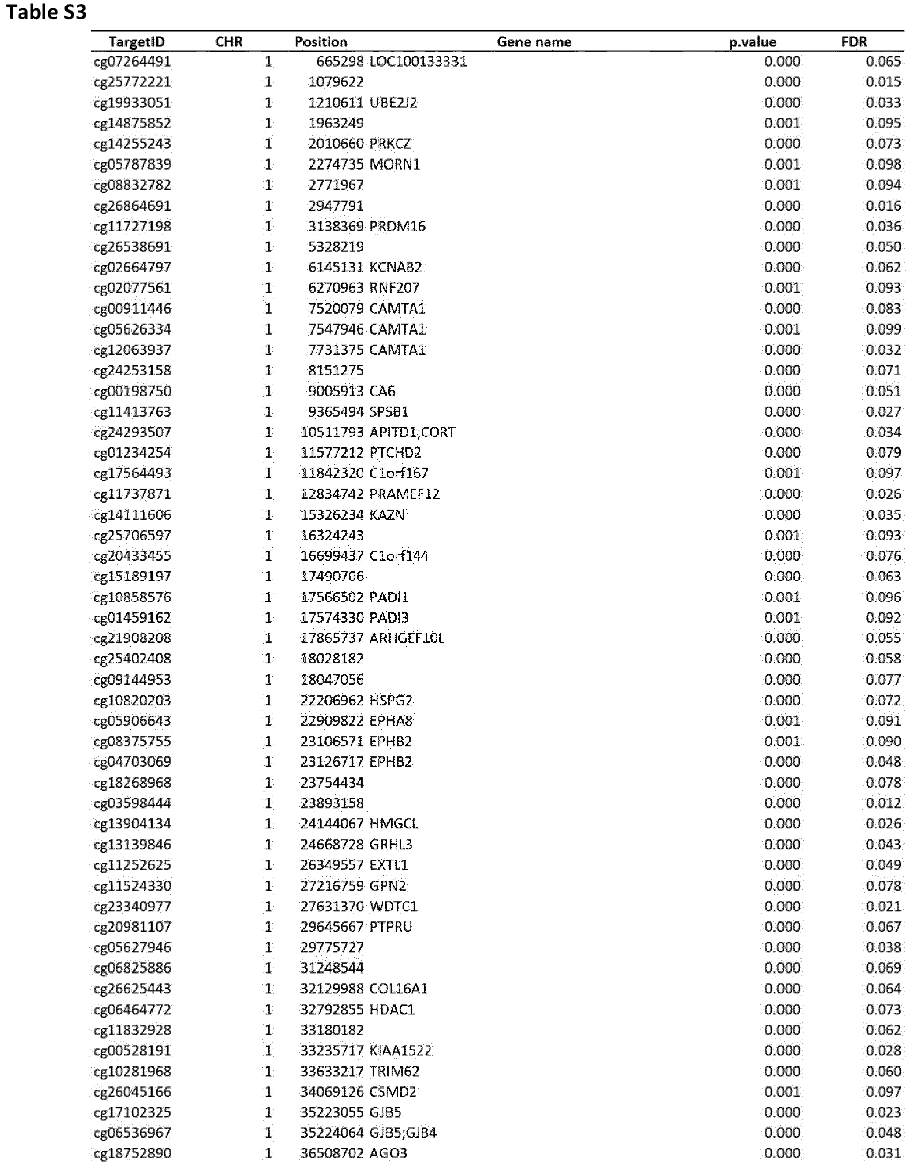

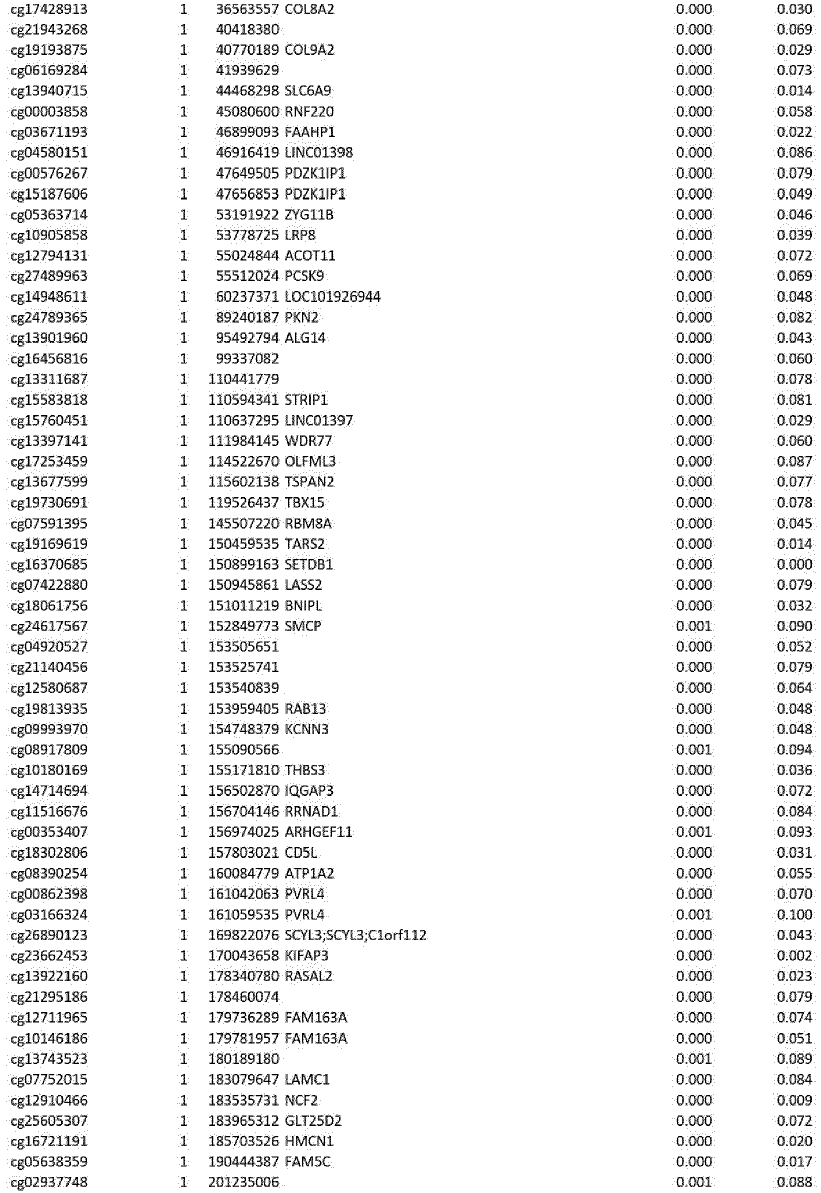

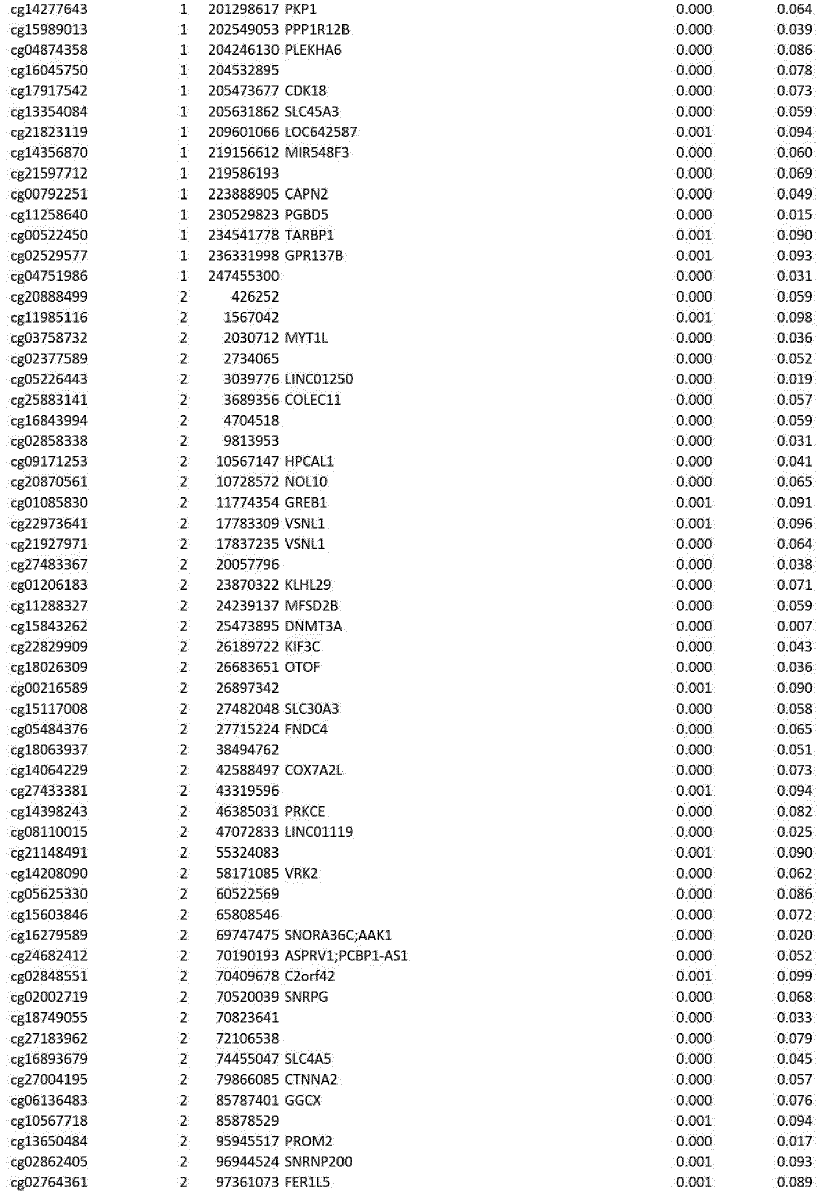

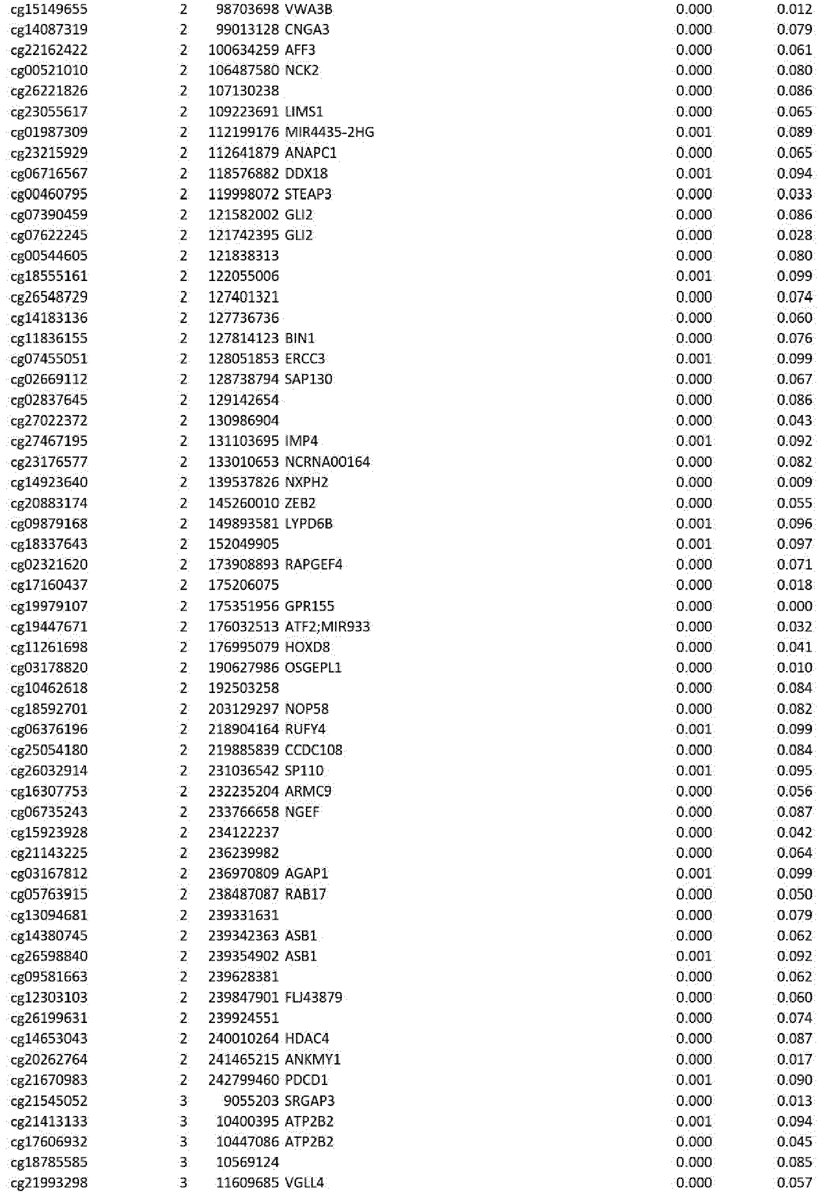

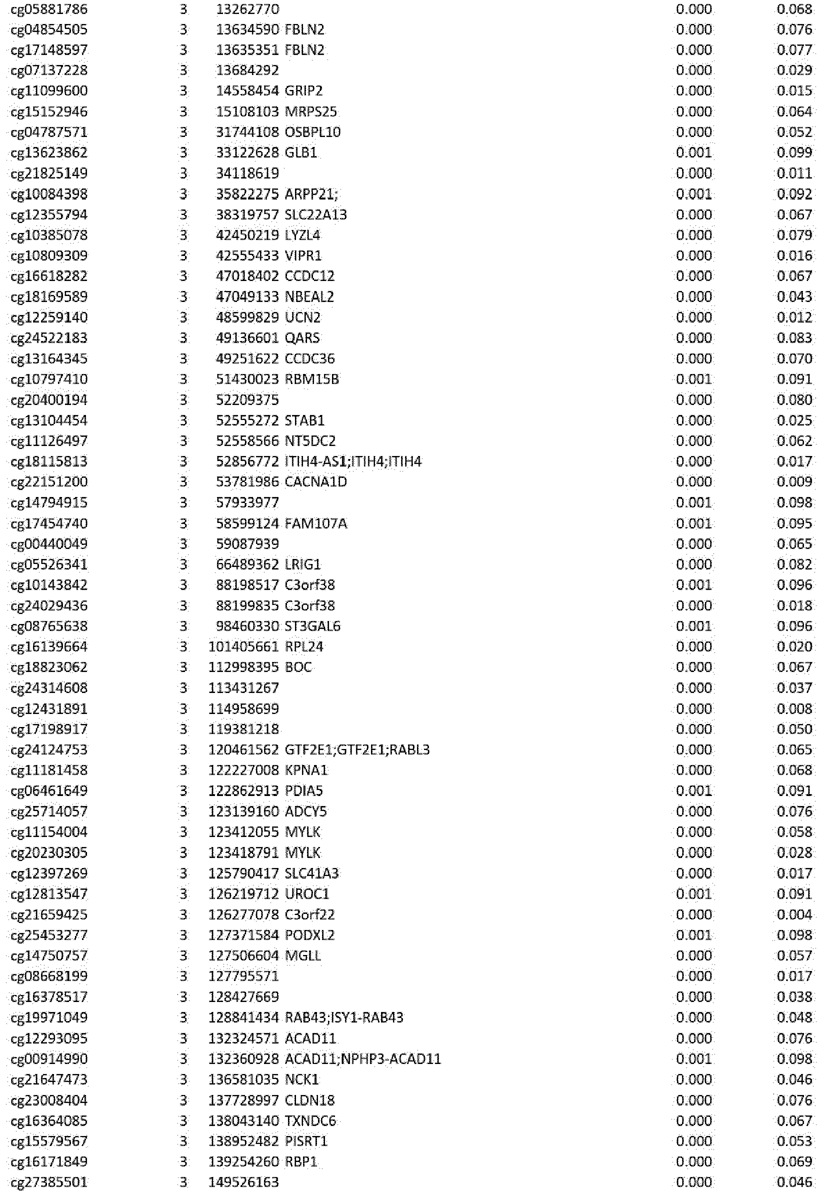

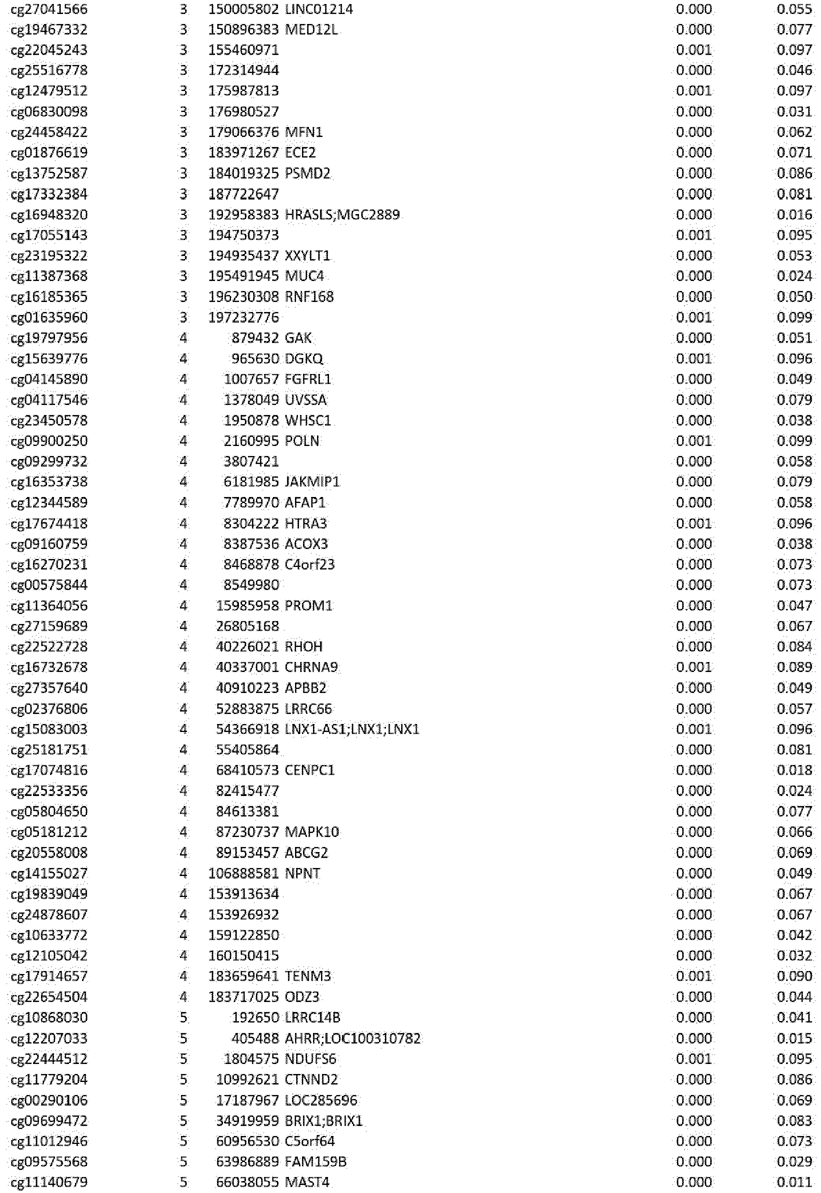

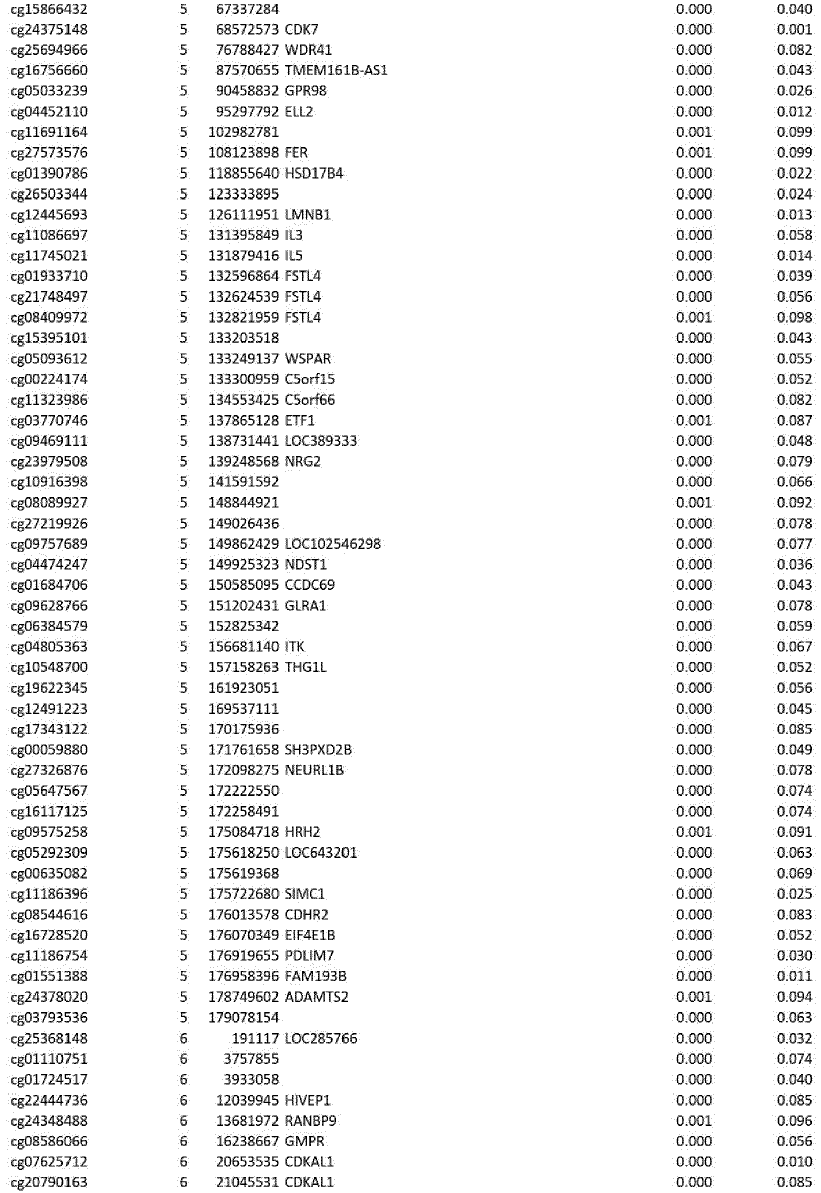

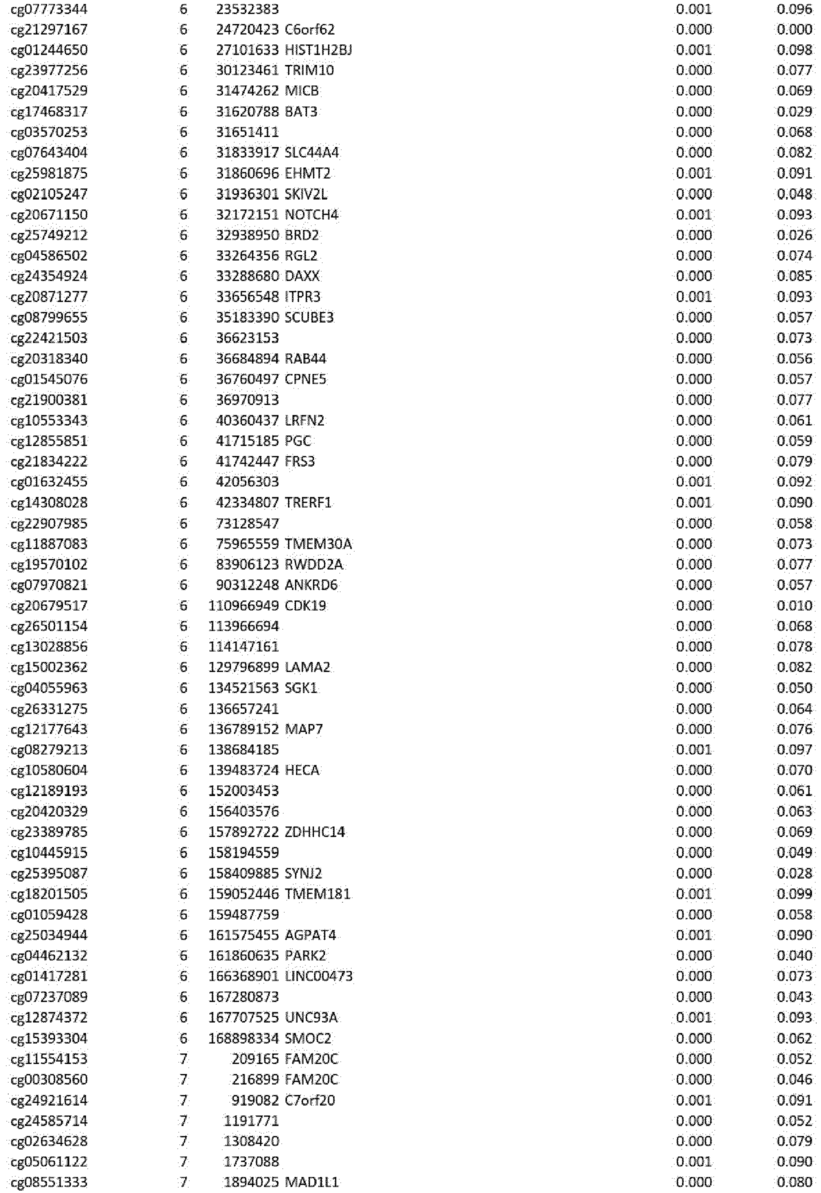

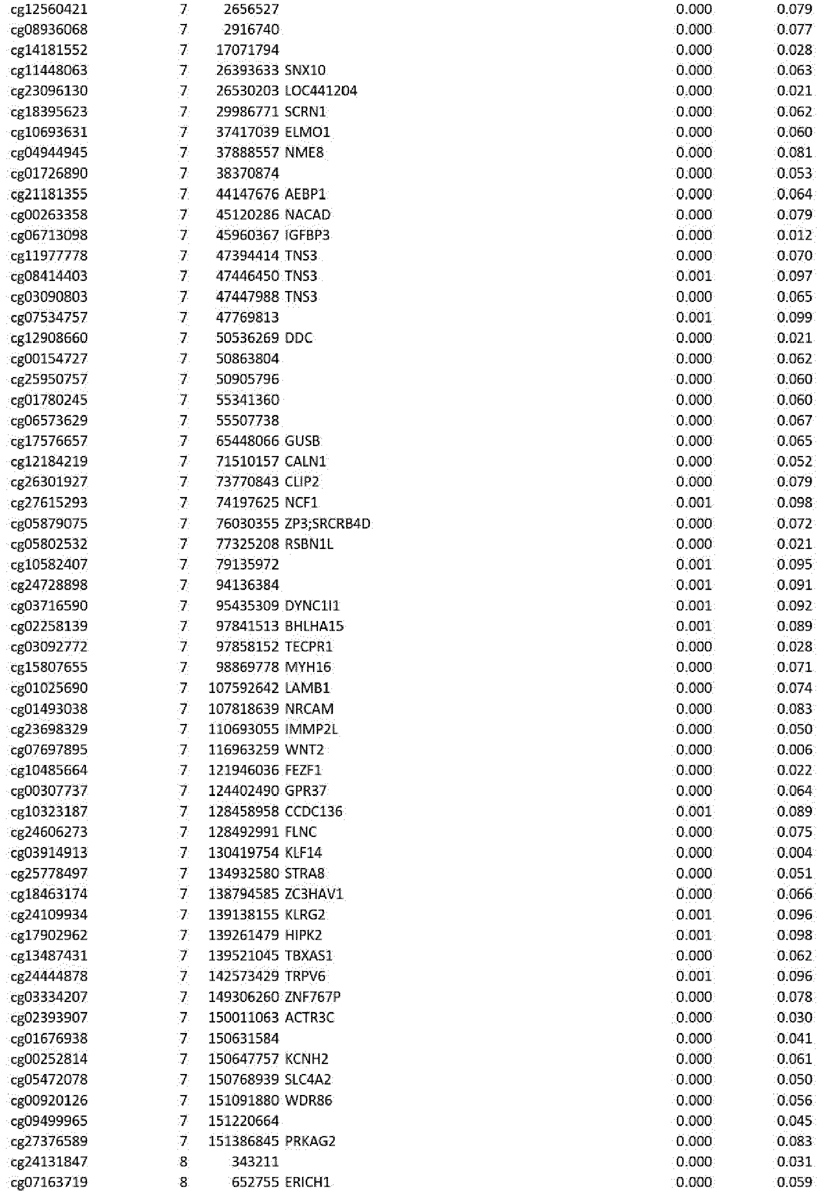

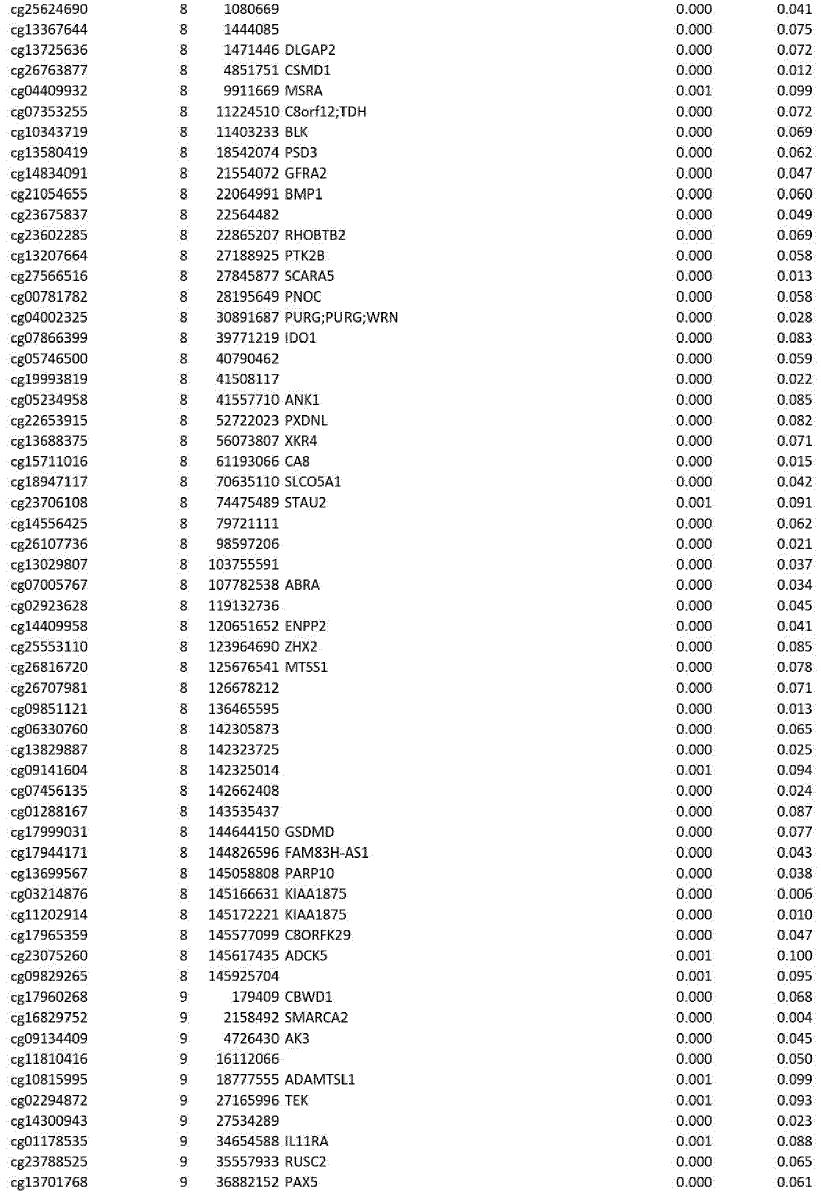

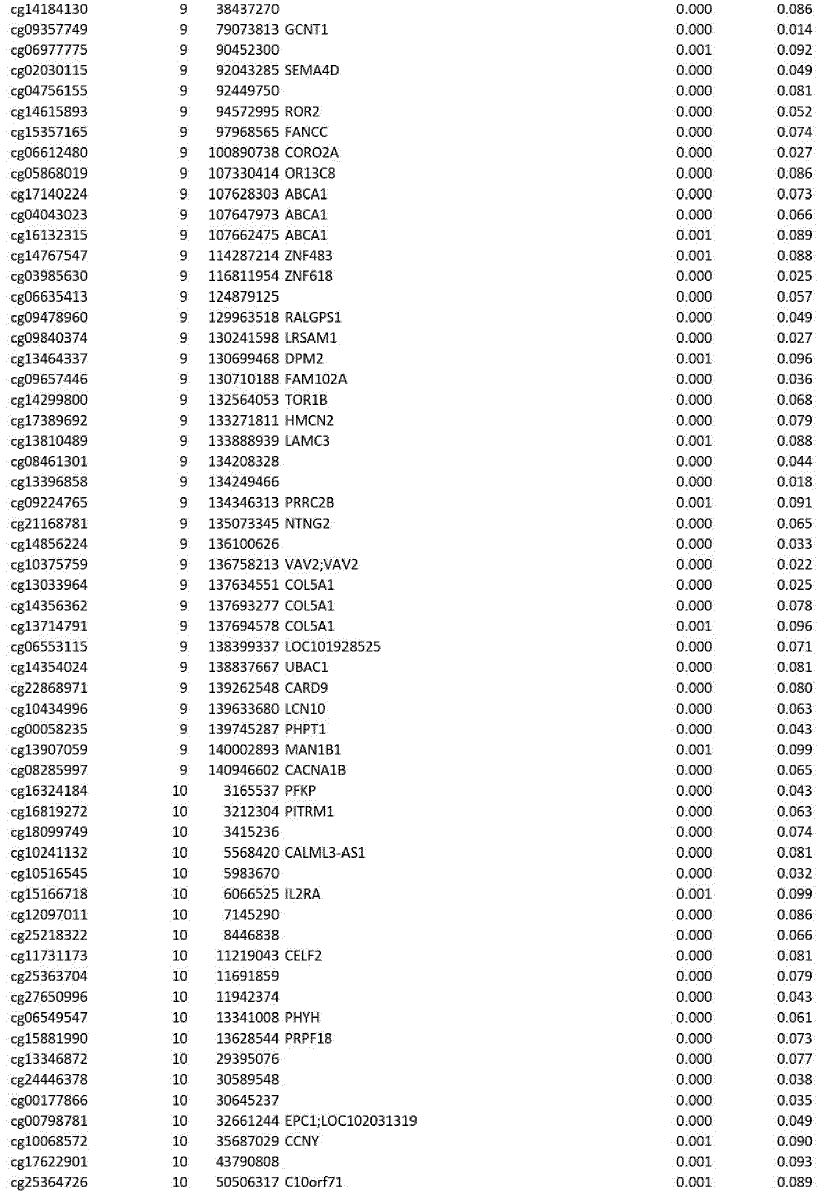

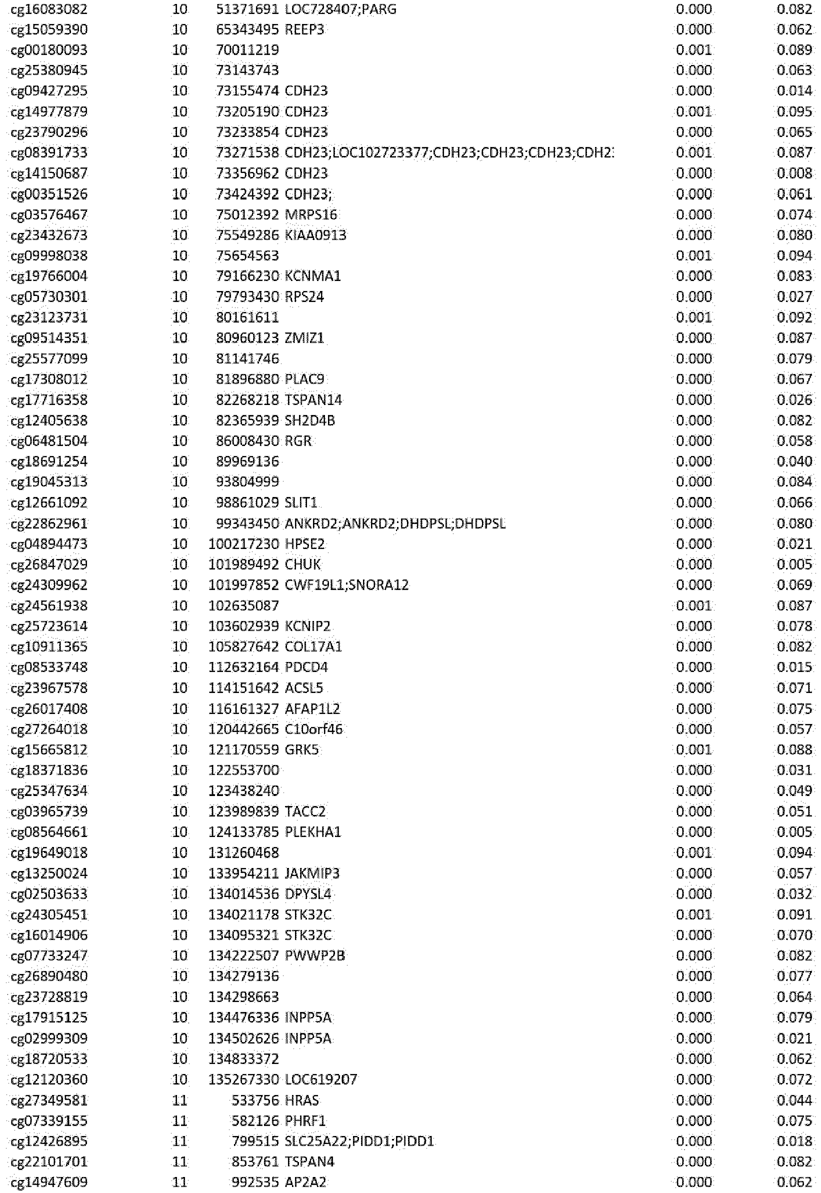

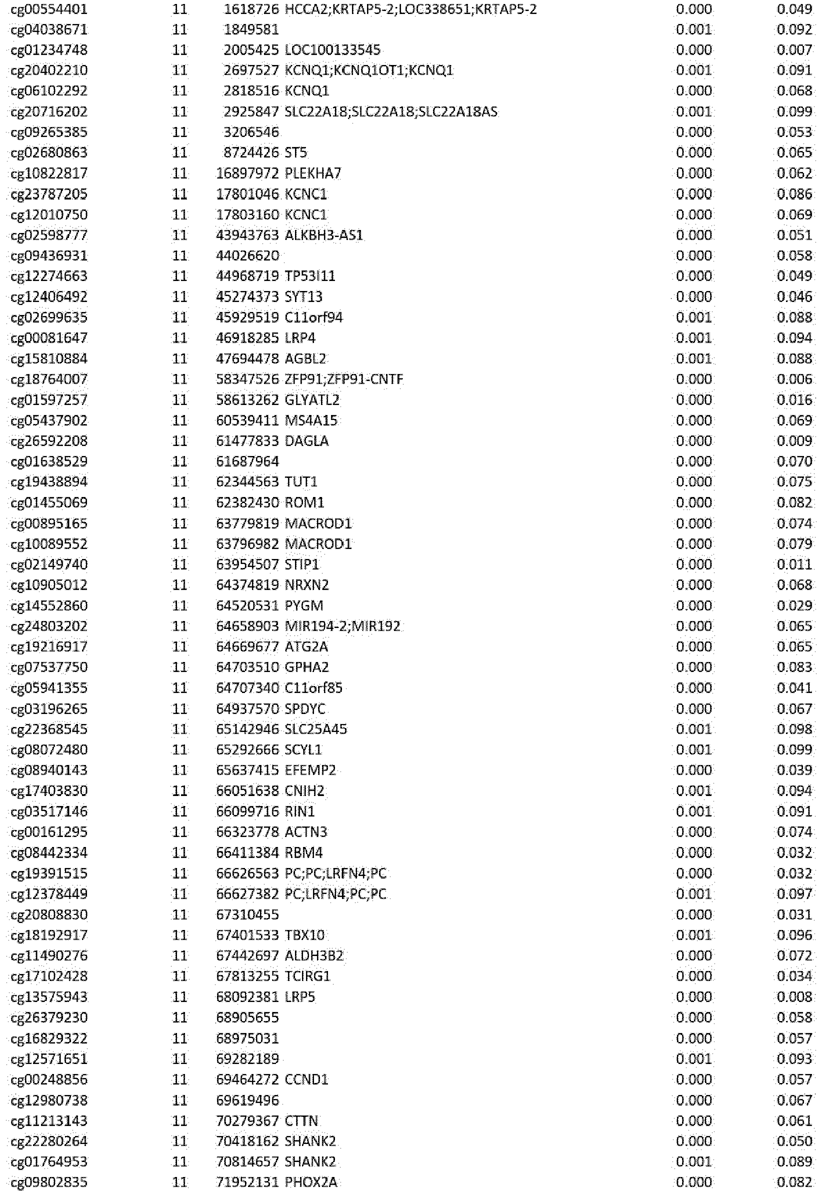

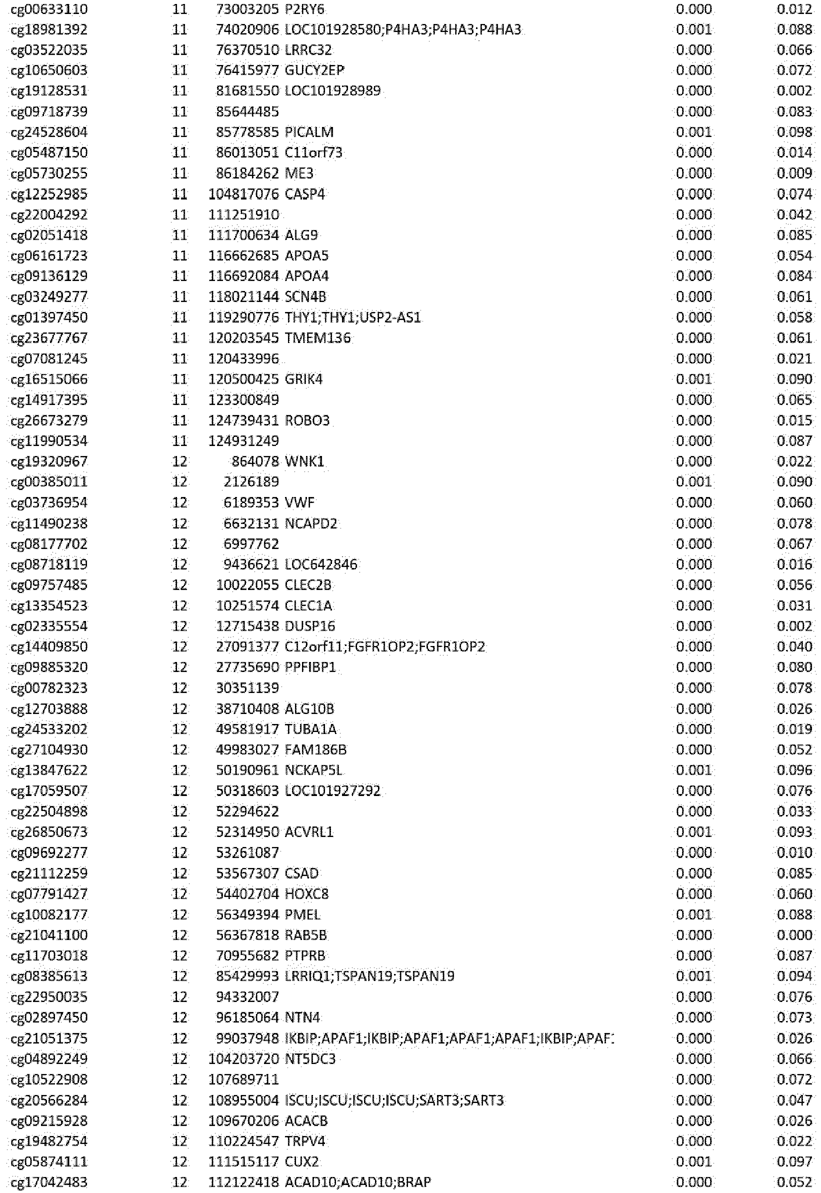

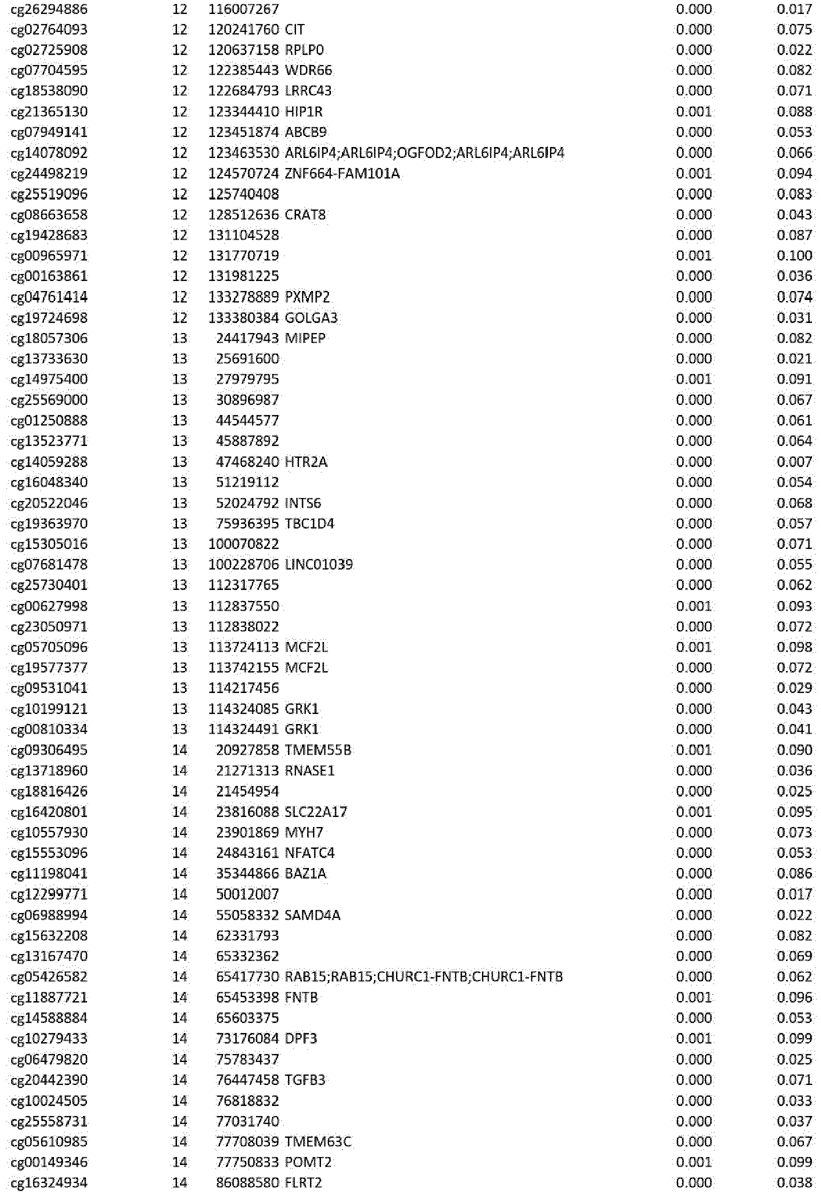

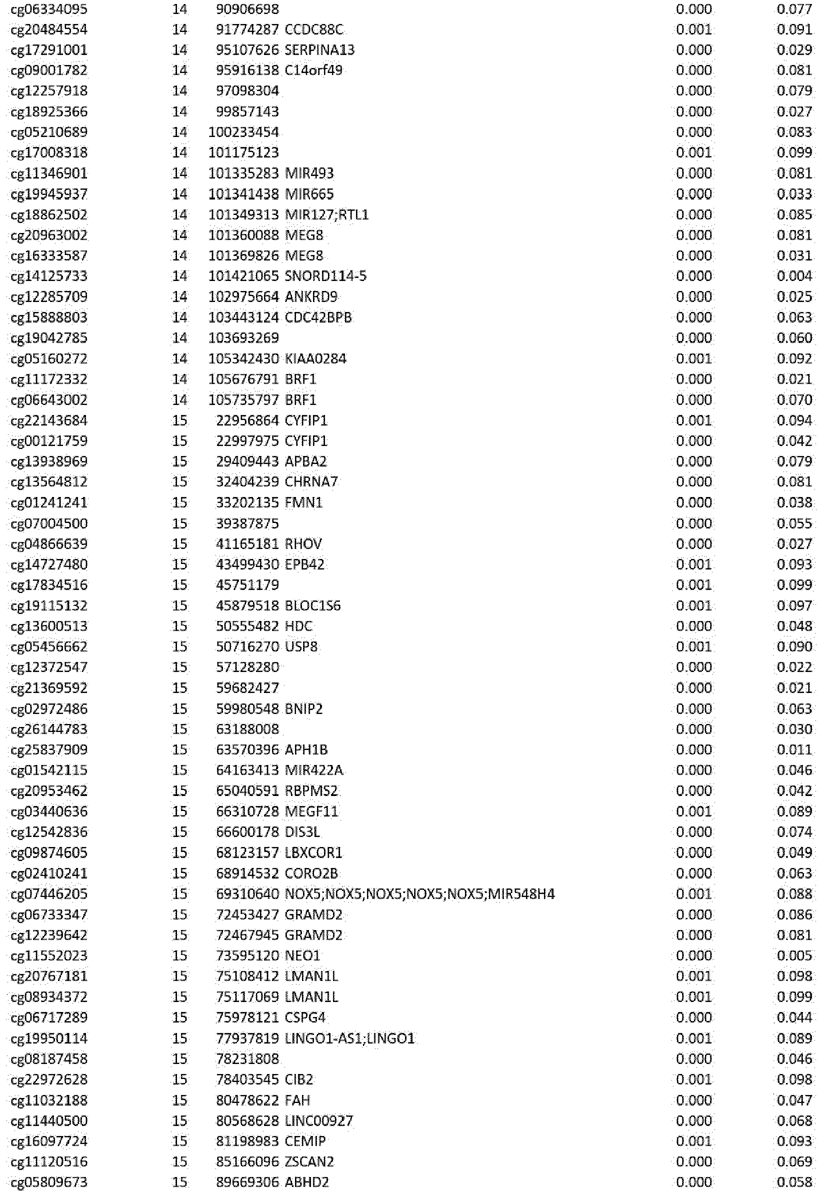

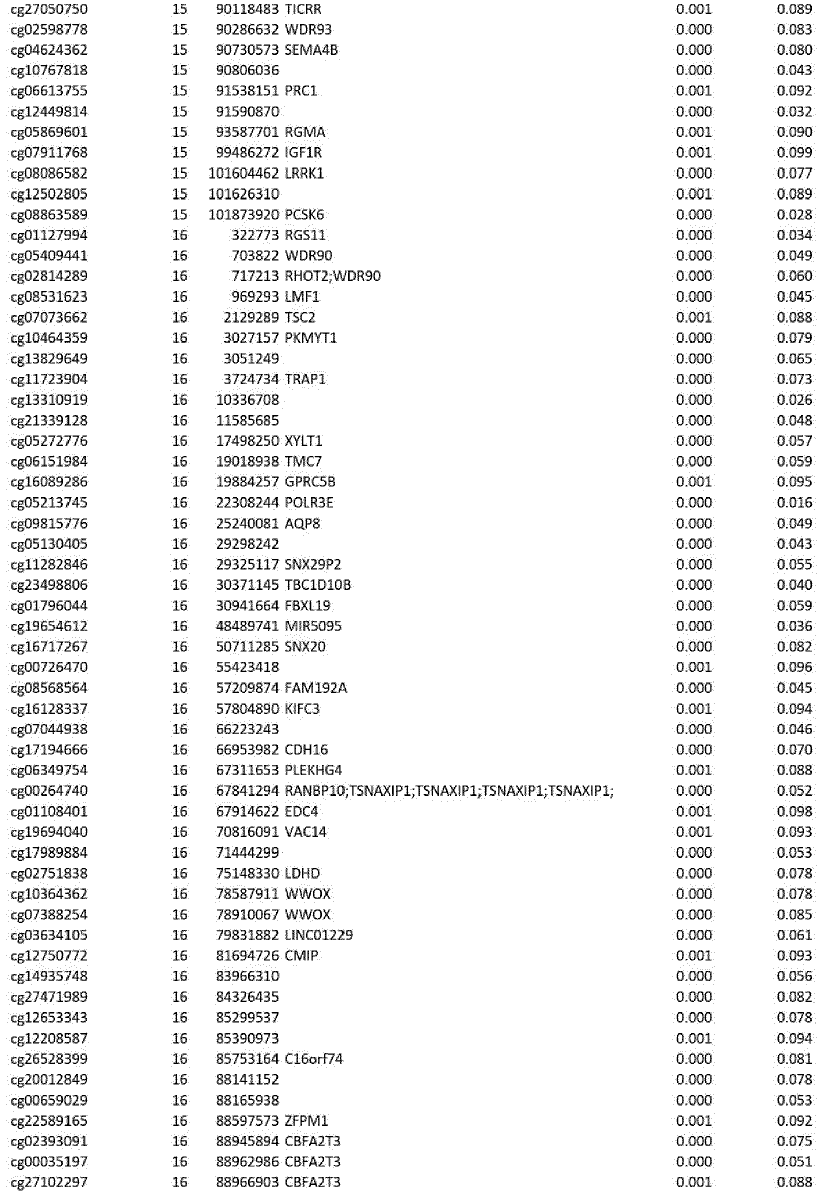

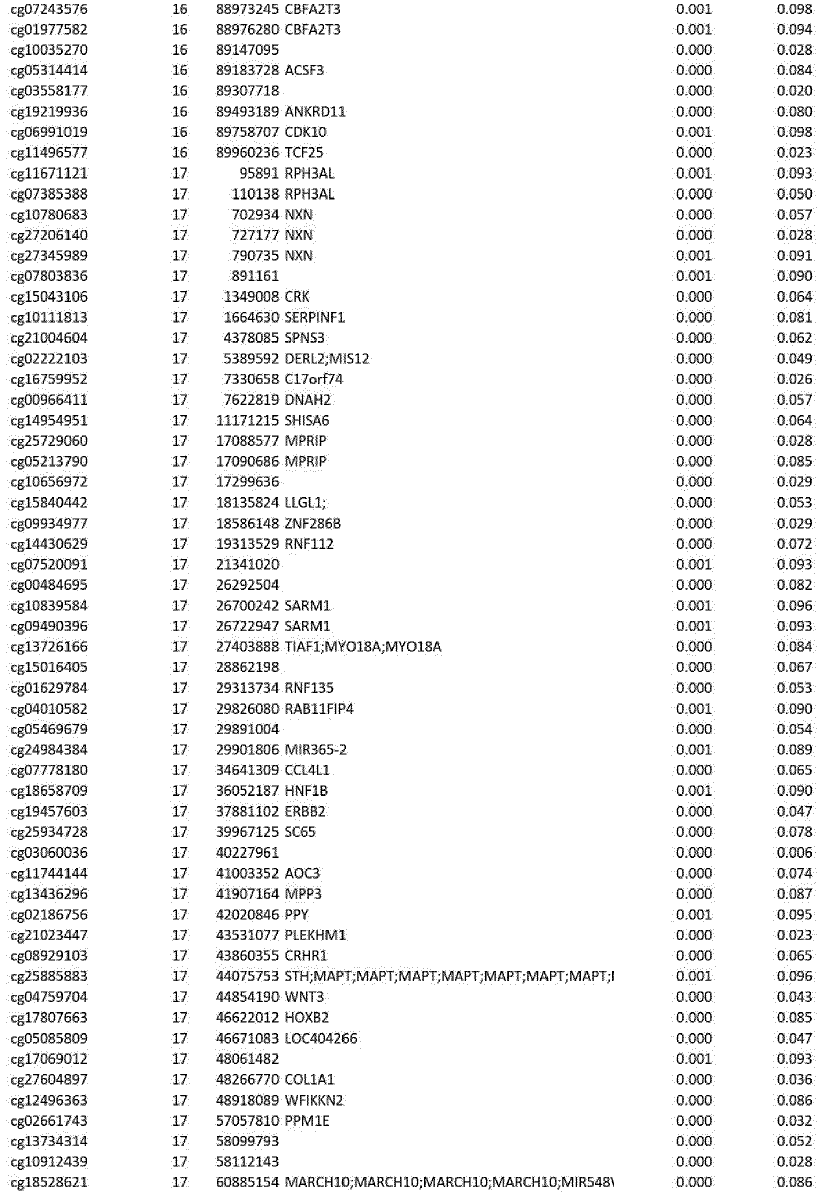

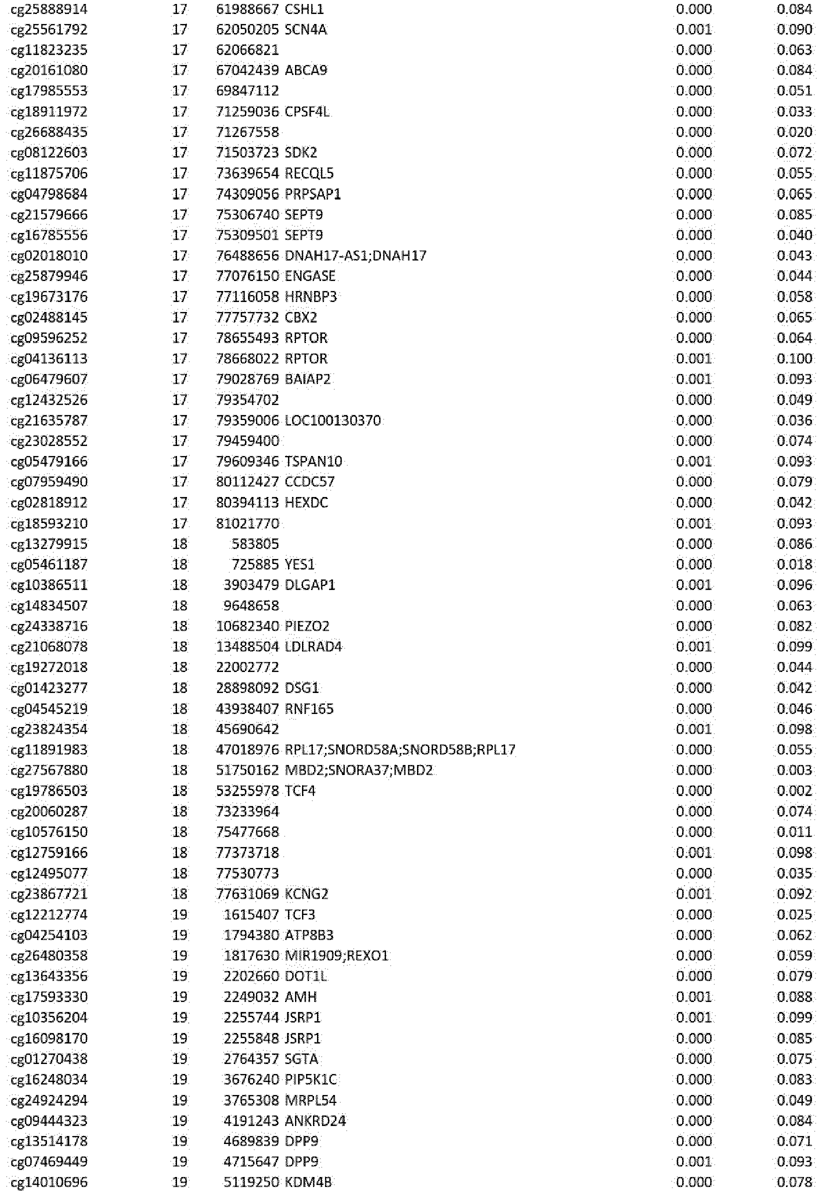

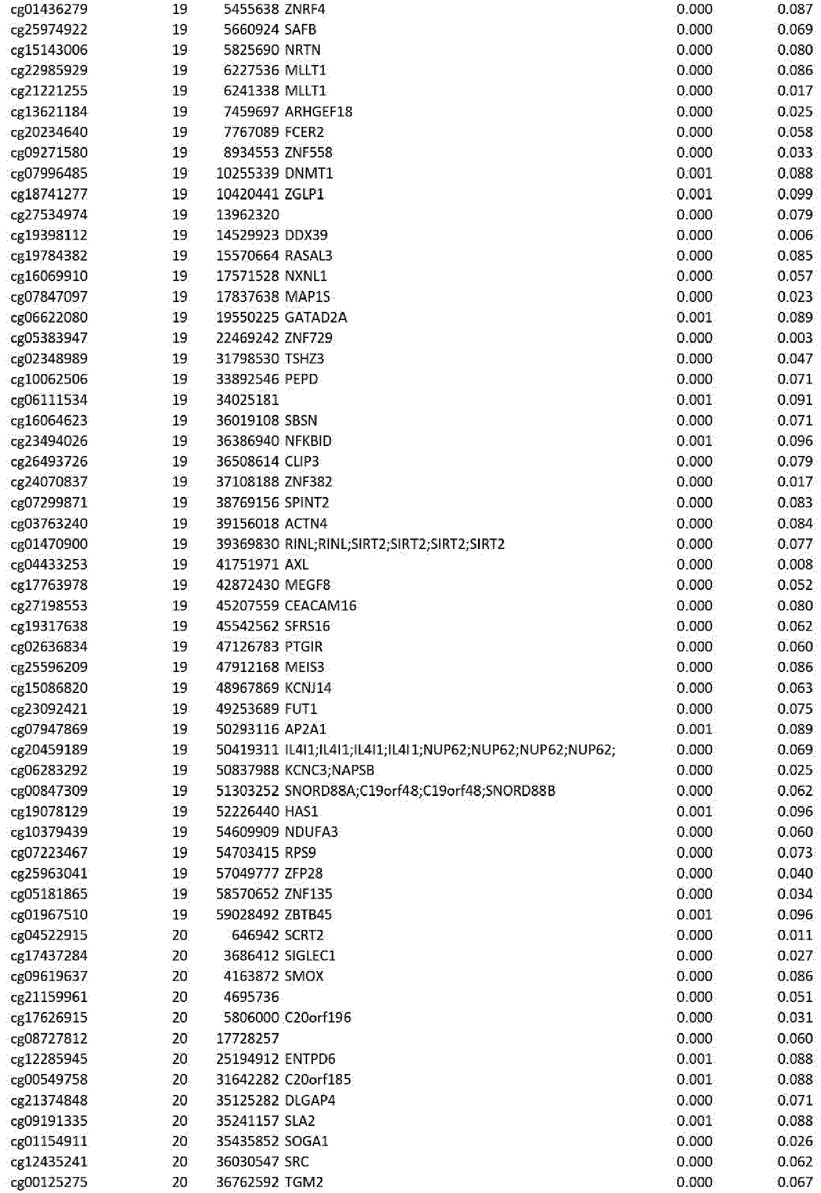

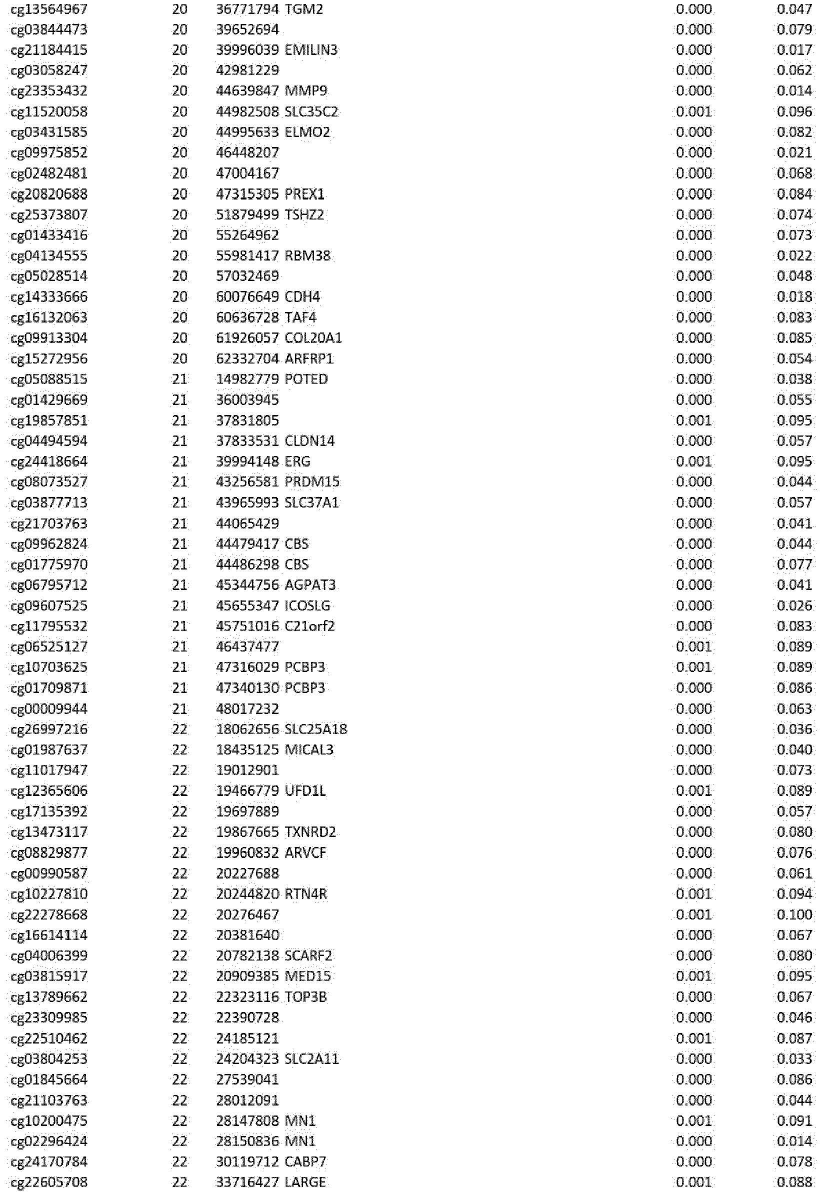

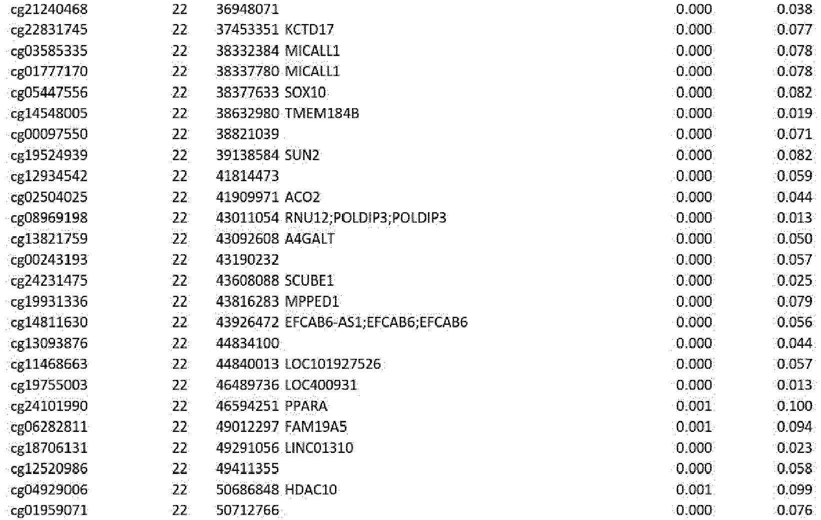

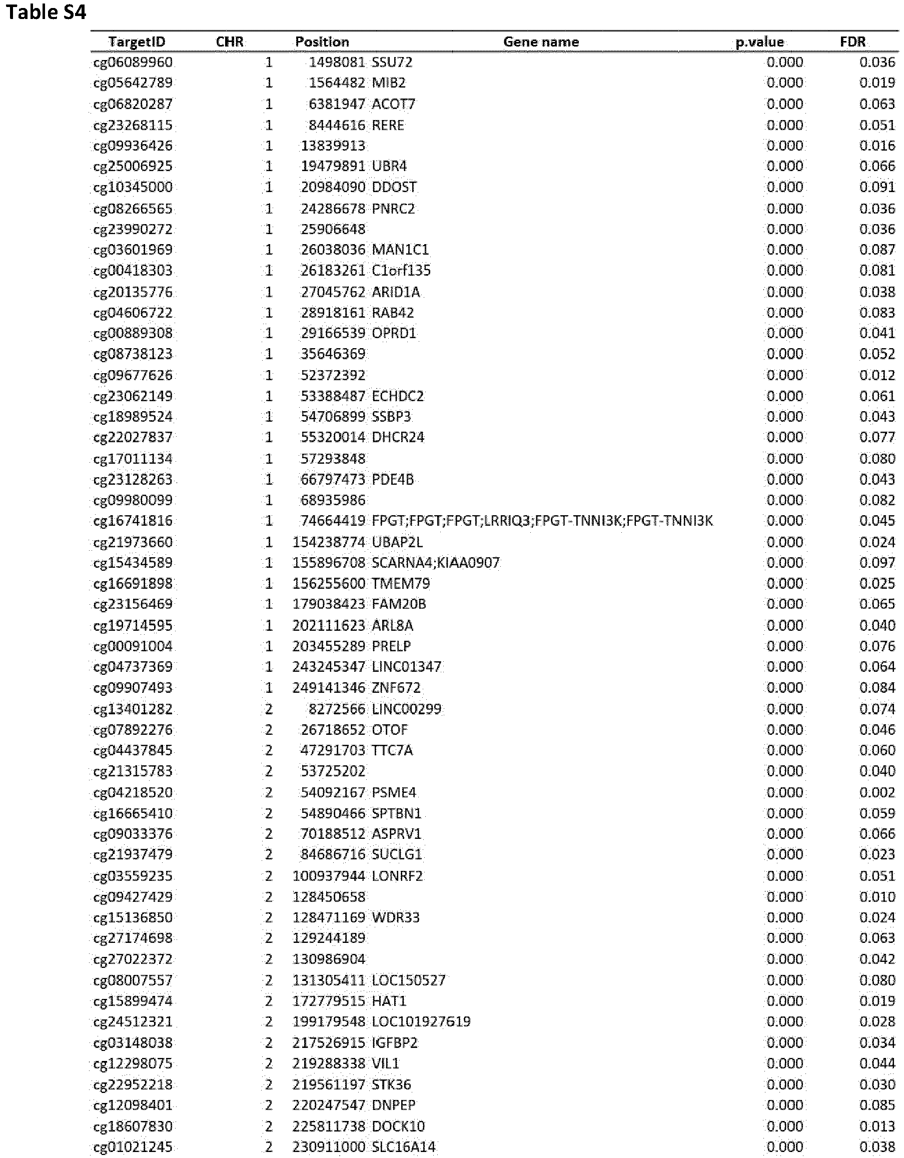

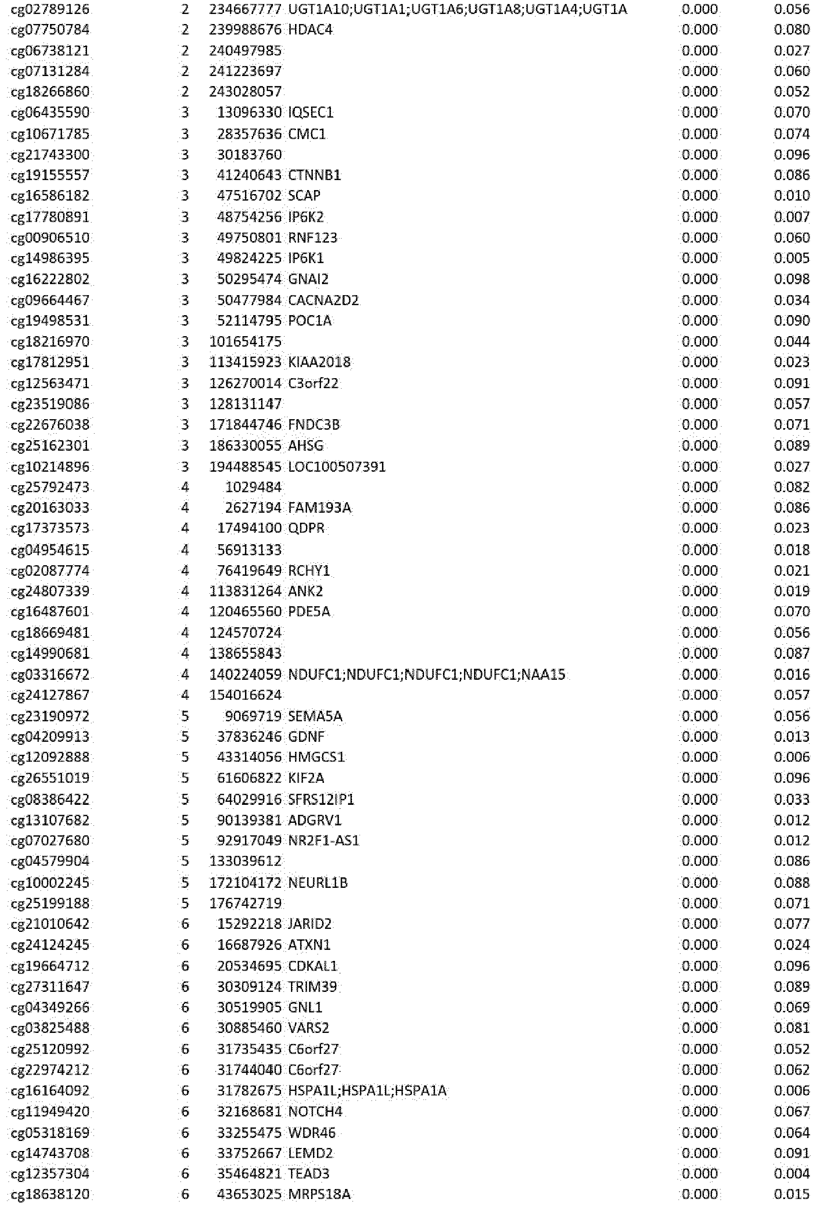

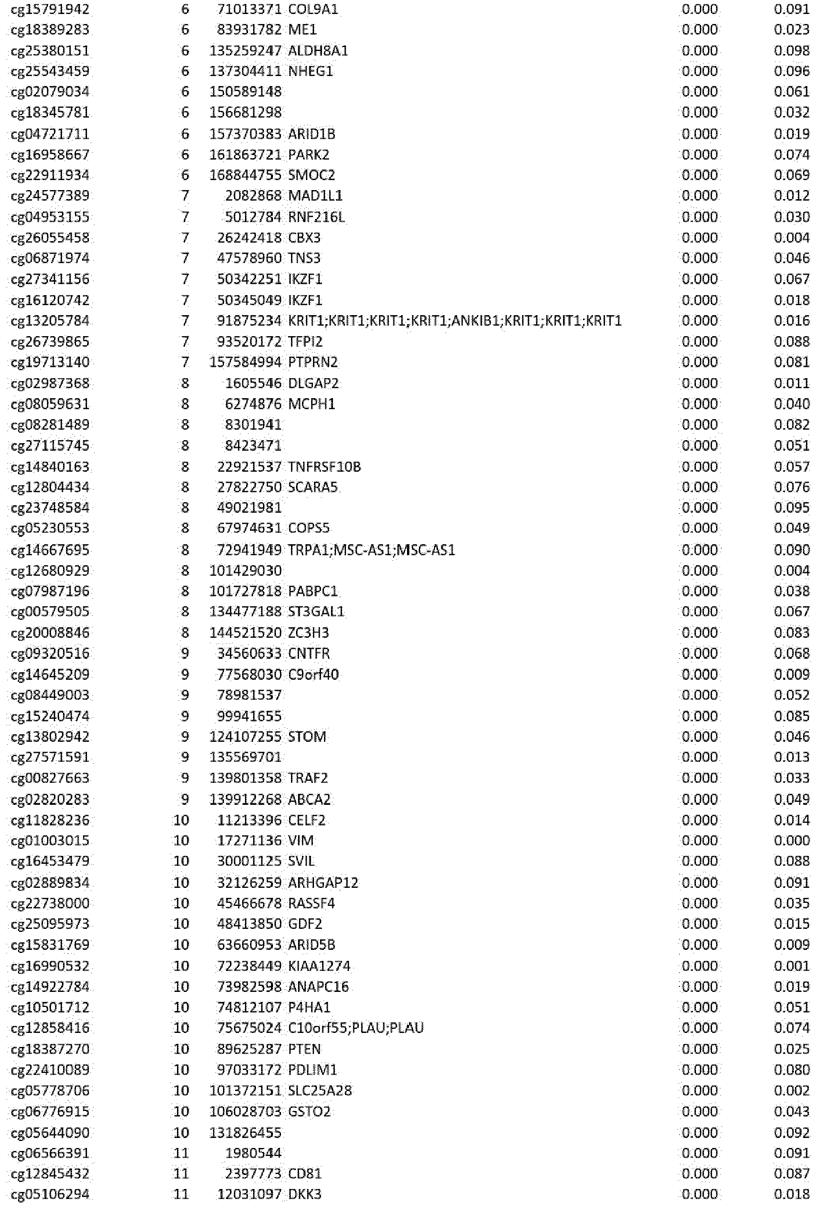

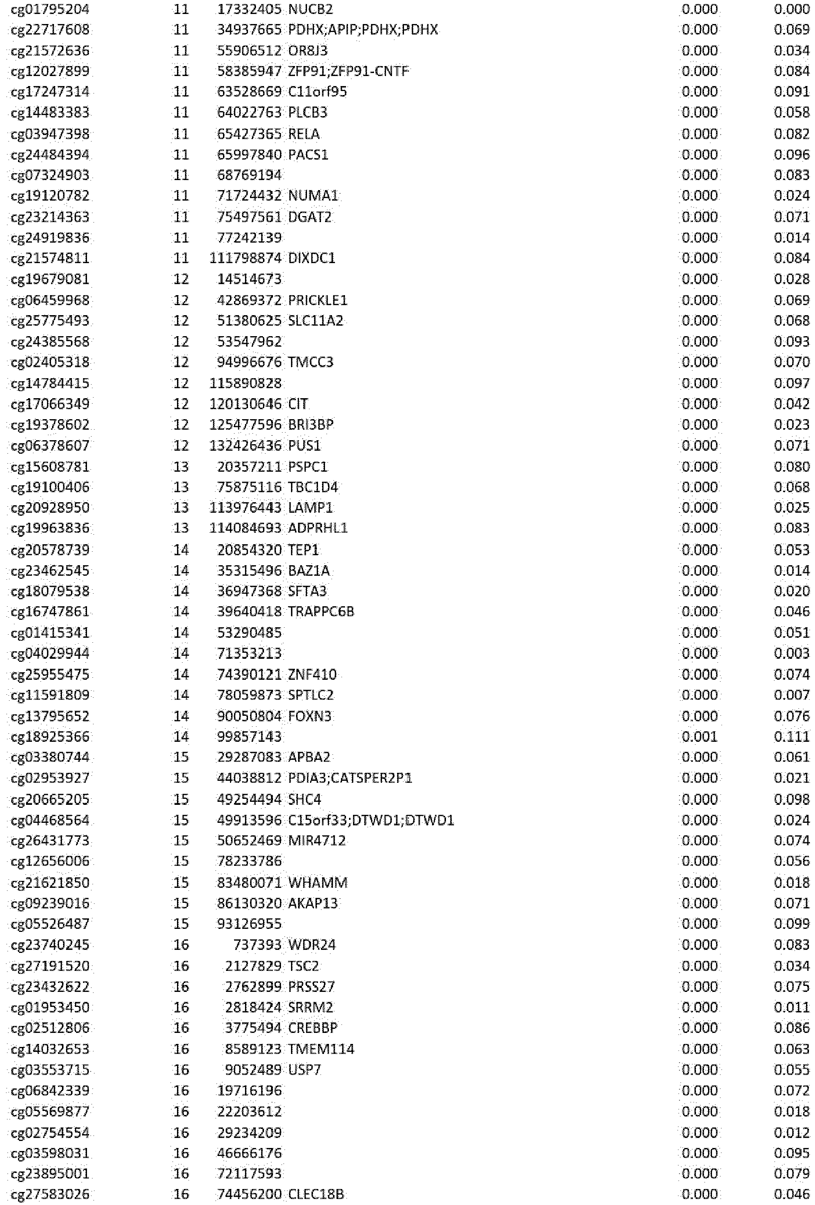

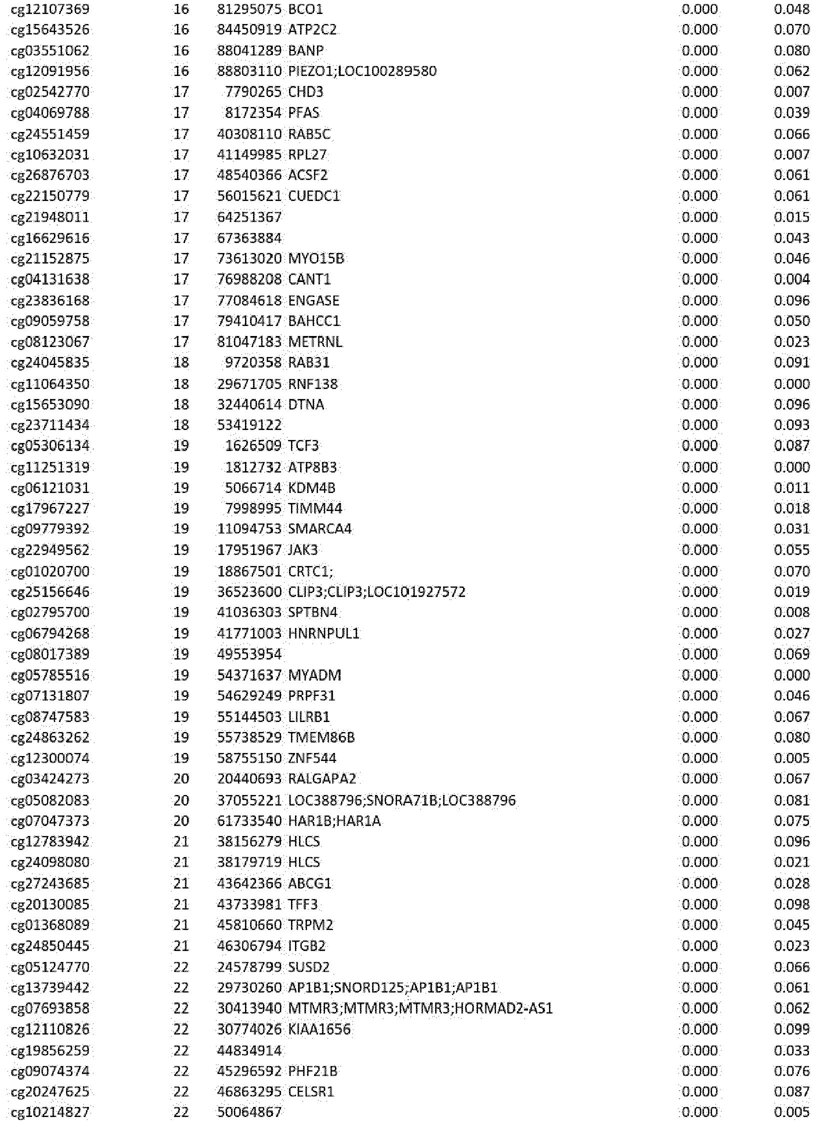

## Claims

1. *In vitro* method for screening and/or selecting genes whose methylation status indicates whether a patient suffering from obesity is responding or will respond to a treatment with a very-low-calorie ketogenic diet (VLCKD), which comprises:
a. Selecting those CpG sites **characterized by** being differentially methylated, showing a differential β value ≥ 2% and FDR < 0.10, between patients who are in the period of maximum ketosis that occurs between days 30 and 90 of the diet and the patients who are at the end of the diet that occurs between days 120 and 180, with respect to a control value represented by the level of methylation in patients who are in baseline state that occurs at the beginning of the diet; and/or
b. Selecting those CpG sites **characterized by** being differentially methylated, showing a differential β value ≥ 2% and FDR < 0.10, between patients who are in the period of maximum ketosis that occurs between days 30 and 90 of the diet, with respect to a control value represented by the level of methylation in patients who are in baseline state that occurs at the beginning of the diet, and discarding those CpG sites that are differentially methylated between the patients who are in the period of maximum ketosis that occurs between days 30 and 90 of the diet and the patients who are at the end of the diet that occurs between days 120 and 180; and
c. Selecting those genes which comprises in the promoter region at least two of the differentially methylated CpG sites which have been selected according to the steps a) and/or b).

2. *In vitro* method, according to claim 1, wherein the genes and CpG sites which are selected according to the steps a) and c) of claim 1 are comprised in Table 2 and their methylation status indicates whether a patient suffering from obesity is responding or will respond to VLCKD giving rise to both weight loss and nutritional ketosis induction, and wherein the genes and CpG sites which are selected according to the steps b) and c) of claim 1 are comprised in Table 3 and their methylation status indicates whether a patient suffering from obesity is responding or will respond to VLCKD due to the induction of nutritional ketosis.

3. *In vitro* method for monitoring or predicting whether a patient suffering from obesity is responding or will respond to a VLCKD, which comprises determining the methylation status of at least a gene selected according to the method of claim 1, wherein a statistically significant variation or deviation of level of methylation, as compared with a pre-established level of methylation, is an indication that the subject is responding or will respond to a VLCKD.

4. *In vitro* method, according to claim 3, for monitoring or predicting whether a patient suffering from obesity is responding or will respond to a VLCKD giving rise to both weight loss and nutritional ketosis induction **characterized by** low fat consumption, which comprises determining the methylation status of at least a gene selected from Table 2, wherein a statistically significant variation or deviation of level of methylation, as compared with a pre-established level of methylation, is an indication that the subject is responding or will respond to a VLCKD.

5. *In vitro* method, according to claim 4, wherein the methylation status of the genes is determined in at least a CpG site selected from Table 2.

6. *In vitro* method, according to claims 3 or 4, for monitoring or predicting whether an improvement of metabolic parameters has occurred in a patient suffering from obesity after the induction of nutritional ketosis by means of a treatment with a VLCKD, which comprises determining the methylation status of at least a gene selected from Table 3 in a biological sample obtained from the patient, wherein a statistically significant variation or deviation of level of methylation, as compared with a pre-established level of methylation, is an indication that ketosis reduction has occurred.

7. *In vitro* method, according to claim 6, wherein the methylation status of the genes is determined in at least a CpG site selected from Table 3.

8. *In vitro* method, according to any of the previous claims, wherein an overall hypomethylation of the genes is observed when the patient is responding or will respond to the treatment with a VLCKD.

9. *In vitro* method, according to any of the previous claims, wherein the methylation status detection is conducted by means of a technique selected from the group consisting of: methylation specific PCR, bisulfite sequencing, techniques based on restriction-digestion, pyrosequencing, assay ChIP-on-chip, differential conversion, differential restriction and/or differential weight of site(s) methylated.

10. *In vitro* method, according to any of the previous claims, wherein biological sample isolated from the patient is whole blood, preferably blood leucocytes.

11. *In vitro* use of the methylation status of at least a gene selected according to the method of claim 1 for monitoring or predicting whether a patient suffering from obesity is responding or will respond to a VLCKD.

12. *In vitro* use, according to claim 11, of the methylation status of at least a gene selected from Table 2 for monitoring or predicting whether a patient suffering from obesity is responding or will respond to a VLCKD giving rise to both weight loss and nutritional ketosis induction, **characterized by** low fat consumption.

13. *In vitro* use, according to claim 12, of the methylation status of at least a CpG site selected from Table 2.

14. *In vitro* use, according to claims 11 or 12, of the methylation status of at least a gene selected from Table 3 for monitoring or predicting whether an improvement of metabolic parameters has occurred in a patient suffering from obesity after the induction of nutritional ketosis by means of a treatment with a VLCKD.

15. *In vitro* use, according to claim 14, of the methylation status of at least a CpG site selected from Table 3.
